(19) Europäisches Patentamt
European Patent Office
Office européen des brevets

(11) Veröffentlichungsnummer: 0 295 630 A2

(12) EUROPÄISCHE PATENTANMELDUNG

(21) Anmeldenummer: 88109479.1

(22) Anmeldetag: 14.06.88

(51) Int. Cl.4: C07D 501/36, C07D 501/26, C07D 501/38, A61K 31/545

Patentansprüche für folgende Vertragsstaaten: GR + ES.

(30) Priorität: 16.06.87 US 62928
03.05.88 US 189936

(43) Veröffentlichungstag der Anmeldung: 21.12.88 Patentblatt 88/51

(84) Benannte Vertragsstaaten: AT BE CH DE ES FR GB GR IT LI LU NL SE

(71) Anmelder: F. HOFFMANN-LA ROCHE & CO. Aktiengesellschaft

CH-4002 Basel(CH)

(72) Erfinder: Wei, Chung-Chen
244 Malapardis Road
Cedar Knolls, N.J. 07927(US)
Erfinder: West, Kevin Francis
4 Crestwood Avenue
Fairfield, N.J. 07006(US)

(74) Vertreter: Lederer, Franz, Dr. et al
Patentanwält Dr. Franz Lederer Lucile Grahnstrasse 22
D-8000 München 80(DE)

# (54) Cephalosporinderivate.

(57) Acylderivate der allgemeinen Formel

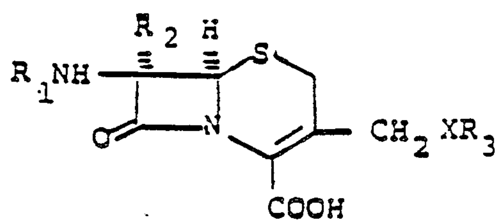

I

in der X -OCO-, -SCO-, -NHCO- oder -OCONH-; $R_1$ eine Acylgruppe; $R_2$ Wasserstoff oder niederes Alkoxy; und $R_3$ die Gruppe

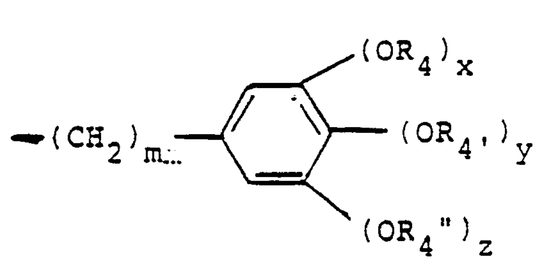

oder

EP 0 295 630 A2

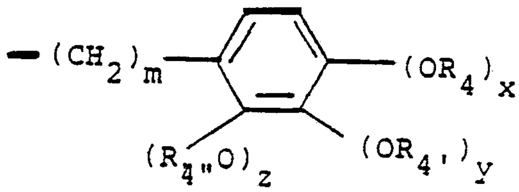

oder

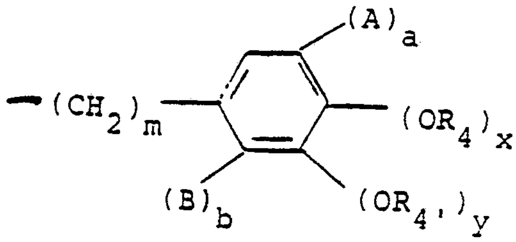

darstellen,

worin $R_4$, $R_4'$ und $R_4''$ unabhängig voneinander Wasserstoff oder $-COR_{200}$ mit $R_{200}$ geradkettigem oder verzweigtem niederem Alkyl, A und B Halogen und a, b, x, y und z unabhängig voneinander Null (wobei in der entsprechenden Ringposition ein Wasserstoffatom vorliegt) oder 1 bedeuten, wobei mindestens zwei der Symbole x, y und z 1 darstellen, und m Null oder eine Zahl von 1 bis 8 bedeutet,

sowie leicht hydrolysierbare Ester und pharmazeutisch verträgliche Salze dieser Verbindungen und Hydrate der Verbindungen der Formel I bzw. von deren Estern und Salzen.

Die Produkte sind antibakteriell wirksam.

## Cephalosporinderivate

Die vorliegende Erfindung betrifft Acylderivate der allgemeinen Formel

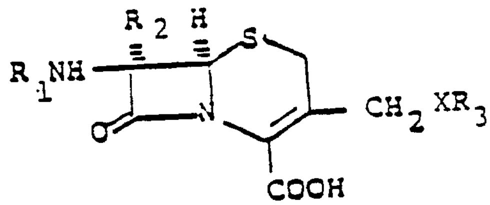

I

in der X -OCO-, -SCO-, -NHCO- oder -OCONH-, $R_1$ eine Acylgruppe; $R_2$ Wasserstoff oder niederes Alkoxy; und $R_3$ die Gruppe

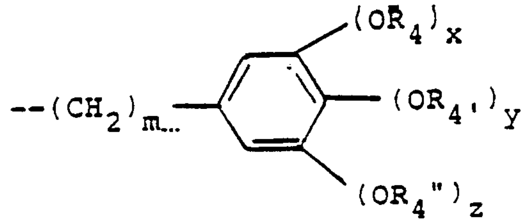

oder

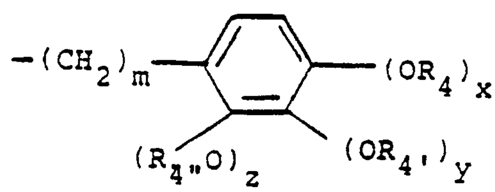

oder

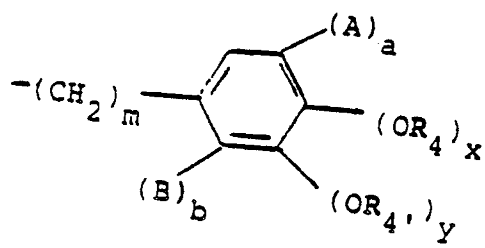

darstellen,

worin $R_4$, $R_4'$ und $R_4''$ unabhängig voneinander Wasserstoff oder $-COR_{200}$ mit $R_{200}$ geradkettigem oder verzweigtem niederem Alkyl, A und B Halogen und a, b, x, y und z unabhängig voneinander Null (wobei in der entsprechenden Ringposition ein Wasserstoffatom vorliegt) oder 1 bedeuten, wobei mindestens zwei der Symbole x, y und z 1 darstellen, und m Null oder eine Zahl von 1 bis 8 bedeutet,
sowie leicht hydrolysierbare Ester und pharmazeutisch verträgliche Salze dieser Verbindungen und Hydrate der Verbindungen der Formel I bzw. von deren Estern und Salzen.

Der Ausdruck "niederes Alkyl" oder "Alkyl" bezieht sich auf gerad- und verzweigtkettige Kohlenwasserstoffgruppen mit 1-8, bevorzugt 1-4 Kohlenstoffatomen, z. B. Methyl, Ethyl, n-Propyl, Isopropyl, t-Butyl oder dgl.

Der Ausdruck "niederes Alkoxy" oder "Alkoxy" bezieht sich auf gerad- oder verzeigtkettige Kohlenwasserstoff-Oxygruppen, worin der Alkylrest die oben gegebene Bedeutung hat. Beispiele sind Methoxy, Ethoxy, n-Propoxy und dgl.

Der Ausdruck "Halogen" bezieht sich auf Chlor, Brom, Jod oder Fluor, sofern nicht anderweitig definiert.

Der Ausdruck "Acyl" steht für alle organische Reste welche durch Entfernung der Hydroxygruppe aus einer Carbonsäure gebildet werden. Gewisse Acylreste sind bevorzugt, wie nachstehend beschrieben.

Der Ausdruck "Aryl" bedeutet einen gebenenfalls substituierten aromatischen Rest, wie z.B. Phenyl, Tolyl, Xylyl, Mesityl, Cumyl (Isopropylphenyl), Naphthyl und dgl., wobei die Arylgruppe z.B. 1-3 geeignete Substituenten tragen kann, wie Halogen (z.B. Fluor, Chlor, Brom), Hydroxy oder dgl.

Der Ausdruck "niederes Alkanoyl" oder "Alkanoyl" bezeichnet einen Rest der allgemeinen Formel

$R_{25}$-CO-

in der $R_{25}$ Wasserstoff oder $C_{1-6}$ Alkyl darstellt.

Beispiele dieser Gruppen sind Acetyl, Formyl, Propionyl, n-Butyryl und dgl.

Der Ausdruck "substituiertes Phenyl" bedeutet eine mono- oder di-substituierte Phenylgruppe, wobei als Substituenten Halogen (z.B. Chlor, Brom, Fluor), niederes Alkyl, Amino, Nitro oder Trilfuormethyl in Frage kommen.

Der Ausdruck "substituiertes Alkyl" bedeutet eine niedere Alkylgruppe, die z.B. durch Halogen (z.B. Chlor, Fluor, Brom), Trifluormethyl, Amino, Cyano, etc. substituiert sein kann.

Der Ausdruck "niederes Alkenyl" oder "Alkenyl" bedeutet gerad- oder verzweigtkettige Kohlenwasserstoffgruppen mit einer olefinischen Doppelbindung und 2-6 Kohlenstoffatomen, d.h. den Rest von Verbindungen der Formel $C_nH_{2n}$ worin n 2-6 ist, z.B. Allyl, Vinyl, etc.

Der Ausdruck "Aralkyl" bedeutet eine Kohlenwasserstoffgruppe mit aromatischer und aliphatischer Struktur, d.h. eine Kohlenwasserstoffgruppe, worin ein Wasserstoffatom der niederen Alkylgruppe durch eine monocyclische Arylgruppe substituiert ist, z.B. durch Phenyl, Tolyl, etc.

Der Ausdruck "5-, 6- oder 7-gliedriger heterocyclischer Ring mit 1-4 Sauerstoff, Stickstoff und/oder Schwefelatomen" bedeutet z.B. die folgenden Gruppen: 6-gliedrige, Stickstoff enthaltende heterocyclische Ringe, wie Pyridyl, Piperidyl, Piperidino, N-Oxido-pyridyl, Pyrimidyl, Piperazinyl, Pyridazinyl, N-Oxido-pyridazinyl, etc.; 5-gliedrige Stickstoff enthaltende heterocyclische Ringe, z.B. Pyrazolyl, Pyrrolidinyl, Imidazolyl, Thiazolyl, 1,2,3-Thiadiazolyl, 1,2,4-Thiadiazolyl, 1,3,4-Thiadiazolyl, 1,2,5-Thiadiazolyl, 1,2,3-Oxadiazolyl, 1,2,4-Oxadiazolyl, 1,3,4-Oxadiazolyl, 1,2,5-Oxadiazolyl, 1,2,3-Triazolyl, 1,2,4-Triazolyl, 1H-Tetrazolyl, 2H-Tetrazolyl, etc.. Diese heterocyclischen Ringe können weiter substituiert sein, wobei z.B. folgende Substituenten in Frage kommen: niederes Alkyl wie Methyl, Ethyl, n-Propyl etc., niederes Alkoxy, z.B. Methoxy, Ethoxy etc., Halogen wie Chlor, Brom etc., Halogen-niederes Alkyl wie Trifluormethyl, Trichlorethyl etc., Amino, Mercapto, Hydroxy, Carbamoyl oder Carboxy etc..

Der Ausdruck "Cyclo-niederes-Alkyl" oder "Cycloalkyl" bedeutet einen 3-7-gliedrigen gesättigten carboxylischen Rest, z.B. Cyclopropyl, n-Cyclobutyl, n-Cyclohexyl, etc. "Cyclo-niederes-Alkenyl" oder "Cycloalkenyl" bedeutet entspechende 3-7-gliedrige ungesättigte carboxylische Reste wie n-Cyclohexenyl.

Beispiele für Acylgruppen $R_1$ sind diejenigen, welche bisher zur Acylierung von $\beta$-Lactam-Antibiotika, einschliesslich der 6-Aminopenicillansäure und deren Derivaten sowie der 7-Aminocephalosporansäure und deren Derivaten, verwendet worden sind; siehe z.B. Cephalosporins and Penicillins, Verlag Flynn, Academic Press (1972), Belgische Patentschrift No. 866.038, veröffentlicht am 17. Oktober 1978, Belgische Patentschrift No. 867,994, veröffentlicht am 11. Dezember 1978, U.S. Patentschrift No. 4,152,432, veröffentlicht am 1. Mai 1979, U.S. Patentschrift No. 3,971,778, veröffentlicht am 27. Juli 1976 und U.S. Patentschrift No. 4,173,199, veröffentlicht am 23. Oktober 1979. In diesen Publikationen werden verschiedene Acylreste, die im vorliegenden Falle Verwendung finden, erwähnt. Die nachfolgend aufgeführten Acylreste veranschaulichen weiter den Begriff "Acyl", beschränken ihn jedoch in keiner Weise. Beispiele für Acylreste sind:

a) Aliphatische Gruppen der Formel

$R_5$CO-

in der $R_5$ niederes Alkyl, niederes Cycloalkyl, niederes Alkoxy, niederes Alkenyl, niederes Cycloalkenyl oder Cyclohexadienyl; oder durch einen oder mehrere der Reste Halogen, Cyan, Nitro, Amino, Mercapto, niederes Alkylthio oder Cyanmethylthio substituiertes niederes Alkyl oder niederes Alkenyl darstellt.

b) Carbocyclische aromatische Gruppen der allgemeinen Formel

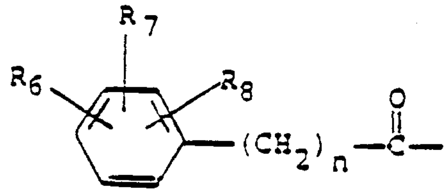

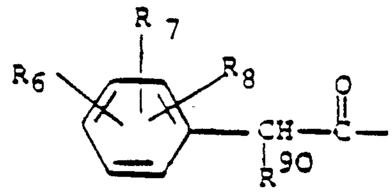

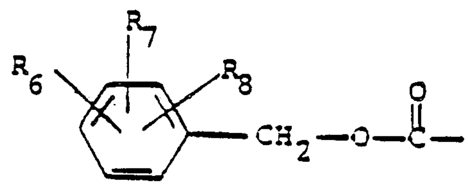

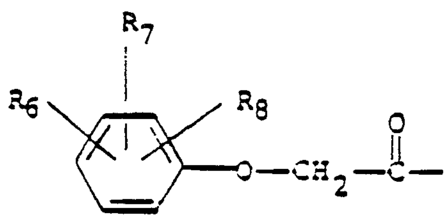

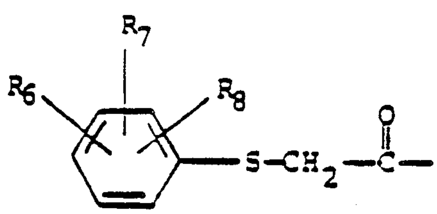

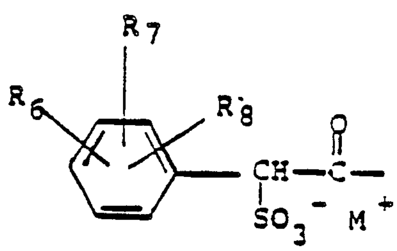

und

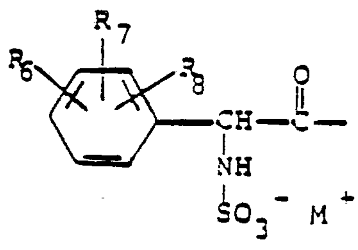

in der n 0, 1, 2 oder 3; $R_6$, $R_7$ und $R_8$ unabhängig voneinander Wasserstoff, Halogen, Hydroxy, Nitro, Amino, Cyan, Trifluormethyl, $C_1$-$C_4$ Alkyl, $C_1$-$C_4$ Alkoxy oder Aminomethyl; $R_{90}$ Amino, Acylamino, Hydroxy, ein Carboxysalz, geschütztes Carboxy, wie z.B. Benzyloxycarbonyl, Formyloxy, Azido oder ein Sulfosalz und M ein Kation darstellen.

Bevorzugte carbocyclische aromatische Acylgruppen sind diejenigen der Formel

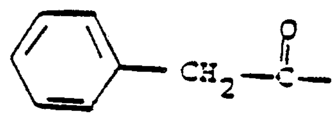

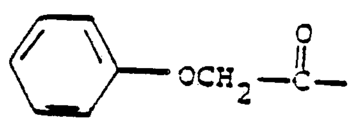

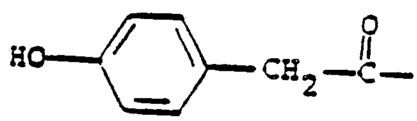

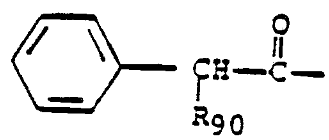

und

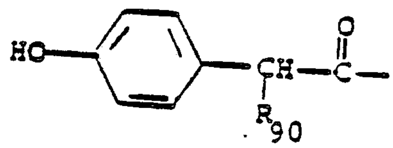

$R_{90}$ ist bevorzugt eine Aminogruppe, eine Hydroxygruppe oder ein Carboxy- oder Sulfosalz.

Beispiele anderer erfindungsgemäss einsetzbarer Acylgruppen sind:

Sulfophenylacetyl, Hydroxysulfonyloxyphenylacetyl, Sulfamoylphenylacetyl, (Phenoxycarbonyl)-phenylacetyl, (p-Tolyloxycarbonyl)phenylacetyl, Formyloxyphenylacetyl, Carboxyphenylacetyl, Formylaminophenylacetyl, Benzyloxycarbonylphenylacetyl,2-(N,N-Dimethylsulfamoyl)-2-phenylacetyl, 2-Bromo-2-thienylacetyl, etc.

(c) Heteroaromatische Gruppen der allgemeinen Formel

$$R_{101}-(CH_2)_n-CO-$$

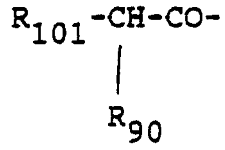

$$R_{101}-O-CH_2-CO-$$

$$R_{101}-S-CH_2-CO-$$

$$R_{101}-CO-CO-$$

in der $R_{90}$ die oben gegebene Bedeutung hat, n 0, 1, 2 oder 3 und $R_{101}$ einengegebenenfalls substituierten 5-, 6- oder 7-gliedrigen heterocyclischen Ring mit 1, 2, 3 oder 4 (vorzugsweise 1 oder 2) Stickstoff-, Sauerstoff- und/oder Schwefelatomen darstellt.

Beispiele heterocyclischer Ringe sind Thienyl, Furyl, Pyrrolyl, Pyridinyl, Pyrazinyl, Thiazolyl, Pyrimidinyl und Tetrazolyl. Beispiele für Substituenten am heterocyclischen Ring sind Halogen, Hydroxy, Nitro, Amino, Cyan, Trifluoromethyl, $C_1$-$C_4$ Alkyl sowie $C_1$-$C_4$ Alkoxy.

Bevorzugte heteroaromatische Acylgruppen sind diejenigen, worin $R_{101}$ 2-Amino-4-thiazolyl, 2-Amino-5-halo-4-thiazolyl, 4-Aminopyridin-2-yl, 2-Amino-1,3,4-thiadiazol-5-yl, 2-Thienyl, 2-Furanyl, 4-Pyridinyl or 2,6-Dichloro-4-pyridinyl bedeutet.

(d) [[(4-Substituierte-2,3-dioxo-1-piperazinyl)carbonyl]amino]arylacetylgruppen der Formel

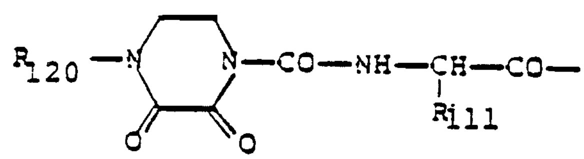

in der $R_{111}$ niederes Alkyl, Hydroxy-niederes Alkyl oder eine aromatische Gruppe der allgemeinen Formel

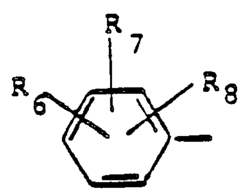

(in der $R_6$, $R_7$ und $R_8$ die obige Bedeutung haben) oder einen wie für $R_{101}$ definierten heterocyclischen Ring und $R_{120}$ gegebenenfalls durch einen oder mehreren der Reste Halogen, Cyan, Nitro, Amino oder Mercapto substituiertes niederes Alkyl darstellen, z.B. 4-niederes Alkyl (vorzugsweise Ethyl oder Methyl)-2,3-dioxo-1-piperazincarbonyl-D-phenylglycyl.

(e) Substituierte Oxyimino, Arylacetylgruppen der allgemeinen Formel

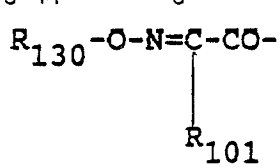

in der $R_{101}$ die obige Bedeutung hat und $R_{130}$ Wasserstoff, niederes Alkyl, $C_3$-$C_7$ Cycloalkyl, Carboxy-

$C_3$-$C_7$-Cycloalkyl oder substituiertes niederes Alkyl darstellt, wobei die niedere Alkylgruppe substituiert ist durch einen oder mehrere der Reste Halogen, Cyan, Nitro, Amino, Mercapto, niederes Alkylthio, eine durch $R_{111}$ oben definierte aromatische Gruppe, Carboxy (einschliesslich dessen Salze), Carbamoyl, niederes Alkoxycarbonyl, Phenylmethoxycarbonyl, Diphenylmethoxycarbonyl, Hydroxy-niederes-Alkoxyphosphinyl, Dihydroxyphosphinyl, Hydroxy(phenylmethoxy)phosphinyl oder di-niederes-Alkoxyphosphinyl.

Beispiele für Reste

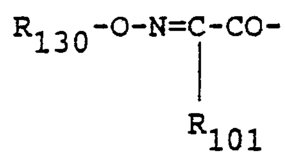

sind:
2-[(2-Chloracetamidothiazol-4-yl)-2-[(p-nitrobenzyloxycarbonyl]methoxyimino]acetyl, 2-(2-Chloracetamidothiazol-4-yl)-2-methoxyiminoacetyl, 2-(2-Aminothiazol-4-yl)-2-isopropoxyiminoacetyl, 2-(2-Aminothiazol-4-yl)-2-methoxyiminoacetyl, 2-(2-Aminothiazol-4-yl)-2-hydroxyiminoacetyl, 2-Thienyl-2-methoxy iminoacetyl, 2-Furyl-2-methoxyiminoacetyl, 2-(4-Hydroxyphenyl)-2-methoxyiminoacetyl, 2-Phenyl-2-methoxyiminoacetyl, 2-Phenyl-2-hydroxyiminoacetyl, 2-Thienyl-2-hydroxyiminoacetyl, 2-Thienyl-2-(dichloracetyloxyimino)acetyl, 2-[4-( -D-glutamyloxy)phenyl]-2-hydroxyiminoacetyl, 2-[4-(3-Amino-3-carboxypropoxy)phenyl]-2-hydroxyiminoacetyl, 2-(5-Chlor-2-chloracetamidothiazol-4-yl)-2-methoxyiminoacetyl, 2-(5-Chlor-2-aminothiazol-4-yl)-2-methoxyiminoacetyl, 2-[2-(t-Butoxycarbonyl)isopropoxyimino]-2-(2-sulfoaminothiazol-4-yl)acetyl, 2-[2-(t-Butoxycarbonyl-isopropoxyimino]-2-(2-triphenylmethylaminothiazol-4-yl)acetyl, 2-(2-Chloracetamidothiazol-4-yl)-2-isopropoxyiminoacetyl, 2-Methoxyimino-2-(2-sulfoaminothiazol-4-yl)acetyl, 2-[(2-Aminothiazol-4-yl)-2-carboxymethoxyimino]acetyl, 2-[2-(2-Mesylaminothiazol-4-yl)-2-isopropoxyiminoacetyl, 2-(2-Imino-3-mesyl-4-thiazolin-4-yl)-2-isopropoxyiminoacetyl, 2-[(2-Aminothiazol-4-yl)-2-(carboxyisopropoxyimino)acetyl, etc.

(f) (Acylamino) arylacetylgruppen der allgemeinen Formel

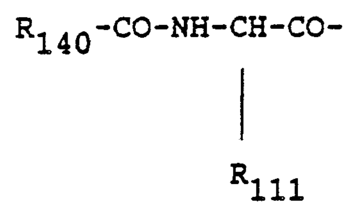

in der $R_{111}$ die obige Bedeutung hat und $R_{140}$ eine Gruppe der allgemeinen Formel

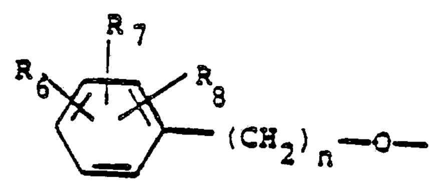

(worin $R_6$, $R_7$, $R_8$ und n die obige Bedeutung haben) Wasserstoff, niederes Alkyl, substituiertes niederes Alkyl, Amino, niederes Alkylamino, di-niederes Alkylamino, Cyan-niederes Alkylamino, Hydrazino, niederes Alkylhydrazino, Arylhydrazino oder Acylhydrazino darstellt.

Bevorzugte (Acylamino)arylacetylgruppen der obigen Formel sind diejenigen worin $R_{140}$ Amino oder Acylamino darstellt. Ebenfalls bevorzugt sind diejenigen Gruppen, worin $R_{111}$ Phenyl oder 2-Thienyl darstellt.

(g) (Substituierte-Acyloxyimino)arylacetylgruppen der Formel

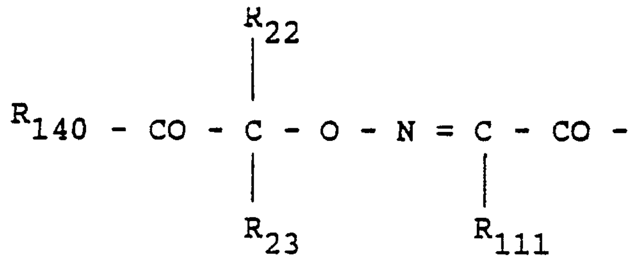

in der $R_{11'}$ und $R_{140}$ die obige Bedeutung haben und $R_{22}$ und $R_{23}$ unabhängig voneinander Wasserstoff oder niederes Alkyl darstellen oder $R_{22}$ und $R_{23}$ zusammen mit dem benachbarten Kohlenstoffatom einen $C_3$-$C_7$ carbocyclischen Ring bilden, z.B. Cyclopopyl, n-Cyclobutyl oder n-Cyclopentyl.

Bevorzugte (substituierte-Acyloxyimino)arylacetylgruppen der obigen Formel sind diejenigen, worin $R_{140}$ Amino darstellt. Ebenfalls bevorzugt sind Gruppen, worin $R_{111}$ 4-Thiazolyl bedeutet.

(h) [[[-Substituierte-2-oxo-1-imidazolidinyl]carbonyl] amino]acryacetylgruppen der allgemeinen Formel

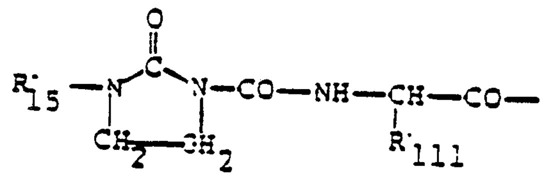

in der $R_{111}$ die obene gegebene Bedeutung hat und $R_{15}$ Wasserstoff, niederes Alkylsulfonyl, Arylmethylenamino (d.h. -N=$CHR_{111}$ worin $R_{111}$ die oben gegebene Bedeutung hat), -$COR_{16}$ (worin $R_{16}$ Wasserstoff oder gegebenenfalls durch Halogen substituiertes niederes Alkyl darstellt), eine wie unter $R_{111}$ oben definierte aromatische Gruppe oder gegebenenfalls durch einen oder mehrere der Reste Halogen, Cyan, Nitro, Amino und/oder Mercapto substituiertes niederes Alkyl darstellt.

Bevorzugte [[[3-Substituierte-2-oxo-1-imidazolidinyl] carbonyl]amino]arylacetylgruppen der obigen Formel sind diejenigen, worin $R_{111}$ Phenyl oder 2-Thienyl darstellt. Ebenfalls bevorzugt sind diejenige Gruppen, worin $R_{15}$ Wassestoff, Methylsulfonyl, Phenylmethylenamino oder 2-Furylmethylenamino darstellt.

Eine bevorzugte Gruppe der Verbindungen besitzt die Formel

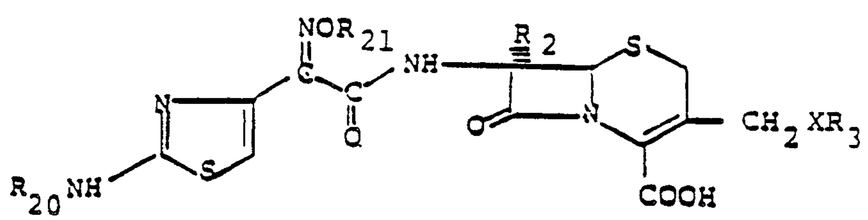 (II)

in der X, $R_2$ und $R_3$ die oben gegebene Bedeutung haben, $R_{20}$ eine Aminoschutzgruppe, z.B. Trityl oder Chloracetyl oder, vorzugsweise, Wasserstoff und $R_{21}$ Wasserstoff, niederes Alkyl oder eine Gruppe der Formel

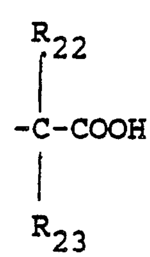

worin $R_{22}$ und $R_{23}$ die oben gegebene Bedeutung haben, darstellen.

Insbesondere bevorzugt sind Verbindungen der Formel II, worin $R_{20}$ Wasserstoff und $R_{21}$ Methyl oder eine Gruppe der Formel

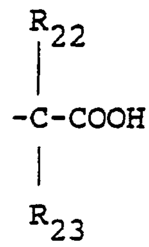

worin $R_{22}$ und $R_{23}$ Wasserstoff oder Methyl darstellt.

Eine weitere bevorzugte Gruppe der Verbindungen besitzt die Formel

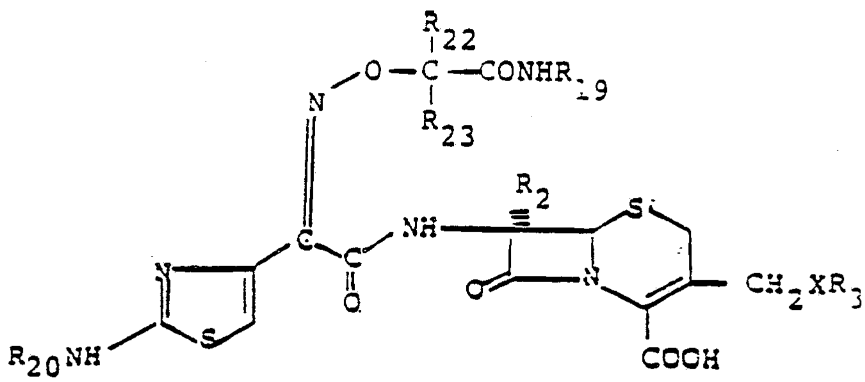

(III)

in der X, $R_2$ und $R_3$ die oben gegebene Bedeutung haben, $R_{19}$ Wasserstoff, niederes Alkyl, Amino, niederes Alkylamino, Arylamino oder Acylamino, und $R_{20}$, $R_{22}$ und $R_{23}$ die oben gegebene Bedeutung haben.

Vorzugsweise liegen die Gruppen

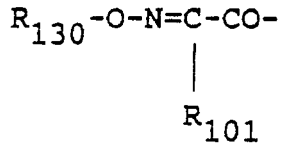

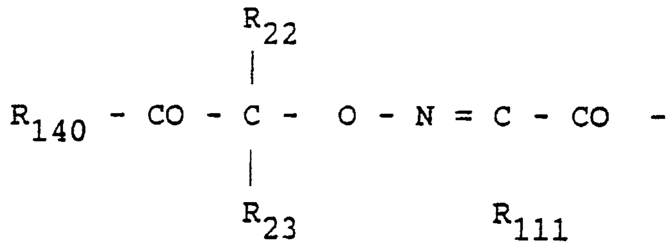

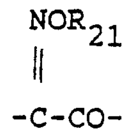

and

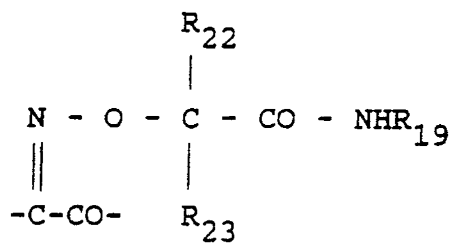

in der syn-Form vor, d.h. Z-Form, oder als Gemische, in denen die syn-Form vorweigt.

Eine weitere bevorzugte Gruppe der Verbindungen besitzt die Formel

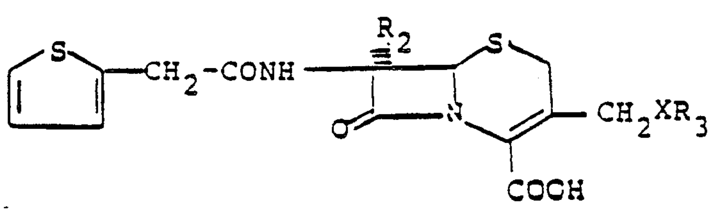

(IV)

worin X, $R_2$ und $R_3$ die oben gegebene Bedeutung haben.

Ebenfalls bevorzugt sind Verbindungen der Formel

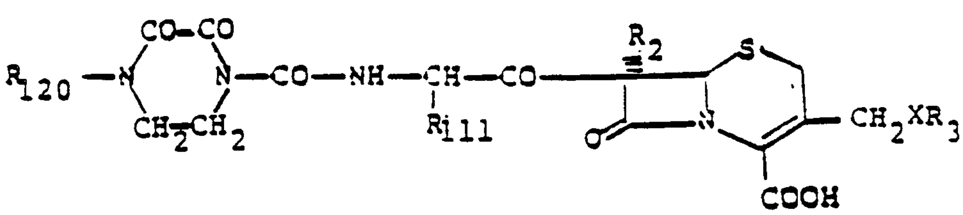

(V)

worin X, $R_2$, $R_3$, $R_{111}$ und $R_{120}$ die oben gegebene Bedeutung haben.

Als leicht hydrolysierbare Ester der Verbindungen der Formel I, sind Verbindungen der Formel I zu verstehen, deren Carboxygruppe bzw. Carboxygruppen in Form einer leicht hydrolysierbaren Estergruppe vorliegt/vorliegen. Beispiele solcher Ester, die herkömmlicher Art sein können, sind die niederen Alkanoyloxyalkylester, z.B. der Acetoxymethyl-, Pivaloyloxymethyl-, 1-Acetoxyethyl- und der 1-Pivaloyloxyethylester; die niederen Alkoxycarbonyloxyalkylester, z.B. der Methoxycarbonyloxymethyl-, 1-Ethoxycarbonyloxyethyl- und der 1-Isopropoxycarbonyloxyethylester; die Lactonylester, z.B. der Phthalidyl- und der Thiophthalidylester; die niederen Alkoxymethylester, z.B. der Methoxymethylester; und die niederen Alkanoylaminomethylester, z.B. der Acetamidomethylester. Auch andere Ester, z.B. die Benzyl- und Cyanmethylester, können brauchbar sein.

Beispiele von Salzen der Verbindungen der Formel I sind Alkalimetallsalze, wie das Natrium und das Kaliumsalz, Ammoniumsalze; Erdalkalimetallsalze wie das Calciumsalz; Salze mit organischen Basen wie die Salze mit Aminen, z.B. Salze mit N-Ethylpiperidin, Procain, Dibenzylamin, N,N′-Dibenzylethylendiamin, Alkylaminen oder Dialkylaminen, sowie Salze mit Aminosäuren, wie z.B. Salze mit Arginin oder Lysin.

Die Verbindungen der Formel I einschliesslich deren Salze und leicht hydrolysierbare Ester können hydratisiert sein. Die Hydratisierung kann im Zuge des Herstellungsverfahrens erfolgen oder allmählich als Folge hygroskopischer Eigenschaften eines zunächst wasserfreien Produktes auftreten.

Die obigen Acylderivate werden erfindungsgemäss dadurch hergestellt, dass man

a) eine Verbindung der allgemeinen Formel

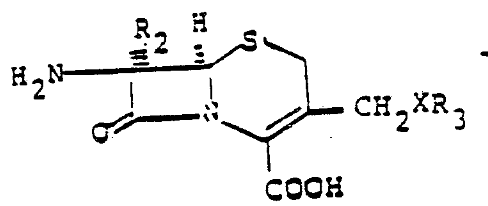

XVIII

in der X und $R_3$ die obige Bedeutung haben und die Carboxygruppe und/oder die Aminogruppe in geschützter Form vorliegen können,
mit einer Säure der Formel $R_1COOH$, worin $R_1$ die obige Bedeutung hat, oder mit einem reaktionsfähigen Derivat davon umsetzt, oder dass man

b) zur Herstellung einer Carbonsäure der Formel I einen Ester der Formel

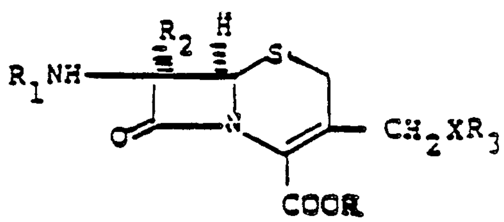

(XIX)

in der $R_1$, $R_2$ und $R_3$ die obige Bedeutung haben und R eine Carbonsäureschutzgruppe darstellt, in die Carbonsäure der Formel I überführt, oder dass man

c) zur Herstellung einer Verbindung der Formel I, worin $R_1$ einen Aminosubstituenten enthält, oder eines Esters oder Salzes davon, die Aminoschutzgruppe im Substituenten $R_{10}$ einer Verbindung der Formel

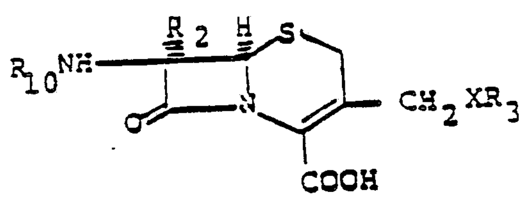

(XX)

in der $R_2$ und $R_3$ die oben gegebene Bedeutung haben und $R_{10}$ einen eine geschützte Aminogruppe enthaltenden Acylrest darstellt,
oder eines ihrer Ester oder Salze abspaltet, oder dass man

(d) zur Herstellung einer Verbindung der Formel I, worin X -OCO- oder -SCO- darstellt, eine Verbindung der Formel

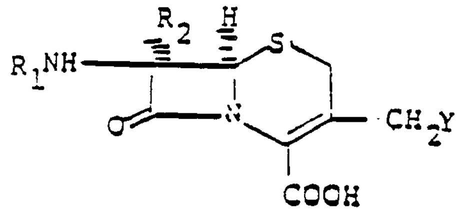

(XXI)

in der $R_1$ die obige obige Bedeutung hat und Y eine austretende Gruppe darstellt, oder einen Ester oder Salz davon mit einem Salz der Carbonsäure $R_3COQH$, worin $R_3$ die obige Bedeutung hat und Q O oder S darstellt, umsetzt, oder dass man

(e) zur Herstellung einer Verbindung der Formel I, worin X -OCO- oder -OCONH- darstellt, eine Verbindungen der Formel

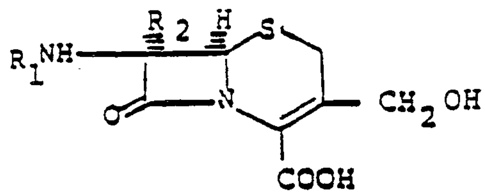

(XXII)

in der $R_1$ und $R_2$ die obige Bedeutung haben, oder einen Ester oder Salz davon mit einer Verbindung der Formel $R_3COZ$-, $R_3NHCOZ$ oder $R_3NCO$, worin $R_3$ die obige Bedeutung hat und Z eine acylaktivierende Gruppe darstellt, umsetzt, oder dass man

(f) zur Herstellung einer Verbindung der Formel I, worin X -NHCO- darstellt, eine Verbindung der Formel

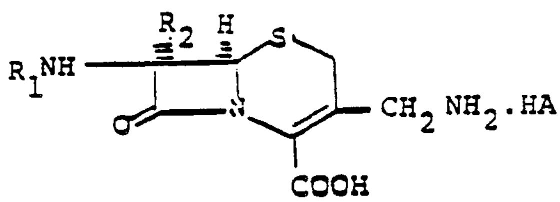

XXIII

in der $R_1$ und $R_2$ die obige Bedeutung haben und A das Anion einer Säure darstellt, oder einen Ester oder Salz davon mit einer Säure der Formel $R_3$-COOH, worin $R_3$ die obige Bedeutung hat, umsetzt, oder dass man

(g) zur Herstellung eines leicht hydrolysierbaren Esters einer Verbindung der Formel I eine Carbonsäure der Formel I einer entsprechenden Veresterung unterwirft, oder dass man

(h) zur Herstellung von Salzen oder Hydraten einer Verbindung der Formel I bzw. von Hydraten dieser Salze eine Verbindung der Formel I in ein Salz oder Hydrat bzw. in ein Hydrat dieses Salzes überführt.

Die Umsetzung der Verbindungen XVIII mit den Säuren $R_1COOH$ oder ihren reaktionsfähigen Derivaten gemäss Variante (a) kann in an sich bekannter Weise durchgeführt werden. Die Carboxygruppe der Verbindungen XXVIII kann wahlweise geschützt sein, z.B. durch Veresterung zu einem leicht spaltbaren Ester, wie dem Silylester, z.B. dem Trimethylsilylester. Es kommen auch die oben erläuterten, leicht hydrolysierbaren Ester in Betracht. Die Carboxygruppe kann auch durch Salzbildung mit einer anorganischen oder tertiären organischen Base, wie Triethylamin, geschützt werden. In Gruppen $R_1$ vorhandene Aminogruppen können geschützt sein. Mögliche Schutzgruppen sind beispielsweise sauer-hydrolytisch abspaltbare Schutzgruppen wie z.B. t-Butoxycarbonyl oder Trityl, oder auch basisch-hydrolytisch abspaltbare Schutzgruppen wie z.B. Trifluoracetyl. Bevorzugte Schutzgruppen sind Chlor-, Brom- und Jodacetyl, insbesondere Chloracetyl. Letztere Schutzgruppen können durch Behandeln mit Thioharnstoff abgespalten werden. Die 7-Aminogruppe in Verbindung XVIII kann geschützt sein, z.B. durch eine Silylschutzgruppe wie

z.B. Trimethylsilyl.

Als reaktionsfähige funktionelle Derivate von Säure der Formel $R_1COOH$ kommen z.B. Halogenide, d.h. Chloride, Bromide und Fluoride; Azide; Anhydride, insbesondere gemischte Anhydride mit stärkeren Säuren; reaktionsfähige Ester, wie z.B. N-Hydroxysuccinimidester; und Amide, z.B. Imidazolide in Betracht.

Die Umsetzung der 7-Aminoverbindung der Formel XVIII mit der Säure der Formel $R_1COOH$ oder einem reaktionsfähigen funktionellen Derivat davon kann in an sich bekannter Weise durchgeführt werden. So kann man z.B. eine freie Säure der Formel $R_1COOH$ mit einem der erwähnten Ester entsprechend Formel XVIII mittels eines Carbodiimids, wie Dicyclohexylcarbodiimid, in einem inerten Lösungsmittel, wie Essigester, Acetonitril, Dioxan, Chloroform, Methylenchlorid, Benzol oder Dimethylformamid kondensieren und anschliessend die Estergruppe abspalten. Anstelle von Carbodiimiden lassen sich als Kondensationsmittel auch Oxazoliumsalze z.B. N-Ethyl-5-phenyl-isoxazolium-3′-sulphonat, verwenden.

Nach einer anderen Ausführungsform setzt man ein Salz einer Säure der Formel XVIII, z.B. ein Trialkylammoniumsalz, wie das Triethylammoniumsalz, mit einem reaktionsfähigen funktionellen Derivat einer Säure der Formel $R_1COOH$ wie oben erwähnt in einem inerten Lösungsmittel, z.B. einem der oben genannten Lösungsmittel, um.

Nach einer weiteren Ausführungsform wird ein Säurehalogenid, vorzugsweise das Chlorid einer Säure der Formel $R_1COOH$ mit dem Amin der Formel XVIII umgesetzt. Die Umsetzung erfolgt vorzugsweise in Gegenwart eines säurebindenden Mittels z.B. in Gegenwart von wässrigem Alkali vorzugsweise Natriumhydroxid, oder auch in Gegenwart eines Alkalimetallcarbonats, wie Kaliumcarbonat, oder in Gegenwart eines nieder-alkylierten Amins, wie Triethylamin. Als Lösungsmittel wird vorzugsweise Wasser verwendet, gegebenenfalls im Gemisch mit einem inerten organischen Lösungsmittel, wie Tetrahydrofuran oder Dioxan. Es kann auch in einem aprotischen organischen Lösungsmittel, wie z.B. Dimethylformamid, Dimethylsulfoxid oder Hexamethylphosphorsäuretriamid, gearbeitet werden. Bei Verwendung von silylierten Ausgangsverbindungen der Formel XVIII wird in wasserfreiem Medium gearbeitet.

Bevorzugte Varianten der Acylierung, wobei eine vorhandene Aminogruppe in der Gruppe $R_1$ nicht geschützt zu sein braucht, betrifft die Verwendung eines 2-Benzthiazolylthioesters oder eines 1-Hydroxybenzotriazolesters der Säure $R_1COOH$. Vorzugsweise kann der 2-Benzthiazolylester mit einer Verbindung XVIII in einem inerten organischen Lösungsmittel, wie z.B. einem chlorierten Kohlenwasserstoff, z.B. Methylenchlorid, in Aceton, Essigester oder in einer Mischung solcher Lösungsmittel mit Wasser umgesetzt werden. Der 1-Hydroxybenzotriazolester kann in der Weise verwendet werden, dass man die Säure $R_1COOH$ mit 1-Hydroxybenzotriazol und einem Carbodiimid, insbesondere N,N′-Dicyclohexylcarbodiimid oder N,N′-Diisopropylcarbodiimid, in einem inerten organischen Lösungsmittel, vorzugsweise Methylenchlorid, Dimethylformamid, Tetrahydrofuran, Acetonitril oder Essigester, umsetzt.

Die erfindungsgemässe Umsetzung der 7-Aminoverbindung der Formel XVIII mit einer Säure der Formel $R_1COOH$ oder einem reaktionsfähigen Derivat hiervon kann zweckmässig bei einer Temperatur zwischen etwa -40 °C und +60 °C, z.B. bei Zimmertemperatur, durchgeführt werden.

Esterschutzgruppen der gemäss Variante (b) des erfindungsgemässen Verfahrens verwendeten Ester der Formel XIX sind vorzugsweise solche, welche unter milden Bedingungen in die freie Carboxygruppe umgewandelt werden können, z.B. t-Butyl, p-Nitrobenzyl, Benzhydryl, Allyl, etc.. Auch die oben erläuterten Reste eines leicht hydrolysierbaren Esters können verwendet werden. Die Esterschutzgruppen werden z.B. wie folgt abgespalten: p-Nitrobenzyl durch Hydrolyse in Gegenwart von Natriumsulfid bei (oder unterhalb) 0 °C bis Zimmertemperatur in einem Lösungsmittel, wie z.B. Dimethyl-formamid (vorzugsweise wässrig); t-Butyl durch Umsetzung mit Trifluoressigsäure in Gegenwart von Anisol bei etwa 0 °C bis Zimmertemperatur mit oder ohne zusätzliches Lösungsmittel, wie z.B. Methylenchlorid; Allyl durch Palladium-(O)-katalysierte Transallylierung in Gegenwart eines Natrium- oder Kaliumsalzes der 2-Ethylcapronsäure, siehe z.B. J. Org. Chem., 1982, 47, 587.

Durch Abspaltung der Aminoschutzgruppe im Substituenten $R_{10}$ einer Verbindung der Formel XX (oder eines Esters oder Salzes davon) gemäss Variante (c) des erfindungsgemässen Verfahrens erhält man entsprechende Verbindungen der Formel I (sowie Ester und Salze davon) mit einer freien Aminogruppe. Mögliche Aminoschutzgruppen sind diejenigen, welche in der Peptidchemie verwendet werden, z.B. Alkoxycarbonylgruppen, z.B. t-Butoxycarbonyl, etc., substituierte Alkoxycarbonylgruppen, z.B. Trichlorethoxycarbonyl, etc., substituierte Aralkoxycarbonylgruppen, z.B. p-Nitrobenzyloxycarbonyl, Aralkylgruppen, z.B. Trityl oder Benzhydryl, oder Halogenalkanoylgruppen wie z.B. Chloracetyl, Bromacetyl, Jodacetyl oder Trifluoracetyl.

Bevorzugte Aminoschutzgruppen sind t-Butoxycarbonyl (t-BOC) und Trityl.

Die Aminoschutzgruppppen sind beispielsweise durch saure Hydrolyse abspaltbar (z.B. t-Butoxycarbonyl oder Trityl) oder auch durch basische Hydrolyse abspaltbar (z.B. Trifluoracetyl). Die Chlor-, Brom- und Jodacetylgruppen können durch Behandeln mit Thioharnstoff abgespalten werden.

Durch saure Hydrolyse abspaltbare Schutzgruppen werden vorzugsweise mit Hilfe einer niederen Alkancarbonsäure, die gegebenenfalls halogeniert sein kann, entfernt. Insbesondere verwendet man Ameisensäure oder Trifluoressigsäure. Die Temperatur ist in der Regel Raumtemperatur, obwohl auch leicht erhöhte bzw. leicht erniedrigte Temperaturen angewendet werden können z.B. im Bereiche von etwa 0° C bis +40° C. Alkalisch abspaltbare Schutzgruppen werden im allgemeinen mit verdünnten wässrigen Laugenbei 0° C bis +30° C hydrolysiert. Die Chlor-, Brom- und Jodacetylschutzgruppen können mittels Thioharnstoff in saurem, neutralem oder alkalischem Milieu bei etwa 0° C bis 30° C abgespalten werden.

Die erfindungsgemässe Umsetzung der Verbindungen XXI bzw. ihrer Ester oder Salze mit den Salzen der Verbindungen $R_3COQH$ gemäss Variante (d) des erfindungsgemässen Verfahrens wird vorzugsweise in einem nicht-hydroxylierten Lösungsmittel durchgeführt, wie z.B. Dimethylformamid, Methylenchlorid oder N,N′-Dimethylacetamid. Andere nicht-hydroxylierte Lösungsmittel können ebenfalls verwendet werden. Geeignete Salze der Verbindungen $R_3COQH$ sind z.B. wie Natrium-, Kalium-, Cäsium-, Tetrabutylammonium- oder Tetramethylammoniumsalze. Hal ist ein Halogen, vorzugsweise Brom oder Jod. Die Umsetzung kann ebenfalls in einer wässrigen Bicarbonatlösung mit einer der besagten Salze der Verbindung $R_3COQH$ duchgeführt werden. Die Umsetzung wird vorzugsweise bei etwa 0° C bis 80° C, vorzugsweise bei Zimmertemperatur, durchgeführt werden.

Die Umsetzung der Verbindungen XXII mit Verbindungen $R_3COZ$, $R_3NHCOZ$ oder $R_3NCO$ gemäss Variante (e) des erfindungsgemässen Verfahrens wird nachstehend in den Schemas II und V veranschaulicht.

Die erfindungsgemässe Umsetzung der Verbindungen XXIII bzw. ihrer Ester und Salze mit den Säuren $R_3COOH$ gemäss Variante (f) des erfindungsgemässen Verfahrens wird nachstehend in Schema IV veranschaulicht.

Zur Herstellung der leicht hydrolysierbaren Ester der Carbonsäuren der Formel I gemäss Variante (g) des erfindungsgemässen Verfahrens wird die Carbonsäure vorzugsweise mit dem entsprechenden, die Estergruppe enthaltenden Halogenid, bevorzugt mit dem Jodid, umgesetzt. Die Reaktion kann mit Hilfe einer Base, z.B. einem Alkalimetallhydroxid oder -carbonat oder einem organischen Amin, wie Triethylamin, beschleunigt werden. Die Veresterungreaktion wird vorzugsweise in einem inerten organischen Lösungsmittel, wie Dimethylacetamid, Hexamethylphosphorsäuretriamid, Dimethylsulfoxid oder, vorzugsweise, Dimethylformamid durchgeführt. Die Temperatur liegt vorzugsweise im Bereiche von etwa 0° C-40° C.

Die Herstellung der Salze und Hydrate der Verbindungen der Formel I bzw. der Hydrate dieser Salze gemäss Variante (h) des erfindungsgemässen Verfahrens kann in an sich bekannter Weise erfolgen, z.B. durch Umsetzen der Carbonsäure der Formeli oder eines Salzes davon mit der äquimolaren Menge der gewünschten Base, zweckmässig in einem Lösungsmittel, wie Wasser, oder in einem organischen Lösungsmittel, wie Ethanol, Methanol, Aceton und dergleichen. Die Temperatur der Salzbildung ist nicht kritisch; sie liegt im allgemeinem bei Raumtemperatur, kann aber auch leicht darüber oder darunter, etwa im Bereiche von 0° C-+50° C, liegen.

Die Herstellung der Hydrate erfolgt someist automatisch im Zuge des Herstellungsverfahrens oder als Folge hygroskopischer Eigenschaften eines zunächst wasserfreien Produktes. Zur gezielten Hestellung eines Hydrats kann ein ganz oder teilweise wasserfreies Produkt (Carbonsäure der Formel I oder eines ihrer Salze) einer feuchten Atmosphäre, z.B. bei etwa +10° C bis +40° C, ausgesetzt werden.

In den folgenden Reaktionsschemas kann das Symbol $R_3$ in Formel X (Schema I), Formel XIII (Schema III), Formel XVI (Schema IV) und Formel XVII (Schema V) Hydroxyaryl oder entsprechende Alkanoylester davon bedeuten. Im Falle der Alkanoylester kann die Umwandlung in entspechendes Hydroxyaryl mit Hilfe eines Alkohols und einer Base, vorzugsweise Methanol und Natriumbicarbonat, erfolgen.

Die folgenden Reaktionsschemas veranschaulichen das Verfahren zur Herstellung der erfindungsmässen Produkte:

Schema I

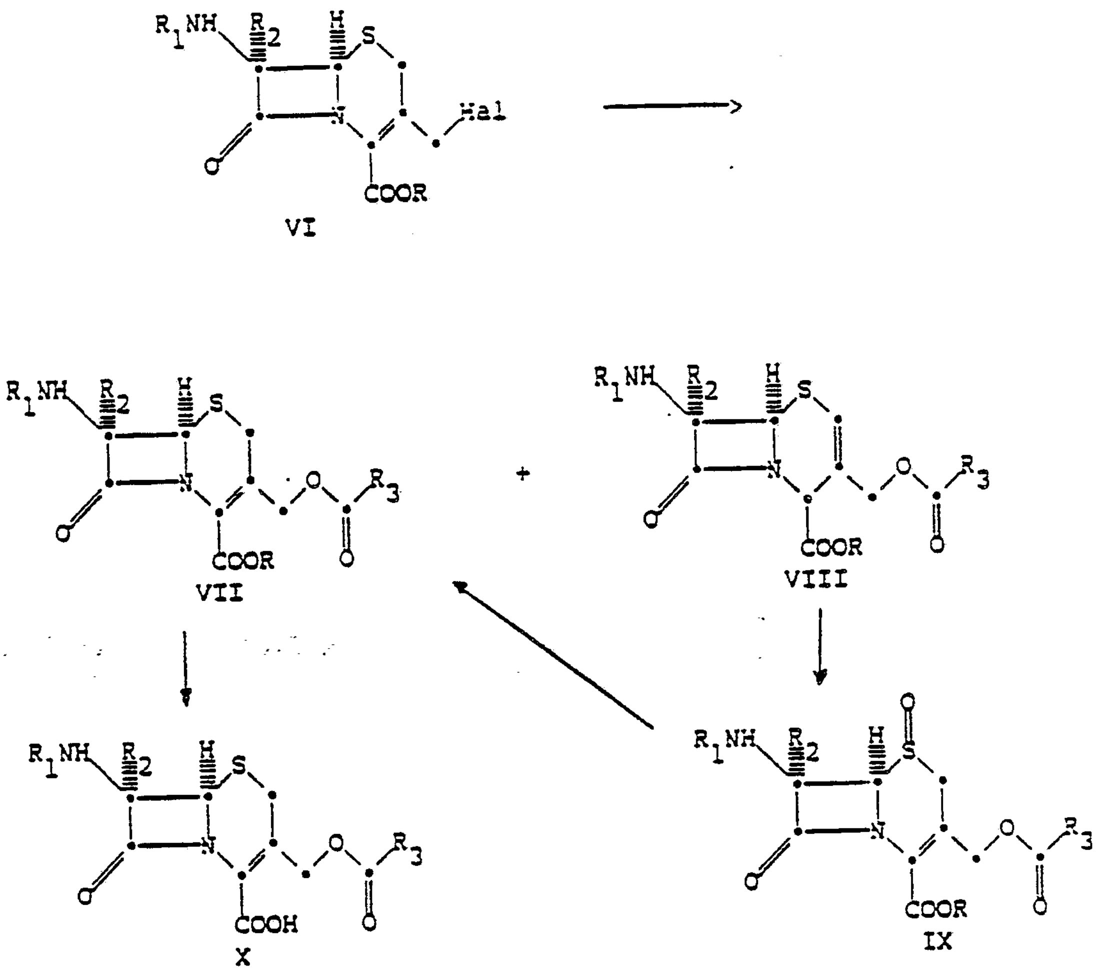

VI

VII + VIII

X

IX

worin $R_1$, $R_2$ und $R_3$ die oben gegebene Bedeutung haben.

Je nach Wahl der Esterschutzgruppe R und des Halogens Hal erhält man in bezug auf die Doppelbildung im Cephemring ein Δ3- oder Δ2-Isomeres der Formel VII bzw. VIII. Ein erhaltenes Δ3/Δ2-Gemisch kann notwendigenfalls durch Herstellung des Sulfoxids der Formel IX und anschliessende Reduktion dieser Verbindung oder Trennung der beiden Komponenten zum gewünschten Δ3-Isomeren gereinigt werden.

Erläuterungen zum Schema I:

<u>VI → VII + VIII</u>

Die Verbindung der Formel VI ist eine bekannte Verbindung oder ist in analoger Weise erhältlich (siehe z.B. die U.S. Patentschriften Nos. 4,406,899 und 4,266,049). Sie wird mit einem Salz des gewählten Chinolons umgesetzt. Die Umsetzung erfolgt wie oben für die Verfahrensalternative (d) beschrieben.

VII → X

Die Esterschutzgruppe R der Verbindung der Formel VII wird anschliessend abgespalten wie oben für die Verfahrensalternative (b) beschrieben, wobei eine Carbonsäure der Formel X oder ein Gemisch davon mit dem Δ2-Isomeren erhalten wird.

VIII → IX

Falls die Doppelbildung des Δ3-Isomeren isomerisiert wird, wird die so erhaltene Verbindung oxidiert, z. B. mit einer Persäure, wie z.B. m-Chlorperbenzoesäure, in einem Lösungsmittel, wie z.B. Methylenchlorid, bei einer Reaktionstemperatur von etwa -20° C bis 40° C, vorzusgweise bei etwa 0° C.

IX → VII

Die Verbindung der Formel IX wird anschliessend in das gewünschte Endprodukt der Formel VII reduziert, wobei eine Vielzahl von Verfahrensmöglichkeiten vorliegen, z.B. Behandlung mit Phosphortrihalogenid in Dimethylformamid oder Trifluoressigsäureanhydrid in Gegenwart von Natriumjodid in Aceton/Methylenchlorid. Die Reaktionstemperatur für diese Umsetzungen liegt z.B bei etwa 0.° C bis -20° C, wobei 0° C bevorzugt ist.

## Schema II

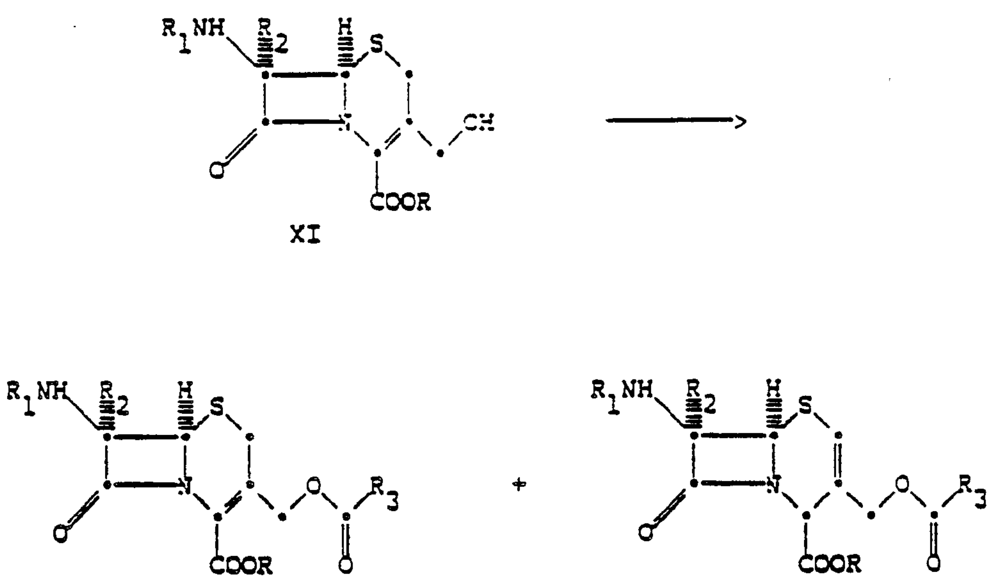

XI

VII + VIII

worin R, $R_1$, $R_2$ und $R_3$ die oben gegebene Bedeutung haben.

Erläuterungen zu Schema II:

XI → VII + VIII

Die Verbindung XI ist bekannt bzw. herstellbar gemäss J. Antibiotics, 1981, 34, 1300; sie wird umgesetzt mit einer Verbindung der Formel $R_3$-CO-Z, in der Z eine acylaktivierende Gruppe darstellt. Bevorzugte acylaktivierende Gruppen Z sind Halogen,

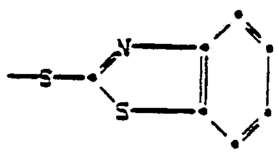
,
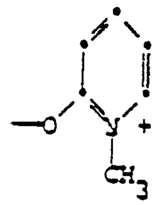
,
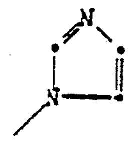

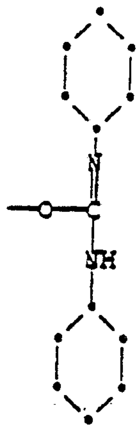
, und
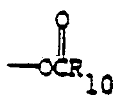

(worin $R_{10}$ $C_1$-$C_3$-Alkyl darstellt).

Diese Reaktion wird in einem Lösungsmittel durchgeführt, wie z.B. in Methylenchlorid, bei einer Temperatur von etwa -20° C bis +100° C, vorzugsweise bei etwa 25° C. Die Doppelbindung im Cephemring des erhaltenen Produktes kann in Δ3- oder in Δ2-Stellung des erhaltenen Produktes vorliegen. Dieses gemischte Produkt kann in ein einziges Isomeres übergeführt werden, und zwar wie oben für Schema I beschrieben, oder durch Oxidation mit einer Persäure zum Sulfoxid IX und anschliessende Reduktion zum geschützten Ester VII.

## Schema III

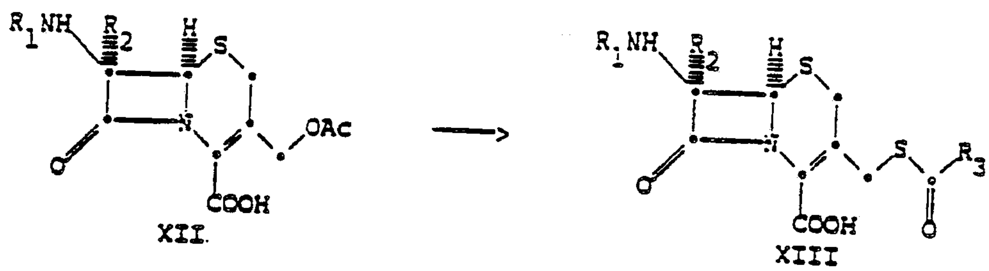

XII XIII

worin $R_1$, $R_2$ und $R_3$ die oben gegebene Bedeutung haben und Ac $CH_3CO$- bedeutet.

Erläuterungen zum Schema III:

XII→XIII

Die Verbindung XII bzw. ein Salz davon wird vorzugsweise in wässrigem Natriumlicarbonat mit einer Formel der Verbindung ASCO-$R_3$ umgesetzt, worin $R_3$ die obige Bedeutung hat und A ein Kation, vorzugsweise Natrium, darstellt. Die Umsetzung wird vorzugsweise bei etwa 25-100 °C, insbesondere bei 40-60 °C, durchgeführt.

Die Verbindung XIII kann ebenfalls nach Schema I hergestellt werden, wobei die gewählte Säure die Formel $R_3COSH$ besitzt.

## Schema IV

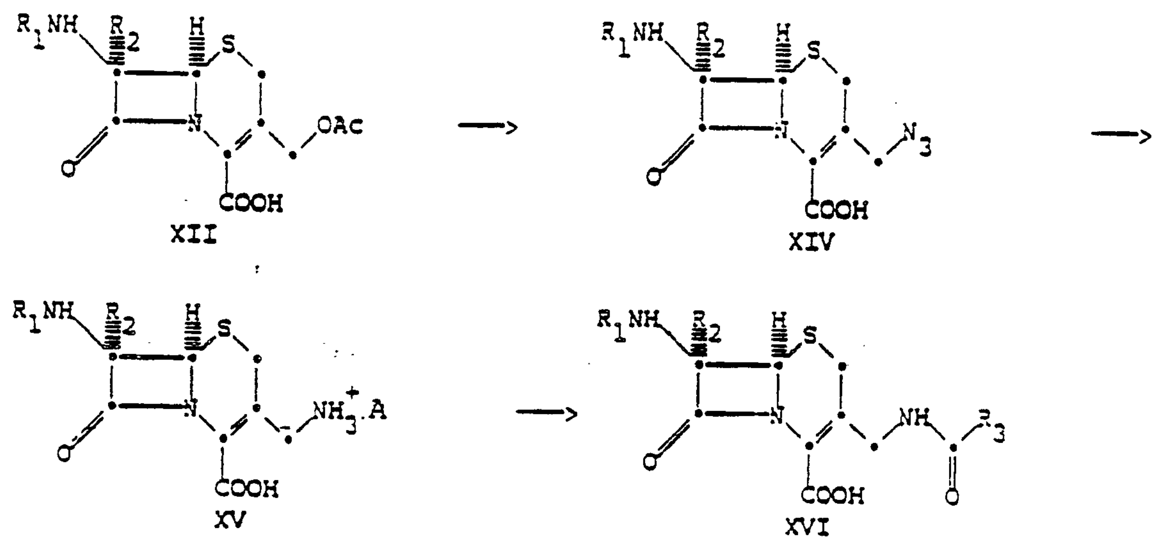

XII XIV XV XVI

worin Ac, A, $R_1$, $R_2$ und $R_3$ die oben gegebene Bedeutung haben.

Erläuterungen zum Schema IV:

XII → XIV

Die Verbindung XII wird mit einem Azidsalz (z.B. Natrium- oder Kaliumsalzes in einer wässrigen Base wie Natriumlicarbonat, bei einer Temperatur von etwa 25-100 °C, insbesondere 40-60 °C, umgesetzt.

XIV → XV

Das erhaltene Azid (XIV) wird mit Wasserstoff und einem Katalysator, wie Palladium oder Platin, oder vorzugsweise mit metallischem Zinn und Salzsäure bei einer Temperatur von etwa 0-50 °C, vorzugsweise 20-35 °C, reduziert.

XV → XVI

Die Amidbindung wird mit Hilfe von Methoden gängig in der Peptidchemie gebildet (siehe z.B. Synthesis, 1972, Seite 453) vorzugsweise durch Verwendung von Dicyclohexylcarbodiimid und N-Hydroxybenzotriazol oder Triphenylphosphin und einem Diaryldisulfid (z.B. 2,2'Dibenzothiazolyldisulfid), bei einer Temperatur von etwa 0-50° C, vorzugsweise 20-35° C, und in einem inerten Lösungsmittel, z.B. Dimethylformamid oder Dimethylsulfoxid.

Schema V

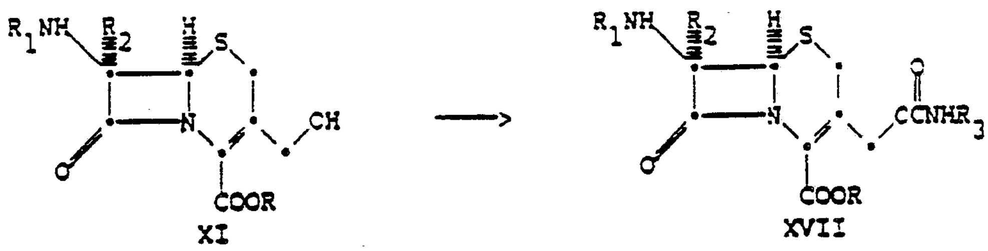

XI

XVII

worin R, $R_1$, $R_2$ und $R_3$ die oben gegebene Bedeutung haben.

Erläuterung zum Schema V:

XI → XVII

Die Verbindung XI wird mit einer Verbindung der Formel $O=C=N-R_3$ oder $ZCONHR_3$ umgesetzt, mit oder ohne Zusatz einer Base, z.B. Pyridin, Picolin oder Lutidin, wobei $R_3$ die oben gegebene Bedeutung hat und Z eine acylaktivierende Gruppe (wie in Schema II beschrieben) darstellt. Die Umsetzung wird in einem Lösungsmittel, z.B. Methylenchlorid und bei einer Temperatur von O-60° C, vorzugsweise 25° C, durchgeführt. Die Verbindung XVII kann anschliessend gespalten werden, gewünschtenfalls mit Hilfe des in Schema I beschrieben Verfahrens.

Verbindungen der Formel I mit den Gruppen

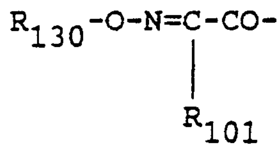

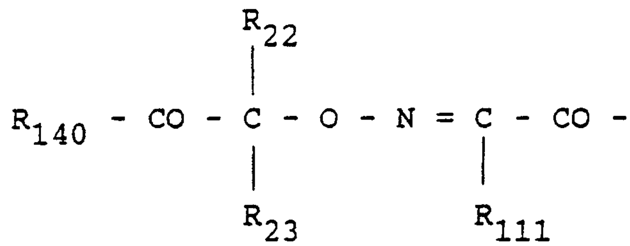

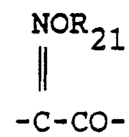

und

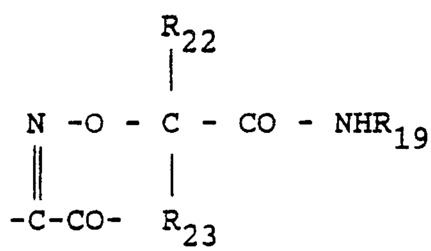

liegen vorzugsweise als syn-Form vor. Diese syn-Formen können erhalten werden durch Verwendung von Ausgangsmaterialien, worin diese syn-Form bereits vorliegt. Wahlweise kann ein erhaltenes syn/anti-Gemisch in üblicher Weise in entsprechenden syn- und anti-Formen aufgetrennt werden, beispielsweise durch Umkristallisation oder durch chromatographische Methoden unter Verwendung eines geeigneten Lösungsmittels bzw. Lösungsmittelgemisches.

Die Verbindungen der Formel I, sowie die Salze, Ester sowie Hydrate dieser Verbindungen sind antibiotisch, insbesondere bakterizid wirksam. Sie können als Agenzien zur Bekämpfung von bakteriellen Infektionen (einschliesslich Harn- und Atemweginfektionen) bei Säugern z.B. Hunden, Katzen, Pferden usw., sowie beim Menschen verwendet werden. Diese Cephalosporine sind wirksam gegen einen breiten Bereich sowohl gram-negativer als auch gram-positiver Bakterien.

Die in vitro Wirksamkeit der erfindungsgemässen Verbindungen gegen eine Vielzahl gram-positiver und gram-negativer Mikroorganismen, ausgedrückt als Mindesthemmkonzentration in Mikrogramm/ml und ermittelt unter Verwendung der Reihenverdünnungsmethode (in Flüssigmedium) ist wie folgt:

Verbindung A:

[6R-[6 alpha,7 beta(Z)]]-7-[[(2-Amino-4-thiazolyl)(methoxyimino)acetyl]amino]-3-[[(3,4-dihydroxybenzoyl)-oxy]methyl]-8-oxo-5-thia-1-azabicyclo[4.2.0]oct-2-en-2-carbonsäure Mononatriumsalz

Verbindung B:

[6R-[6 alpha,7 beta(Z)]]-3-[[[3,4-bis(acetyloxy)benzyol)]oxy]methyl]-7-[[[2-amino-4-thiazolyl][(2-amino-2-oxoethoxy)imino]acetyl]amino]-8-oxo-5-thia-1-azabicyclo[4.2.0]oct-2-en-2-carbonsäure-Mononatriumsalz.

Verbindung C:

[6R-[6 alpha,7 beta(Z)]]-7-[[(2-Amino-4-thiazolyl)(1-carboxy-1-methylethoxy)imino]acetyl]amino]-3-[[(3,4-dihydroxybenzoyl)oxy]methyl]-8-oxo-5-thia-1-azabicyclo[4.2.0]oct-2-en-2-carbonsäure-Dinatriumsalz

Verbindung D:

[6R-[6 alpha,7 beta(R*)]]-7-[[[[(4-Ethyl-2,4-dioxo-1-piperazinyl)carbonyl]amino]phenylacetyl]amino]-3-[[-(3,4-dihydroxy)benzoyl]oxy]methyl]-8-oxo-5-thia-1-azabicyclo[4.2.0]oct-2-en-2-carbonsäure-Mononatriumsalz

TABELLE 1

| In vitro MHK (µg/ml) Agar Diffusionsmethode | |
|---|---|
| Kultur | Verbindung A |
| P. aeruginosa 56 | 0.98 |
| P. vulgris 101N | 0.06 |
| E. coli 1269B | 0.12 |
| K. pneumoniae 369 | 0.49 |
| S. marcescens SM | 0.98 |
| S. aureus 1059B | 7.8 |

TABELLE 2

| In vitro MHK (µg/ml) Agar Vedünnungsmethode | |
|---|---|
| Kultur | Verbindung B |
| E. coli 48 | 0.031 |
| K. pneumoniae A | 0.125 |
| E. cloacae 9570A | 1 |
| E. cloacae P99 | 32 |
| P. vulgaris ATCC 6380 | 0.016 |
| P. mirabilis | 0.063 |
| S. marcescens SM | 1 |
| P. aeruginosa 130 | 4 |
| P. aeruginosa 185/H | 2 |
| P. aeruginosa Stone 130 | 4 |
| S. aureus Giorgid | 8 |
| S. aureus Smith | 4 |
| S. aureus 95 | 64 |
| S. aureus 1059B | 4 |
| S. aureus ATCC 25923 | 4 |

TABELLE 3

| In vitro MHK (µg/ml) Flüssigkeitsverdünnungsmethode | | | |
|---|---|---|---|
| Kultur | Verbindungen | | |
| | A | C | D |
| P. aeruginosa B | 2 | 0.125 | 0.25 |
| P. aeruginosa Stone 130 | 2 | 0.125 | 0.5 |
| P. aeruginosa ATCC 27853 | 8 | 32 | 0.5 |
| P. aeruginosa 8710 | 2 | 0.063 | 0.125 |
| P. aeruginosa 503-56 | 4 | 0.25 | 4 |
| P. aeruginosa 8780 | 0.5 | <0.008 | 0.016 |
| P. aeruginosa 6148B | 2 | 0.031 | 0.125 |
| P. aeruginosa 765 | 4 | 8 | 128 |
| P. aeruginosa 185/H | 0.016 | 0.031 | 0.125 |
| P. aeruginosa 1973E | 4 | 2 | 4 |
| P. aeruginosa 5700 | 8 | 2 | 4 |
| P. aeruginosa K77/WT | 1 | 0.63 | 0.25 |
| P. aeruginosa K77/61 | 0.25 | <0.008 | 0.016 |

Zur Bekämpfung einer bakteriellen Infektion in einem Säuger kann eine erfindungsgemässe Verbindung mit einer Tagesdosis von etwa 5 bis etwa 500 mg/kg, vorzugsweise etwa 10 bis 100 mg/kg, insbesondere etwa 10 bis 55 mg/kg, behandelt werden.

Sämtliche Verabreichungsarten, welche bisher für die Penicillin- und Cephalosporintherapie in Frage kommen, sind ebenfalls für die erfindungsgemässen neuen Cephalosporine verwendbar. Verabreichungsarten sind demnach beispielsweise orale, z.B. durch Tabletten und Kapseln, parenterale, z.B. intravenöse, oder intramuskulare und enterale Administrierungen.

Die erfindungsgemässe Produkte können als Heilmittel z.B. in Form pharmazeutischer Präparate Verwendung finden, welche sie oder ihre Salze in Mischung mit einem für die enterale oder parenterale Applikation geeigneten pharmazeutischen, organischen oder anorganischen inerten Trägermaterial wie z.B. Wasser, Gelatine, Gummi arabicum, Milchzucker, Stärke, Magnesiumstearat, Talk, pflanzliche Oele, Polyalkylenglycole, Vaseline usw. enthalten. Die pharmazeutischen Präparate können in fester Form, z.B. als Tabletten, Dragées, Suppositorien, Kapseln oder in flüssiger Form, z. B. als Lösungen, Suspensionen oder Emulsionen, vorliegen. Gegebenenfalls sind sie sterilisiert und/oder enthalten Hilfsstoffe, wie Konservierungs-, Stabilisierungs-, Netz- oder Emulgiermittel, Salze zur Veränderung des osmotischen Druckes, Anaestetika oder Puffer. Sie können auch noch andere therapeutisch wertvolle Stoffe enthalten. Die Carbonsäuren der Formel I und ihre Salze bzw. Hydrate kommen vorzugsweise für die parenterale Applikation in Betracht und werden zu diesem Zweck bevorzugt als Lyophilisate oder Trockenpulver zur Verdünnung mit üblichen Agenzien, wie Wasser und isotonischen Kochsalzlösung, Lösungsmittelvermittler, wie Propylenglykol, zubereitet. Die leicht hydrolysierbaren Ester der Formel I kommen auch für die enterale Verabreichung in Betracht.

## Beispiel 1

### Herstellung von [6R-[6 alpha, 7 beta(Z)]]-3-(Acetoxymethyl)-7-amino-8-oxo-5-thia-1-azabicyclo[4.2.0]oct-2-en-2-carbonsäure-1,1-dimethylethylester

200 ml eiskaltes Dioxan werden mit 10 ml konzentrierter Schwefelsäure und anschliessend mit 21,78 g (80,0 mmol) 7-Aminocephalosporansäure versetzt. Das Gemisch wird auf -50 °C abgekühlt und mit 50 ml flüssigem Isobutylen (getrennt in einer Argonatmosphäre kondensiert). Das Gemisch wird in einer verschlossenen Druckflasche bei etwa 1,3 - 1,4 kg/cm$^2$ 12 Stunden gerührt, anschliessend auf -50 °C gekühlt und die Flasche geöffnet. Der Inhalt wird langsam in 100 g Natriumbicarbonat in 1500 ml Wasser unter

Rühren gegossen. Die Lösung wird dreimal mit je 500 ml Ethylacetat extrahiert und die vereinigten Extrakte mit Salzlauge gewaschen und über Magnesiumsulfat getrocknet. Nach Entfernen des Lösungsmittels unter vermindertem Druck erhält man ein gelb-oranges Oel. Nach dem Verreiben mit Petroleumether (vom Siedepunkt 30°C - 60°C) erhält man einen Feststoff, der filtriert und getrocknet wird. Der in der Ueberschrift angegebene Ester wird ohne weitere Reinigung verwendet.

NMR ($CDCl_3$): 1.55 (S) 9H (t-bu); 2.07 (s) 3H (OAc); 3.33 3.54 (d von d, J = 10Hz) 2H ($CH_2S$); 4.75, 5.02 (d von d, J = 20Hz) 2H ($CH_2O$); 4.72 (d, J = 10Hz) 1H (C6); 4.91 (d, J = 10Hz) 1H (C7).

## Beispiel 2

Herstellung von [6R-[6 alpha, 7 beta(Z)]]-3-(Acetoxymethyl)-7-[[(methoxyimino)-[2-(tritylamino)-4-thiazolyl]-acetyl]-amino]-8-oxo-5-thia-1-azabicyclo[4.2.0]oct-2-en-2-carbonsäure-1,1-dimethylethylester

Eine Aufschlämmung von 48 g (0,1 mol) 2-[2-(Tritylamino)thiazol-4-yl]-2-methoxyiminoessigsäure in 750 ml wasserfreiem Methylenchlorid wird unter Rühren mit 13,94 ml (0,1 mol) Triethylamin versetzt und das Gemisch bis zur Bildung einer klaren Lösung (etwa 45 min.) gerührt. Nach Zugabe von 20,6 g (0,1 mol) N,N-Dicyclohexylcarbodiimid und 13,5 g (0,1 mol) 1-Hydroxybenzotriazol wird das Gemisch bei Zimmertemperatur 2 Stunden gerührt. Nach Zugabe von 32,8 g (0,1 mol) [6R-[6 alpha, 7 beta(Z)]]-3-(Acetoxymethyl)-7-amino-8-oxo-5-thia-1-azabicyclo[4.2.0]oct-2-en-2-carbonsäure-1,1-dimethylethylester (aus Beispiel 1) in 250 ml wasserfreiem Methylenchlorid wird die erhaltene Lösung 16 Stunden bei Zimmertemperatur gerührt. Der erhaltene Niederschlag wird abfiltriert und das Filtrat nacheinander mit 2 x 250 ml gesättigter wässriger Natriumlicarbonatlösung und 2 x 250 ml gesättigter wässriger Natriumchloridlösung gewaschen und anschliessend über Natriumsulfat getrocknet. Nach dem Abdampfen des Methylenchlorids erhält man das obige Produkt als gummiartiges Oel, das durch präparative Flüssigchromatographie (0 - 10% Ethylacetat in Methylenchlorid) gereinigt wird. Man erhält 57,2 g (76%) eines schwachgelben Feststoffs.

NMR ($CDCl_3$): 1.54 (s) 9H (t-bu) 2.09 (s) 3H (OAc); 3.35, 3.56 (d von d, J = 18Hz) 2H ($CH_2S$); 4.08 (s) 3H ($OCH_3$); 4.86 5.04 (d von d, J = 14Hz) 2H ($CH_2O$); 5.04 (d, J = 6Hz) 1H (C6); 5.93 (d von d, J = 6Hz J = 10Hz), 1H ($C_7$); 6.71 (s) 1H (Thiazol); 6.91 (d, J = 10Hz) 1H (NH); 7.08 (s) 1H (NH); 7.30 (s) 15H ($CPh_3$)

## Beispiel 3

Herstellung von [6R-[6 alpha, 7 beta(Z)]]-3-(Jodmethyl)-7-[[(methoxyimino)[2-(tritylamino)-4-thiazolyl]-acetyl]amino]-8-oxo-5-thia-1-azabicyclo[4.2.0]oct-2-en-2-carbonsäure-1,1-dimethylethylester

Eine Lösung von 23,8 g (0,0316 mol) [6R-[6 alpha, 7 beta(Z)]]-3-(Acetoxymethyl)-7-[[(methoxyimino)[2-(tritylamino)-4-thiazolyl]-acetyl]amino]-8-oxo-5-thia-1-azabicyclo[4.2.0]-oct-2-en-2-carbonsäure-1,1-dimethylethylester (aus Beispiel 2) in 250 ml wasserfreiem Methylenchlorid (destilliert aus Phosphorpentoxid) wird bei Zimmertemperatur unter Rühren mit 3 nacheinander folgenden Portionen Jodtrimethylsilan versetzt (2,0 ml/0,014 mol, 2,0 ml/0.014 mol und 1,5 ml/0,0105 mol in 10-minütigen Zeitabständen). Das Gemisch wird anschliessend 2 Stunden bei Zimmertemperatur gerührt und mit weiteren 0,5 ml (0,0035 mol) Jodtrimethylsilan versetzt. Nach weiterem 30-minütigem Rühren wird das Lösungsmittel bei 0°C abgedampft, wobei ein Gummi anfällt, das in 250 ml auf 0°C gekühltes Ethylacetat gelöst wird. Die Ethylacetatlösung wird auf 0°C gekühlt und nacheinander mit 3 x 125 ml kalter 10%iger wässriger Natriumthiosulfatlösung, 125 ml gesättigter wässriger Natriumbicarbonatlösung und 2 x 125 ml gestättigter wässriger Natriumchloridlösung versetzt und anschliessend mit wasserfreiem Natriumsulfat getrocknet. Das Lösungsmittel wird bei 0°C abgedampft. Man erhält ein Gummi, das durch präparative Flüssigchromatographie (Ethylacetat/n-Hexan/Methylenchlorid 1:4:6) gereinigt wird. Man erhält 15,4 g (59%) Produkt als - schwachgelben Feststoff. 5,0 g nicht reagiertes Ausgangsmaterial wird ebenfalls isoliert; demgemäss ist die Ausbeute basierend auf umgesetztes Ausgangsmaterial 75%.

NMR ($CDCl_3$): 1.56 (s) 9H (t-bu); 3.51, 3.76 (d von d, J=20Hz) 2H ($CH_2S$); 4.11 (s) 3H ($OCH_3$); 4.30, 4.44 (d von d, J=10Hz) 2H ($CH_2I$); 5.03 (d, J=6Hz) 1H (C6); 5.89 (d von d, J=6Hz, J=10Hz) 1H (C7); 6.72 (m) 2H (Thiazol, NH), 7.02 (s) 1H (NH); 7.30 (s) 15H ($CPh_3$). IR (KBr): 3385, 3285, 1787, 1717, 1681, 1524, 701.

## Beispiel 4

Herstellung von [6R-[6 alpha, 7 beta(Z)]]-3-[[(3,4-bis(Acetyloxy)benzoyl)oxy]methyl]-8-oxo-7-[[[2-[-(triphenylmethyl)amino]-4-thiazolyl](methoxyimino)acetyl]amino]-5-thia-1-azabicyclo[4.2.0]oct-2-en-2-carbonsäure-1,1-dimethylethylester

Eine Lösung von 6,98 g (0,085 mol) von [6R-[6α7β(Z)][[3-(Jodmethyl)-7-[[[(methoxyimino)-2-[-(triphenylmethyl)amino]-4-thiazolyl]acetyl]amino]-8-oxo-5-thia-1-azabicyclo[4.2.0]oct-2-en-2-carbonsäure-1,1-dimethylethylester (aus Beispiel 3) in 100 ml trockenem Dimethylformamid wird unter Rühren mit einer Lösung von 2,29 g (0,088 mol) Natrium(3,4-diacetoxy)benzoat in 100 ml trockenem Dimethylformamid (eine Stunde vorgetrocknet über 4Å Molekularsieb) in einer Argonatmosphäre bei Zimmertemperatur tropfenweise versetzt. Das Reaktionsgemisch wird 3 Stunden gerührt und das Lösungsmittel unter vermindertem Druck entfernt. Der ölige Rückstand wird in 100 ml Methylenchlorid/Ethylacetat (9:1) gelöst und durch eine kurze Kieselgelkolonne chromatographiert. Die das Produkt enthaltenden Fraktionen werden vereinigt; man erhält 7,25 g Rohprodukt. Weitere Reinigung unter Verwendung von präparativchromatographischer Technik (n-Hexan/Ethylacetat 5:4) liefert 3,9 g (42%) eines cremefarbenen Feststoffs.

NMR ($CDCl_3$): 1.44 (s) 9H (t-bu); 2.27 (s) 3H (OAc); 2.29 (s) 3H (OAc) 3.63 3.73 (d von d, J=10Hz)2H ($CH_2S$); 3.81 (s) 3H ($OCH_3$); 4.91, 5.19 (d von d, J=16Hz) 2H ($CH_2O$); 5.13 (d, J=6Hz) 1H (C6); 5.99, 6.01 (d von d, J=6Hz, J=6Hz) 1H (C7); 6.68 (s) 1H (Thiazol) 7.2-7.4 (m) 15H ($CPh_3$); 7.44 (d, J=9Hz) 1H (Ar); 7.84 (s) 1H (Ar); 7.91 (d, J=9Hz) 1H (Ar); 8.86 (s) 1H (NH); 9.59 (d, J=10Hz) 1H (NH). IR (KBr): 3300, 1778, 1772, 1682. UV (EtOH): λmax 234 (ε=36,500); M.S.: m/z 932 (M + H).

## Beispiel 5

Herstellung von [6R-[6 alpha, 7 beta(Z)]]-3-[[(3,4-Dihydroxybenzoyl)oxy]methyl]-8-oxo-7-[[[2-[-(triphenylmethyl)amino]-4-thiazolyl](methoxyimino)acetyl]amino]-5-thia-1-azabicyclo[4.2.0]oct-2-en-2-carbonsäure-1,1-dimethylethylester

In der in Beispiel 4 angegebenen Weise werden 1,65 g (2,0 mmol) [6R-[6α, 7β(Z)]]-3-(Jodmethyl)-7-[[[-(Methoxyimino) -2-[(Triphenylmethyl)amino]-4-thiazolyl]acetyl]amino]-8-oxo-5-thia-1-azabicyclo[4.2.0]oct-2-en-2-carbonsäure-1,1-dimethylethylester in 50 ml Dimethylformamid mit 374 mg (2.12 mmol) Natrium-3,4-dihydroxybenzoat in 80 ml Dimethylformamid versetzt, wonach 1,5 Stunden gerührt wird. Nach dem Chromatographieren an Kieselgel (99:1 Methylenchlorid/Methanol) erhält man 1,71 g (50%) eines cremefarbenen Feststoffs.

NMR ($CDCl_3$) 1.22 (s) 9H (t-bu); 3.30, 3.54 (d von d, J=16Hz) 2H ($CH_2S$); 3.82 (s) 3H ($OCH_3$); 4.04 (br s) 2H (OH); 5.07 (d, J=6Hz) 1H (C6); 5.24, 5.32 (d von d, J=12Hz) 2H ($CH_2O$); 5.95 ( d von d, J=6Hz, J=10Hz) 1H (C7); 6.77 (s) 1H (Thiazol); 6.87 (d, J-6Hz) 1H (Ar); 7.30 (s) 15H ($CPh_3$); 7.49 (m) 3H (Ar). IR (KBr): 3290, 1791, 1690, 1522, 702. UV (EtOH): λ max 260 nm (ε=23,200); M.S.: m/z 848 (M + H).

## Beispiel 6

Herstellung von [6R-[6 alpha, 7 beta(Z)]]-3-[[(2,3-Dihydroxybenzoyl)oxy]-methyl]-8-oxo-7-[[[2-[-(triphenylmethyl)amino]-4-thiazolyl](methoxyimino)acetyl]amino]-5-thia-1-azabicyclo[4.2.0]oct-2-en-2-carbonsäure-1,1-dimethylethylester

In der in Beispiel 4 angegebenen Weise werden 157 mg (0,19 mmol) [6R-[6α, 7β(Z)]]-3-(Jodmethyl)-7-[-[[methoxyimino)-2-[-(triphenylmethyl)amino]-4-thiazolyl]acetyl]amino]-8-oxo-5-thia-1-azabicyclo[4.2.0]oct-2-en-2-carbonsäure-1,1-dimethylethylester in 5 ml Dimethylformamid mit 35,3 mg (0.20 mmol) Natrium-2,3-dihydroxybenzoat in 5 ml Dimethylformamid versetzt. Nach dem Rühren während 2 Stunden 15 Minuten erhält man, nach dem Chromatographieren an Kieselgel (0 - 10% Ethylacetat in Methylenchlorid), 111 mg (69%) eines gräulich-weissen Feststoffs.

NMR ($CDCl_3$): 1.59 (s) 3H (t-bu); 3.48, 3.68 (d von d, J=20Hz) 2H ($CH_2S$); 4.07 (s) 3H ($OCH_3$); 5.10 (m) 2H (C6, 1/2$CH_2O$); 5.48 (d, J=14Hz) 1H (1/2$CH_2O$); 5.99 (m) 1H (C7); 6.67 (br s) 1H (OH) 6.72-6.88 (m) 3H (Thiazol, 2 Ar) 7.02 (br s) 1H; 7.16 (d von d, J=10Hz) 1H (NH) 7.28 (s) 15H ($CPh_3$); 10.71 (s) 1H (OH). IR (KBr): 3400, 1789, 1722, 1688, 700. UV (EtOH): λ max 240 nm (ε=33,900); MS: m/z 848 (M + H).

## Beispiel 7

Herstellung von [6 alpha, 7 beta(Z)]]-3-[[(3,4,5-tris(Acetyloxy)benzoyl)oxy]-methyl]-7-[[[(methoxyimino)[2-(triphenylmethyl)amino]-4-thiazolyl]acetyl]amino]-8-oxo-5-thia-1-azabicyclo[4.2.0]oct-2-en-2-carbonsäure-1,1-dimethylethylester

In der in Beispiel 4 angegebenen Weise werden 160,8 mg [5R-[5α, 7β(Z)]]-3-(Jodmethyl)-7-[[[-(methoxyimino)-2-[(triphenylmethyl)amino]-4-thiazolyl]acetyl]amino]-8-oxo-5-thia-1-azabicyclo[4.2.0]oct-2-en-2-carbonsäure-1,1-dimethylethylester mit 59 mg (0,195 mmol) Natrium-3,4,5-Triacetoxybenzoat in 2 ml Dimethylformamid versetzt. Nach 2-stündigem Rühren erhält man nach dem Chromatographieren an Kieselgel (0 - 15% Ethylacetat in Methylenchlorid) 64,1 mg (33%) eines cremefarbenen Feststoffs.

NMR ($CDCl_3$): 1.52 (s) 9H (t-bu); 3.37, 3.59 (d von d, J=18Hz) 2H ($CH_2S$); 4.06 (s) 3H ($OCH_3$); 5.02, 5.38 (d von d, J=7Hz) 2H ($CH_2O$); 5.05 (d, J=6Hz) 1H (C6); 5.95 (d von d, J=6Hz, J=10Hz) 1H (C7); 6.70 (d, J=10Hz) 1H (NH); 6.72 (s) 1H (Thiazol); 6.98 (s) 1H (NH); 7.29 (s) 15H ($CPh_3$); 7.77 (s) 2H (Ar). IR (KBr): 3340, 1788, 1722, 1690, 701. UV (EtOH): λ max 235 nm (ε=32,750); M.S.: m/z 990 (M + H).

## Besipiel 8

Herstellung von [6R-[6 alpha, 7 beta(Z)]]-3-[[2-(3,4-Dihydroxyphenyl)acetoxy]methyl]-7-[[(methoxyimino)[2-[-(triphenylmethyl)amino]-4-thiazolyl]acetyl]amino]-8-oxo-5-thia-1-azabicyclo[4.2.0]oct-2-en-2-carbonsäure-1,1-dimethylethylester

In der in Beispiel 4 angegebenen Weise werden 328,7 mg (0,400 mmol) [6R-[6α, 7β(Z)]]-3-(Jodmethyl)-7-[[[ (methoxyimino-2-[(triphenylmethyl)amino]-4-thiazolyl] acetyl]amino]-8-oxo-5-thia-1-azabicyclo[4.2.0]oct-2-en-2-carbonsäure-1,1-dimethylethylester in 4 ml Dimethylformamid mit 76 mg (0,400 mmol) 2-(3,4-Dihydroxyphenyl)Essigsäure-Natriumsalz in 4,0 ml Dimethylformamid versetzt. Nach 2,5-stündigem Rühren erhält man, nach dem Chromatographieren an Kieselgel (0 - 20% Ethylacetat in Methylenchlorid), 215 mg (62%) eines cremefarbenen Feststoffs.

NMR (DMSO-$d_6$): 1.44 (s) 9H (t-bu); 3.23-3.45 (m) 4H ($CH_2S$, $CH_2CO$); 3.80 (s) 3H ($OCH_3$); 4.65, 4.95 (d von d, J=14Hz) 2H ($CH_2O$); 5.11 (d, J=4Hz) 1H (C6); 5.69 (d von d, J=4Hz, J=10Hz) 1H (C7); 6.46-6.65 (m) 3H (Ar); 6.69 (s) 1H (Thiazol); 7.19-7.37 (m) 15H ($CPh_3$); 8.80 (s) 1H (OH); 8.86 (s) 1H (NH); 6.88 (s) 1H (OH); 9.57 (d, J=10Hz) 1H (NH). IR (KBr): 3390, 1788, 1723, 1662, 702. UV (EtOH): λmax 225 nm (ε=19,900); M.S.: m/z 861 (M + H).

Beispiel 9

Herstellung von (6R-trans)-3-[[[(3,4-bis(Acetyloxy)benzoyl]oxy]methyl]-8-oxo-7-[(phenoxyacetyl)amino]-5-thia-1-azabicyclo[4.2.0]oct-2-en-2-carbonsäure-1.1-dimethylethylester

In der in Beispiel 4 angegebenen Weise werden 4,75 g (8,95 mmol) (6R-trans)-3-(Jodmethyl)-8-oxo-7-[-(phenoxyacetyl)amino]-5-thia-1-azabicyclo[4.2.0]oct-2-en-2-carbonsäure-1,1-dimethylethylester in 50 ml Dimethylformamid mit 2,33 g (8,95 mmol) Natrium-3,4-diacetoxybenzoat in 100 ml Dimethylformamid versetzt. Nach 2-stündigem Rühren erhält man, nach dem Chromatographieren an Kieselgel (6:4 n-Hexan/Ethylacetat), 3,36 g (58,5%) eines cremefarbenen Feststoffs.

NMR ($CDCl_3$): 1.56 (s) 9H (t-bu); 2.33 (s) (6H)(2 OAc); 3.41, 3.63 (d von d, J-18Hz) 2H ($CH_2S$); 4.60 (s) 3H ($OCH_3$); 5.06, 5.40 (d von d, J=14Hz) 2H ($CH_2O$); 5.07 (d, J=6Hz) 1H (C6); 5.97 (d von d, J=6Hz, J=10Hz) 1H (C7); 6.98 (d, J=8Hz) 1H (Ar); 7.09 (d, J=10Hz) 1H (NH); 7.12-7.40 (m) 5H (Ar); 7.90 (s) 1H (Ar); 7.98 (d, J=8Hz) 1H (Ar).

Beispiel 10

Herstellung von [6R-[6 alpha, 7 beta(Z)]]-7-[[(2-Amino-4-thiazolyl)(methoxyimino)acetyl]amino]-3-[[[(3,4-bis-(acetyloxy)benzoyl)]oxy]methyl]-8-oxo-5-thia-1-azabicyclo[4.2.0]oct-2-en-2-carbonsäure-mononatriumsalz

Eine Lösung von 1,90 g (2,04 mmol) [6R-[6 alpha, 7 beta(Z)]]-3-[[[3,4-bis(Acetyloxy)benzoyl]oxy]-methyl]-8-oxo-7-[[[2-[(triphenylmethyl)amino]-4-thiazolyl](methoxyimino)acetyl]amino]-5-thia-1-azabicyclo-[4.2.0]oct-2-en-2-carbonsäure-1,1-dimethylethylester (aus Beispiel 4) in 38 ml trockenem Methylenchlorid wird auf 0°C gekühlt und mit 3,8 ml Anisol und anschliessend mit 37,5 ml Trifluoressigsäure behandelt. Das Reaktionsgemisch wird 6 Stunden bei 0°C gerührt und das Lösungsmittel unter vermindertem Druck abgedampft. Das zurückbleibende Oel wird in 40 ml Ethylacetat aufgenommen und 9 mal mit je 40 ml 1%-iger wässriger Natriumlicarbonatlösung gewaschen. Die Fraktionen 4 - 7 enthalten das Produkt und werden zur Reinigung durch eine kurze $C_{18}$ Reverse Phase Kieselgel-Kolonne gegeben (mit Wasser zur Entfernung von überflüssigem Natriumbicarbonat gefolgt durch 25% Acetonitril in Wasser zur Entfernung des organischen Materials). Die das Produkt enthaltenden Fraktionen werden vereinigt und lyophilisiert; (760 mg Rohstoff). Das lyophilisierte Pulver wird weiter gereinigt unter Verwendung von präperativer Reverse Phase Chromatographie (0 - 30% Acetonitril); man erhält 580 mg (43%) eines weissen Pulvers.

NMR (DMSO-$d_6$): 2.29 (s) 3H (OAc); 2.30 (s) 3H (OAc); 3.26, 3.60 (d von d, J=16Hz) 2H ($CH_2S$) 3.84 (s) 3H ($OCH_3$); 4.95, 5.21 (d von d, J=10Hz) 2H ($CH_2O$); 4.97 (d, J=6Hz) 1H (C6); 5.53 (d von d, J=6Hz, J=10Hz) 1H (C7); 6.69 (s) 1H (Thiazol); 7.19 (s) 1H (NH), 7.39 (d, J=8Hz) 1H (Ar); 7.80 (s) 1H (Ar) 7.87 (d, J(8Hz) 1H (Ar); 9.51 (d, J=8Hz) 1H (NH). IR (KBr): 3350, 1769, 1715, 1685, 1610; M.S.: m/z 656 (M + H). UV ($H_2O$): $\lambda$ max = 235 nm ($\epsilon$=26,500). HRMS berechnet (M + H) für $C_{25}H_{23}NaN_5O_{11}S_2$: 656.0733. Gefunden: 656.0732.

Beispiel 11

Herstellung von [6R-[6 alpha, 7 beta(Z)]]-7-[[(2-Amino-4-thiazolyl)(methoxyimino)acetyl]amino]-3-[[(3,4-dihydroxybenzoyl)oxy]methyl]-8-oxo-5-thia-1-azabicyclo[4.2.0]-oct-2-en-2-carbonsäure-mononatriumsalz.

In der in Beispiel 10 angegebenen Weise werden 52,3 mg (0,092 mmol) [6R-[6 alpha, 7 beta(Z)]]-3-[[-(3,4-Dihydroxybenzoyl)oxy]methyl]-8-oxo-7-[[[2-[(triphenylmethyl)amino]-4-thiazolyl](methoxyimino)acetyl]-amino]-5-thia-1-azabicyclo[4.2.0]oct-2-en-2-carbonsäure-1,1-dimethylethylester (Beispiel 5) in 1 ml Methylenchlorid und 100µl Anisol mit 1 ml Trifluoressigsäure behandelt. Nach Rühren während 3 Stunden und 40 Minuten erhält man,nach Reverse Phase Chromatographie (0 - 30% Acetonitril in Wasser), 11,3 mg (32%) eines weissen Pulvers.

NMR ($D_2O$): 3.47, 3.73 (d, J=18Hz) 2H ($CH_2S$); 3.98 (s) 3H ($OCH_3$); 4.89, 5.11 (d von d, J=10Hz) 2H ($CH_2O$); 5.21 (d, J=6Hz) 1H (C6); 5.80 (d, J=6Hz) 1H (C7); 6.91 (d, J=8Hz) 1H (Ar); 7.00 (s) 1H (Thiazol); 7.48 (s) 1H (Ar); 7.52 (d, J=8Hz) 1H (Ar). IR (KBr): 3320, 1762, 1680, 1610, 1530. MS: m/z 572 (M + H). UV ($H_2O$): λ max = 260 nm (ε=21,800). HRMS berechnet (M + H) für $C_{21}H_{19}NaN_5O_9S_2$: 572.0522. Gefunden: 572.0533.

## Beispiel 12

Herstellung von [6R-trans)-3-[[[3,4-bis(Acetyloxy)benzoyl]oxy]methyl]-8-oxo-7-[(phenoxyacetyl)amino]-5-thia-1-azabicyclo[4.2.0] oct-2-en-2-carbonsäure-mononatriumsalz

In der in Beispiel 10 angegebenen Weise werden 129 mg (0,201 mmol) (6R-trans)-3-[[[3,4-bis-(Acetyloxy)benzoyl]oxy]methyl]-8-oxo-7-[(phenoxyacetyl)amino]-5-thia-1-azabicyclo[4.2.0]oct-2-en-2-carbonsäure-1,1-dimethylethylester (Beispiel 9) in 2,5 ml Methylenchlorid und 220 µl Anisol mit 2,2 ml Trifluoressigsäure behandelt. Das Ganze wird 6,5 Stunden gerührt; man erhält, nach Reverse Phase Chromatographie (0 - 30% Acetonitril in Wasser), 42 mg (34%) eines weissen Pulvers.

NMR ($D_2O$): 2.37 (s) 6H (2 OAc); 3.42, 3.70 (d von d, J=18Hz) 2H ($CH_2S$); 4.96, 5.16 (d von d, J=12Hz) 2H ($CH_2OCO$); 5.11 (d, J=6Hz) 1H (C6); 6.70 (d, J=6Hz) 1H (C7); 7.01 (d, J=8Hz) 1H (Ar); 7.05 (M) 1H (Ar) 7.32-7.43 (m) 4H (Ar) 7.92 (s) 1H (Ar) 8.00 (d, J=8Hz) 1H (Ar) ($CH_2OAr$ durch HOD verdunkelt). IR (KBr): 3450, 1782, 1718, 1692, 1608. MS: m/z 607 (M + H). UV ($H_2O$): λ max = 236 nm (ε=13,400). HRMS berechnet (M + H for $C_{27}H_{24}NaN_2O_{11}S_2$: 607.0999. Gefunden: 607.1007.

## Beispiel 13

Herstellung von [6R-[6 alpha, 7 beta(Z)]]-7-[[(2-Amino-4-thiazolyl)(methoxyimino)acetyl]amino]-3-[[[(2,3-dihydroxyphenyl)carbonyl]oxy]methyl]-8-oxo-5-thia-1-azabicyclo[4.2.0]-oct-2-en-2-carbonsäuremononatriumsalz

In der in Beispiel 10 angegebenen Weise werden 100 mg (0,118 mmol) [6R-[6 alpha, 7 beta(Z)]]-3-[[(2,3-Dihydroxybenzoyl)oxy]methyl]-8-oxo-7[[[2-[(triphenylmethyl)amino]-4-thiazolyl](methoxyimino)acetyl]-amino]-5-thia-1-azabicyclo[4.2.0]oct-2-en-2-carbonsäure-1,1-dimethylethylester (Beispiel 6) in 3,0 ml Methylenchlorid und 300 µl Anisol mit 3,0 ml Trifluoressigsäure behandelt. Nach 6-stündigem Rühren erhält man, nach Reverse Phase Chromatographie (0 - 30% Acetonitril in Wasser), 28,2 mg (42%) eines weissen Pulvers.

NMR ($D_2O$): 3.30, 3.57 (d von d, J=16Hz) 2H ($CH_2S$); 3.78 (s) 3H ($OCH_3$); 4.78, 5.02 (d von d, J=12Hz) 2H ($CH_2OCO$); 5.04 (d, J=6Hz) 1H (C6); 5.66 (d, J=6Hz) 1H (C7); 6.64 (m) 1H (Ar); 6.81 (s) 1H (Thiazol); 6.91 (d, J=8Hz) 1H (Ar); 7.24 (d, J=6Hz) 1H (Ar). IR (KBr): 3350, 1765, 1672, 1612. MS: m/z 572 (M + H). UV ($H_2O$) λ max 245 nm (ε=21,400), λ max 298 nm (ε=8600). HRMS berechnet (M + H) für $C_{21}H_{19}NaN_5O_9S_2$: 572.0522. Gefunden 572.0505.

## Beispiel 14

Herstellung von [6R-[6 alpha, 7 beta(Z)]]-3-[[3,4,5-tris(Acetyloxy)benzoyl]oxy]-methyl]-7-[[(2-amino-4-thiazolyl)(methoxyimino)acetyl]amino]-8-oxo-5-thia-1-azabicyclo[4.2.0]oct-2-en-2-carbonsäuremononatriumsalz

In der in Beispiel 10 angegebenen Weise werden 160 mg (0,161 mmol) [6R-[6 alpha, 7 beta(Z)]]-3-[-[3,4,5-tris(Acetyloxy)benzoyl]oxy]methyl-7-[[(methoxyimino)[(triphenylmethyl)amino]-4-thiazolyl]acetyl]-amino]-8-oxo-5-thia-1-azabicyclo[4.2.0]oct-2-en-2-carbonsäure-1,1-dimethylethylester (Beispiel 7) in 3,2 ml Methylenchlorid und 300 µl Anisol mit 3,0 ml Trifluoressigsäure behandelt. Das Ganze wird 6,5 Stunden gerührt; man erhält, nach Reverse Phase Chromatographie (0 - 30% Acetonitril in Wasser), 69 mg (60%) eines weissen Feststoffs.

NMR ($D_2O$): 2.38 (S) 3H (OAc); 2.39 (s) 3H (OAc); 3.51, 3.78 (d von d, J=16Hz) 2H ($CH_2S$); 5.00, 5.21 (d von d, J=12Hz) 2H ($CH_2O$); 5.24 (d, J=6Hz) 1H (C6); 5.82 (d, J=6Hz) 1H (C7); 7.03 (s) 1H (Thiazolyl); 7.79 (s) 2H (Ar). IR (KBr): 3360, 1772, 1718, 1675, 1610. MS: m/z 714 (M + H). UV ($H_2O$): $\lambda$ max = 234 nm ($\epsilon$=18,100). HRMS berechnet (M + H) für $C_{27}H_{25}NaN_5O_{13}S_2$: 714.0788. Gefunden: 714.0745

## Beispiel 15

Herstellung von [6R-[6 alpha, 7 beta(Z)]]-7-[[(2-Amino-4-thiazolyl)(methoxoyimino)acetyl]amino]-3-[[2-(3,4-dihydroxyphenyl)acetoxy]methyl]-8-oxo-5-thia-1-azabicyclo[4.2.0]oct-2-en-2-carbonsäure-mononatriumsalz

In der in Beispiel 10 angegebenen Weise werden 175 mg (0,203 mmol) [6R-[6 alpha, 7 beta(Z)]]-3-[[2-(3,4-Dihydroxyphenyl)-acetoxy]methyl]-7[[(methoxyimino)[2-[(triphenylmethyl)amino]-4-thiazolyl]acetyl]-amino]-8-oxo-5-thia-1-azabicyclo[4.2.0]oct-2-en-2-carbonsäure-1,1-dimethylethylester (Beispiel 8) in 3,5 ml Methylenchlorid und 350 µl Anisol mit 3,5 ml Trifluoressigsäure behandelt. Nach 6-stündigem Rühren erhält man, nach Reverse Phase Chromatographie (0 - 30% Acetonitril in Wasser), 22 mg (19%) eines weissen Feststoffs.

NMR ($D_2O$): 3.27, 3.49 (d von d, J=16Hz) 2H ($CH_2S$); 3.62 (s) 2H ($CH_2O$); 4.00 (s) 3H ($OCH_3$); 5.00 (d, J=12Hz) 1H (1.2 $CH_2O$) (das andere Doublet von HOD verdunkelt); 5.17 (d, J=6Hz) 1H (C6); 5.82 (d, J=6Hz) 1H (C7); 6.75 (d, J=8Hz) 1H (Ar); 6.86 (s) 1H (Ar); 6.90 (d, J=8Hz) 1H (Ar); 7.04 (s) 1H (Thiazol). IR (KBr): 3340, 1763, 1670, 1610. MS: m/z 586 (M + H), 564 (M + H freie Säure). UV ($H_2O$) sh 230 nm ($\epsilon$=16,900), sh 260 nm ($\epsilon$13,300). HRMS berechnet (M + H) für $C_{22}H_{21}NaN_5O_9S_2$: 586.0678. Gefunden: 586.0641.

## Beispiel 16

Herstellung von (6R-trans)-7-Amino-3-[[[3,4-bis(acetyloxy)-benzoyl]oxy]methyl]-8-oxo-5-thia-1-azabicyclo-[4.2.0]oct-2-en-2-carbonsäure-1,1-dimethylethylester-monohydrochlorid

Eine Lösung von 1,66 ml (20,0 mmol) Pyridin in 60 ml trockenem Methylenchlorid wird in einer Argonatmosphäre auf 0 °C gekühlt und mit 4,16 g (20,0 mmol) Phosphorpentachlorid behandelt. Das Gemisch wird 45 Minuten bei 0 °C gerührt. Dieses Gemisch wird tropfenweise mit einer Lösung von 8,80 g (6R-trans)-3-[[[3,4-bis(Acetyloxy)benzoyl]oxy]methyl]-8-oxo-7-[(phenoxyacetyl)amino]-5-thia-1-azabicyc lo[4.2.0]-oct-2-en-2-carbonsäure-1,1-dimethylethylester (Beispiel 9) in 40 ml trockenem Methylenchlorid innerhalb Minuten versetzt. Das Reaktionsgemisch wird 1,5 Stunden bei 0 °C gerührt und anschliessend tropfenweise mit 24 ml 1-Propanol versetzt. Nach 1-stündigem Rühren wird das Ganze mit 60 ml Wasser versetzt und während 40 Minuten gerührt. Das Lösungsmittel wird unter vermindertem Druck abgedampft. Die zurückbleibende wässrige Lösung wird mit 300 ml Diethylether unter heftigem Rühren behandelt; das Produkt kristallisiert dabei als weisser kristalliner Feststoff. Ausbeute 5,28 g (71%).

NMR (DMSO-$d_6$): 1.49 (s) 9H (t-bu); 2.32 (s) 3H (OAc); 2.33 (s) 3H (OAc); 3.78 3.87 (d von d, J=18Hz) 2H ($CH_2S$); 4.97 (m) 1H (C6); 5.24 (m) 3H (C7, $CH_2O$); 7.47 (d, J=8Hz) 1H (Ar); 7.85 (s) 1H (Ar); 7.92 (d, J=8Hz) 1H (Ar); 9.05 (br) 3H ($NH_3$+). IR (KBr): 3400, 1778, 1772. UV (EtOH): $\lambda$ max 238 nm ($\epsilon$=15,580).

## Beispiel 17

Herstellung von [6R-[6 alpha, 7 beta(Z)]]-3-[[[3,4-bis (Acetyloxy)benzoyl]oxy]-methyl]-7-[[[(2-amino-4-thiazolyl)[1,1-dimethyl-2-(1,1-dimethylethoxy)-2-oxoethoxy]imino]acetyl]amino]-8-oxo-5-thia-1-azabicyclo[4.2.0]oct-2-en-2-carbonsäure-1,1-dimethylethylester

Eine Aufschlämmung von 1,00 g (1,84 mmol) (6R-trans)-7-Amino-3-[[[3,4-bis(acetyloxy)benzoyl]oxy]-methyl]-8-oxo-5-thia-1-azabicyclo[4.2.0]oct-2-en-2-carbonsäure-1,1-dimethylethylester-monohydrochlorid (Beispiel 16) in 25 ml Methylenchlorid wird mit 25 ml einer wässrigen gesättigten Kochsalz/Natriumlicarbonatmischung (4:1) gründlich gewaschen. Die organische Lösung wird abgedampft und über Magnesiumsulfat getrocknet. Diese Lösung wird mit 1,10 g (2,30 mmol) S-2-Benzothiazolyl-2-amino-alpha-[(Z)-[1-(tertbutoxycarbonyl)-1-methylethoxy]imino]thio-4-thiazolacetat versetzt. Die erhaltene Lösung wird 16 Stunden gerührt und das Lösungsmittel unter vermindertem Druck abgedampft. Der Rückstand wird an Kieselgel chromatographiert (mit n-Hexan/Ethylacetat 1:1 und anschliessend mit Ethylacetat); man erhält 1,16 g (77%) eines cremefarbenen Feststoffs.

NMR ($CDCl_3$): 1.41 (s) 9H (t-bu); 1.52 (s) 9H (t-bu); 1.56 (s) 3H ($CH_3$); 1.58 (s) 3H ($CH_3$); 2.29 (s) 6H (2OAc); 3.38, 3.59 (d von d, J=18Hz) 2H ($CH_2S$); 5.04 (m) 2H (C6, 1/2 $CH_2O$); 5.38 (d, J=12Hz) 1H (1/2 $CH_2O$); 6.01 (d von d, J=6Hz, J=10Hz) 1H (C7); 6.10 (s) 2H ($NH_2$); 6.88 (s) 1H (Thiazol); 7,25 (d, J=12Hz) 1H (NH); 7.68 (d, J=8Hz) 1H (Ar), 7.82 (s) 1H (Ar); 7.91 (d, J=8Hz) 1H (Ar). IR (KBr): 3300, 1782, 1722, 1688, 1535. MS: m/z 818 (M + H). UV (EtOH) λ max 235 nm (ε=28,450).

## Beispiel 18

Herstellung von [6R-[6 alpha, 7 beta(Z)]]-3-[[[3,4-bis(Acetyloxy)benzoyl]oxy]-methyl]-7-[[[[(2-amino-2-oxoethoxy)imino]-4-thiazolyl]acetyl]amino]-8-oxo-5-thia-1-azabicyclo[4.2.0]oct-2-en-carbonsäure-1,1-dimethylethylester

Eine Aufschlämmung von 234 mg (0.430 mmol) (6R-trans)-7-Amino-3-[[[3.4-bis(acetyloxy)benzoyl]oxy]-methyl]-8-oxo-5-thia-1-azabicyclo[4.2.0]oct-2-en-2-carbonsäure-1,1-dimethylethylester-monohydrochlorid in 10 ml Methylenchlorid wird mit 10 ml einer gesättigten wässrigen Kochsalz/Natriumlicarbonatlösung (4:1) gewaschen. Die organische Lösung wird abgetrennt und über Magnesiumsulfat getrocknet. Diese Lösung wird tropfenweise zu einer Lösung von 157 mg (0.400 mmol) S-(2-Benzothiazolyl)-Z-aminothio-4-thiazolglyoxylat- 0-(carbamoylmethyl)oxim in 5 ml Methylenchlorid und 8 ml trockenem Dimethylformamid gegeben. Nach 3-stündigem Rühren wird das Lösungsmittel unter vermindertem Druck abgedampft. Der Rückstand wird chromatographiert (0 - 10% Methanol in Methylenchlorid); man erhält 220 mg (70%) eines cremefarbenen Produkts.

NMR ($CDCl_3$): 1.51 (s) 9H (t-bu); 2.28 (s) 3H (OAc); 2.29 (s) 3H (OAc); 3.48, 3.51 (d von d, J=18Hz) 2H ($CH_2S$); 4.60, 4.81 (d von d, J=18Hz) 2H ($CH_2CON$); 4.96, 5.37 (d von d, J=12Hz) 2H ($CH_2O$); 5.10 (d, J=6Hz) 1H (C6); 5.83 (br) 2H ($NH_2$) 5.83 (br) 2H ($NH_2$) 6.01 (d von d, J=6Hz, J=10Hz) 1H (C7); 6.76 (s) 1H (Thiazolyl); 7.27 (d, J=8Hz) 1H (Ar); 7.85 (s) 1H (Ar); 7.93 (d, J=8Hz) 1H (Ar). IR (KBr): 3450, 3350, 1778, 1720, 1680, 1532. MS: m/z 733 (M + H). UV (EtOH) λ max 236 nm (ε=29,700).

## Beispiel 19

Herstellung von [6R-[6 alpha, 7 beta(Z)]]-3-[[[3,4-bis (Acetyloxy)benzoyl]oxy]methyl]-7-[[(2-amino-4-thiazolyl)[(1-carboxy-1-methylethoxy)imino]acetyl]amino]-8-oxo-5-thia-1-azabicyclo[4.2.0]oct-2-en-2-carbonsäure-1,1-dimethylethylester-natriumsalz

In der in Beispiel 10 angegebenen Weise werden 247 mg (0,301 mmol) [6R-[6 alpha, 7 beta(Z)]]-3-[[[3,4-bis(Acetyloxy)benzoyl]oxy]methyl]-7-[[[(2-amino-4-thiazolyl)[1,1-dimethyl-2-(1,1-dimethylethoxy)-2-oxoethoxy]imino]acetyl]amino]-8-oxo-5-thia-1-azabicyclo[4.2.0]-oct-2-en-2-carbonsäure-1,1-dimethylethylester (Beispiel 17) in 2,5 ml Methylenchlorid und 250 µl Anisol mit 2,5 ml Trifluoressigsäure behandelt. Nach 7-stündigem Rühren erhält man, nach Reverse Phase Chromatographie (0 - 30% Acetonitril in Wasser), 61,2 mg (27%) eines weissen Feststoffs.

NMR (DMSO-$d_6$): 1.30 (s) 3H ($CMe_2$); 1.37 (s) 3H ($CMe_2$); 2.20 (s) 3H (OAc); 2.21 (s) 3H (OAc); 3.49 (d, J=18Hz) 1H (1/2 $CH_2S$, andere Hälfte durch HOD verdunkelt); 4.87, 5.23 (d von d, J=12Hz) 2H ($CH_2O$); 4.96 (d, J=6Hz) 1H (C6); 5.60 (d von d, J=6Hz, J=10Hz) 1H (C7); 6.66 (s) 1H (Thiazol); 7.11 (br) 3H (NH, $NH_2$); 7.37 (d, J=8Hz) 1H (Ar); 7.78 (s) 1H (Ar); 7.85 (d, J=8Hz) 1H (Ar). IR (KBr): 3400, 1770, 1718, 1672, 1608. MS: m/z 650 (M + H). UV (EtOH) λ max 235 nm (ε=20,000). HRMS berechnet (M + H) für $C_{28}H_{25}NaN_5O_{13}S_2$: 772.0584. Gefunden: 772.0543.

## Beispiel 20

Herstellung von [6R-[6 alpha, 7 beta(Z)]]-7-[[[(2-Amino-4-thiazolyl)[1-carboxy-1-methylethoxy]imino]acetyl]-amino]-3-[[(3,4-dihydroxybenzoyl)oxy]methyl]-8-oxo-5-thia-1-azabicyclo[4.2.0]oct-2-en-2-carbonsäure-dinatriumsalz

Eine Lösung von 220 ml (0,227 mmol) [6R-[6 alpha, 7 beta(Z)]]-3-[[[3,4-bis(Acetyloxy)benzoyl]oxy]-methyl]-7-[[[(2-amino-4-thiazolyl)[1,1-dimethyl-2-(1,1-dimethylethoxy)-2-oxoethoxy]imino]acetyl]amino]-8-oxo-5-thia-1-azabicyclo[4.2.0]oct-2-en-2-carbonsäure-1,1-dimethylethylester (Beispiel 17) in 10 ml Methylenchlorid und 1 ml Anisol werden bei 0°C mit 10 ml Trifluoressigsäure behandelt. Nach 6-stündigem Rühren bei 0°C wird das Lösungsmittel unter vermindertem Druck abgedampft. Der ölige Rückstand wird mit 5 ml Ethylacetat, 5 ml gesättigter wässriger Natriumbicarbonatlösung und 5 ml Methanol versetzt. Das Zweiphasengemisch wird 30 Minuten gerührt. Die Lösung wird unter vermindertem Druck zu etwa 5 ml eingedampft und mit 5ml Ethylacetat und 5 ml gesättigter wässriger Natriumticarbonatlösung versetzt. Die Lösung wird 15 Minuten gerührt. Die wässrige Schicht wird abgetrennt woraus nach Reverse Phase Chromatographie (0 - 20% Acetonitril in Wasser) 60 mg (33%) eines weissen Feststoffs isoliert wird.

NMR (DMSO-$d_6$): 1.40 (s) 3H ($CMe_2$); 1.49 (s) 3H ($CMe_2$); 3.31, 3.49 (d von d, J=16Hz) 2H ($CH_2S$); 4.96, 5.04 (d von d, J=12Hz) 2H ($CH_2O$); 5.05 (d, J=6Hz) 1H (C6); 5.68 (m) 1H (C7); 6.73 (m) 2H (Ar), Thiazolyl); 7.18 (br) 2H ($NH_2$); 7.27 (d, J=8Hz) 1H (Ar); 7.34 (s) 1H (Ar); 11.70 (d, J=10Hz) 1H (NH). IR (KBr): 3350, 1762, 1670, 1598. MS: m/z 666 (M + H). UV (EtOH) λ max 218 nm (ε=25.050), λ max 262 nm (ε=17,600). HRMS berechnet (M + H) für $C_{24}H_{21}N_5O_{11}S_2Na$: 666.0553. Gefunden: 666.0619.

## Beispiel 21

Herstellung von [6R-[6 alpha, 7 beta(S*)]]-3-[[[3,4-bis(Acetyloxy)benzoyl]oxy]methyl]-7-[[α[[(4-ethyl-2,3-dioxo-1-piperazinyl)carbonyl]amino]phenylacetyl]amino]-8-oxo-5-thia-1-azabicyclo[4.2.0]oct-2-en-2-carbonsäure-1,1-dimethylethylester

Eine Suspension von 275 mg (0,506 mmol) (6R-trans-7-Amino-3-[[[3,4-bis(acetyloxy)benzoyl]oxy]-methyl]-8-oxo-5-thia-1-azabicyclo[4.2.0]oct-2-en-2-carbonsäure-1,1-dimethylethylester-monohydrochlorid in 10 ml Methylenchlorid wird mit 10 ml einer gesättigten wässrigen Natriumchlorid/Natriumbicarbonatlösung gewaschen. Die organische Lösung wird abgetrennt und über Magnesiumsulfat getrocknet. Diese Lösung wird zu einer Lösung von aktivem Ester gegeben (hergestellt durch Zugabe von 69 mg (0.506 mmol) N-Hydroxybenzotriazol gefolgt durch 104 mg (0,506 mmol) 1,3-Dicyclohexylcarbodiimid zu einer Lösung von 161 mg (0.506 mmol) α-[[(4-Ethyl-2,3-dioxo-1-piperazinyl)carbonyl]amino]phenylessigsäure im 10 ml trockenem Tetrahydrofuran und 45-minütiges Rühren). Die Lösung wird 16 Stunden gerührt, filtriert und das Lösungsmittel unter vermindertem Druck abgedampft. Der Rückstand wird chromatographiert (Ethylacetat), und man erhält 250 mg (61%) eines cremefarbenen Feststoffs.

NMR ($CDCl_3$): 1.20 (t, J=3Hz) 3H (Et) 1.52 (s) 9H (t-bu); 2.32 (s) 6H (20Ac) 3.2-3.7 (m) 6H ($CH_2S$, Et, 1/2 $NCH_2CH_2N$) 3.93 (m) 1H (1/4$NCH_2CH_2N$); 4.11 (m) 1H (1/4$NCH_2CH_2N$); 4.91 (d, J=6Hz) 1H (C6); 5.00, 5.30 (d von d, J=14Hz) 2H ($CH_2O$); 5.58 (d, J=8Hz) 1H (NCHCPh); 5.84 (d von d, J=6Hz, J=10Hz) 1H (C7); 6.83 (d, J=10Hz) 1H (NH); 7.20-7.35 (m) 6H (Ar); 7.80 (s) 1H (Ar); 7.89 (d, J=8Hz) 1H (Ar); 10.04 (d, J=8Hz) 1H (NH). IR (KBr): 3320, 1780, 1718, 1688. UV (EtOH): inf 225 nm (ε=23,300), inf 270 nm (ε=10,900).

## Beispiel 22

Herstellung von [6R-[6 alpha, 7 beta(S*)]]-3-[[[3,4-bis(Acetyloxy)benzoyl]oxy]methyl]-7-[[α[[(4-ethyl-2,3-dioxo-1-piperazinyl)carbonyl]amino]phenylacetyl]amino]-8-oxo-5-thia-1-azabicyclo[4.2.0]oct-2-en-2-carbonsäuremononatriumsalz

In der in Beispiel 10 angegebenen Weise werden 100 mg (0,124 mmol) [6R-[6 alpha, 7 beta(S*)]]-3-[[-[3,4-bis(Acetyloxy)benzoyl]oxy]methyl]-7-[[α[[(ethyl-2,3-dioxo-1-piperazinyl)carbonyl]amino]-phenylacetyl]-amino]-8-oxo-5-thia-1-azabicyclo[4.2.0]oct-2-en-2-carbonsäure-1,1-dimethyl ethylester (Beispiel 21) in 5 ml Methylenchlorid und 50 µl Anisol mit 5 ml Trifluoressigsäure behandelt. Nach 5-stündigem Rühren erhält man, nach Reverse Phase Chromatographie (0 - 30% Acetonitril in Wasser), 48 mg (50%) eines weissen Feststoffs.

NMR (DMSO-$d_6$): 1.08 (t, J=8Hz) 3H (NEt); 2.31 (s) 6H (2OAc); 3.21-3.60 (m) 6H ($CH_2S$, NEt, 1/2 $NCH_2CH_2N$) 3.91 (m) 2H (1/2 $NCH_2CH_2N$); 4.92 (d, J=6Hz) 1H (C6); 4.95, 5.25 (d, J=12Hz) 2H ($CH_2O$); 5.57 (m) 1H (C7); 5.75 (d, J=8Hz) 1H (NCHCPh); 7.26-7.48 (m) 6H (Ar); 7.82 (s) 1H (Ar); 7.89 (d, J=8Hz) 1H (Ar); 9.41 (d, J=8Hz) 1H (NH) 9.86 (d, J=10Hz) 1H (NH). IR (KBr): 3420, 3305, 1772, 1715, 1682, 1610. UV (EtOH): λ max 230 nm (ε=22,600). HRMS berechnet (M + H) für $C_{34}H_{33}NaN_5O_{13}S$: 774.1693. Gefunden: 774.1729.

## Beispiel 23

Herstellung von [6R-[6 alpha, 7 beta(S*)]]-7-[[α[[(4-Ethyl-2,3-dioxo-1-piperazinyl)-carbonyl]amino]-phenylacetyl]amino]-3-[(3,4-dihydroxy)benzoyl]oxy]methyl]-8-oxo-5-thia-1-azabicyclo[4.2.0]oct-2-en-2-carbonsäure-mononatriumsalz

In der in Beispiel 20 angegebenen Weise werden 150 mg (0,186 mmol) [6R-[6 alpha, 7 beta(S*)]]-3-[[-[3,4-bis(Acetyloxy)benzoyl]oxy]methyl]-7-[[α[[(4-ethyl-2,3-dioxo-1-piperazinyl]carbonyl]amino]phenylacetyl]-amino]-8-oxo-5-thia-1-azabicyclo[4.2.0]oct-2-en-2-carbonsäue-1,1-dimethylethylester (Beispiel 21) in 5 ml Methylenchlorid und 500 µl Anisol mit 5 ml Trifluoressigsäure behandelt. Das Gemisch wird mit einer Mischung aus Ethylacetat, gesättigter wässriger Natriumbicarbonatlösung und Methanol (1:1:1) gerührt; man erhält, nach Reverse Phase Chromatographie (0 - 20% Acetonitril in Wasser), 68 mg (53%) eines weissen Feststoffs.

NMR (DMSO-$d_6$): 1.01 (t, J=8Hz) 3H (NEt); 3.14, 3.39 (d von d, J=18Hz) 2H ($CH_2S$); 3.30-3.60 (m) 4H (NEt, 1/2$NCH_2CH_2N$); 3.85 (m) 2H (1/2 $NCH_2CH_2N$); 4.80, 5.08 (d von d, J=12Hz) 2H ($CH_2O$); 4.83 (d, J=6Hz) 1H (C6); 5.48 (m) 1H (C7); 5.61 (d, J=8Hz) 1H (NCHCPh); 6.69 (d, J=8Hz) 1H (Ar); 7.16-7.41 (m) 7H (Ar); 9.33 (br) 1H (NH); 9.78 (d, J=8Hz) 1H (NH). IR (KBr): 3400, 3300, 1762, 1712, 1680, 1603. MS: m/z 690 (M + H). UV (EtOH): λ max 263 nm (ε=17,900). HRMS berechnet (M + H) für $C_{30}H_{29}$ $N_5O_{11}$ SNa: 690.1482. Gefunden: 690.1517.

## Beispiel 24

Herstellung von [6R-[6 alpha, 7 beta(Z)]]-3-[[[3,4-bis(Acetyloxy)benzoyl]oxo]methyl]-7-[[[2-amino-4-thiazolyl]-[(2-amino-2-oxoethoxy)imino]acetyl] amino]-8-oxo-5-thia-1-azabicyclo[4.2.0]oct-2-en-2-carbonsäure-mononatriumsalz

In der in Beispiel 10 angegebenen Weise werden 200 mg (0,272 mmol) [6R-[6 alpha, 7 beta(Z)]]-3-[[-[3,4-bis(Acetyloxy)benzoyl]oxy]methyl]-7-[[[2-amino-4-thiazolyl][(2-amino-2-oxoethoxy)imino]acetyl]amino]-8-oxo-5-thia-1-azabicyclo[4.2.0]oct-2-en-2-carbonsäure-1,1-dimethylethylester (Beispiel 18) in 4 ml Methylenchlorid und 400 µl Anisol mit 4 ml Trifluoressigsäure behandelt. Nach 6-stündigem Rühren erhält man, nach Reverse Phase Chromatographie (0 - 30% Acetonitril in Wasser), 40 mg (21%) eines weissen Feststoffs.

NMR (DMSO-$d_6$): 2.28 (s) 3H (OAc); 2.29 (s) 3H (OAc); 3.57 (d, J=18Hz) 1H (1/2 $CH_2S$ durch HOD verdunkelt); 3.99 (s) 2H ($CH_2ON$); 4.94 5.27 (d von d, J=12H) 2H ($CH_2OCO$); 5.01 (d, J=6Hz) 1H (C6); 5.60 (d von d, J=6Hz J=10Hz) 1H (C7); 6.84 (s) 1H (Thiazol); 7.10 (s) 2H ($NH_2$); 7.28 2H ($NH_2$); 7.41 (d, J=8Hz) 1H (Ar); 7.84 (s) 1H (Ar); 7.89 (d, J=8Hz) 1H (Ar). IR (KBr): 3440, 1768, 1710, 1710, 1678, 1610. M/S: m/z 699 (M + H). UV ($H_2O$): λ max 234 nm (ε=26,260). HRMS berechnet (M + H) für $C_{25}H_{26}NaN_6O_{12}S_2$ : 699.0791. Gefunden: 699.0822.

## Beispiel 25

Herstellung von (6R-cis)-3-[[(Aminocarbonyl)oxy]methyl]-7-methoxy-8-oxo-7-(2-thienylacetyl)-amino-5-thia-1-azabicyclo[4.2.0]oct-2-en-2-carbonsäure-(4-nitrophenyl)methylester

3,75 g (8,30 mmol) p-Nitrobenzylbromid in 100 ml trockenem Dimethylformamid werden mit 3,75 g (8,30 mmol) (6R-cis)-3-[[(Aminocarbonyl)oxy]methyl]-7-methoxy-8-oxo-7-[(2-thienylacetyl)amino]-5-thia-1-azabicyclo[4.2.0]oct-2-en-2-carbonsäure-natriumsalz versetzt. Das Gemisch wird 2,5 Stunden gerührt und mit 100 ml Ethylacetat versetzt. Die Lösung wird mit Salzlauge gewaschen, über Magnisiumsulfat getrocknet und das Lösungsmittel unter vermindertem Druck abgedampft. Der Rückstand wird an Kieselgel chromatographiert unter Verwendung von Ethylacetat/n-Hexan als Eluens (1:1, anschliessend 2:1). Das erhaltene Produkt wird aus Ethylacetat/n-Hexan kristallisiert; man erhält 3,4 g (72%) gereinigtes Produkt.

NMR ($CDCl_3$): 3.31, 3.49 (d von d, J=18Hz) 2H ($CH_2S$); 3.44 (s) 3H ($OCH_3$); 3.79 (s) 2H ($CH_2CON$); 4.67 (br) 2H ($NH_2$); 4.82, 5.12 (d von d, J=14Hz) 2H ($CH_2O$); 5.03 (s) 1H (C6); 5.31, 5.60 (d von d, J=14Hz) 2H ($CH_2Ar$); 6.44 (s) 1H (NH); 6.99 (m) 2H (Thiophen); 7.25 (m) 1H (Thiophen); 7.56 (d, J=8Hz) 2H (Ar); 8.19 (d, J=8Hz) 2H (Ar). IR (KBr): 3465, 3335, 1785, 1728, 1698, 1522, 1348.

## Beispiel 26

Herstellung von (6R)-3-(Jodmethyl)-7-methoxy-8-oxo-7-[(2-thienylacetyl)amino]-5-thia-1-azabicyclo[4.2.0]oct-2-en-2-carbonsäure-(4-nitrophenyl)methylester

3,0 g (5,33 mmol) (6R-cis)-3-[[(Aminocarbonyl)oxy]methyl]-7-methoxy-8-oxo-7-[(2-thienylacetyl)amino]-5-thia-1-azabicyclo[4.2.0]oct-2-en-carbonsäure-(4-nitrophenyl)methylester (Beispiel 25) in 75 ml Methylenchlorid werden bei Zimmertemperatur mit 1,40 ml (10,3 mmol) Trimethylsilyljodid versetzt. Das Gemisch wird 30 Minuten gerührt und anschliessend mit wässriger Natriumbicarbonatlösung gewaschen und über Magnesiumsulfat getrocknet. Das Lösungsmittel wird unter vermindertem Druck abgedampft und der Rückstand an Kieselgel chromatographiert (Ethylacetat/n-Hexan 2:3) wobei 2,88 g (86%) eines hellgelben

Feststoffs erhalten wird.

NMR ($CDCl_3$): 3.45, 3.60 (d von d, J=16Hz) 2H ($CH_2S$); 3.49 (s) 3H ($OCH_3$); 3.94 (s) 2H ($CH_2CON$); 4.36, 4.50 (d von d, J=8Hz) 2H ($CH_2I$); 5.07 (s) 1H (C6); 5.36, 5.46 (d von d, J=16Hz) 2H ($CH_2Ar$); 6.46 (s) 1H (NH); 7.05 (m) 2H (Thiophen); 7.30 (m) 1H (Thiophen); 7.65 (d, J=8Hz) 2H (Ar); 8.27 (d, J=8Hz) 2H (Ar).

## Beispiel 27

Herstellung von (6R-cis)-3-[[[3,4-bis(Acetyloxy)benzoyl]oxy]methyl]-7-methoxy-8-oxo-7-[(2-thienylacetyl)-amino]-5-thia-1-azabicyclo[4.2.0]oct-2-en-2-carbonsäure-(4-nitrophenyl)methylester

1,90 g (3,02 mmol) (6R-cis)-3-(Jodmethyl)-7-methoxy-8-oxo-7-[(2-thienylacetyl)amino]-5-thia-1-azabicyclo[4.2.0]oct- 2-en-2-carbonsäure-(4-nitrophenyl)methylester (Beispiel 26) in 15 ml trockenem Dimethylformamid werden bei 0° C mit 0,78 g (3,00 mmol) Natrium-3,4-diacetoxybenzoat versetzt. Die Lösung wird 30 Minuten gerührt und mit 100 ml Ethylacetat versetzt. Die erhaltene Lösung wird mit Salzlauge gewaschen (3 x 50 ml) und über Magnesiumsulfat getrocknet.

Das Lösungsmittel wird unter vermindertem Druck abgedampft. Nach Chromatographie (n-Hexan/Ethylacetat 1:1) erhält man 1,30 g (58%) eines Gemisches der 2 und 3 Isomeren. Dieses Produkt wird in 30 ml Methylenchlorid bei 0° C gelöst und mit 400 mg (2,32 mmol) m-Chlorperbenzosäure versetzt. Das Gemisch wird bei 0° C 2 Stunden gerührt, mit wässriger Natriumbicarbonatlösung gewaschen, über Magnesiumsulfat getrocknet und das Lösungsmittel unter vermindertem Druck abgedampft. Nach Kristallisiation aus Ethylacetat/n-Hexan (5:2) erhält man 870 mg (66%) eines einheitlichen Sulfoxid-Isomeren. Die Mutterlauge wird an Kieselgel chromatographiert (Ethylacetat/Methylenchlorid 1:1) wobei 320 mg (24%) des isomeren Sulfoxids erhalten wird. Die vereinigten Sulfoxide (1.19 g 90%) werden reduziert, indem sie in 5 ml trockenem Dimethylformamid bei -20° C gelöst und mit 0,34 ml Phosphortribromid versetzt werden, gefolgt durch 1-stündiges Rühren. Nach dem Abdampfen des Lösungsmittels unter vermindertem Druck, gefolgt durch Kieselgel-Chromatographie (Ethylacetat/n-Hexan 1:1) erhält man 967 mg (83%) der Titelverbindung.

NMR ($CDCl_3$): 2.29 (s) 6H (2OAc); 3.33, 3.54 (d von d, J=18Hz) 2H ($CH_2S$) 3.44 (s) 3H ($OCH_3$); 3.88 (s) ($CH_2CON$); 4.98 (d, J=12Hz) 1H (1/2 $CH_2O$); 5.04 (s) 1H (C6); 5.34 (m) 3H (1/2 $CH_2O$, $CH_2$); 6.40 (s) 1H (NH); 6.99 (m) 2H (Thiophen); 7.24 (m) 2H (Thiophen, Ar); 7.54 (d, J=8H) 1H (Ar); 7.78 (s) 1H (Ar); 8.87 (d, J=8Hz) 1H (Ar); 8.16 (d, J=8Hz) 1H (Ar). IR (KBr): 3325, 1775, 1722, 1700, 1520, 1348. MS: m/z 740 (M + H).

## Beispiel 28

Herstellung von (6R-cis)-3-[[[3,4-bis(Acetyloxy)benzoyl]oxy]-methyl]-7-methoxy-8-oxo-7-[(2-thienylacetyl)-amino]-5-thia-1-azabicyclo[4.2.0]oct-2-en-2-carbonsäure-mononatriumsalz

Eine Lösung von 100 mg (0,135 mmol) (6R-cis)-3-[[[3,4-bis(Acetyloxy)benzoyl]oxy]methyl]-7-methoxy-8-oxo-7-[2-thienylacetyl)amino]-5-thia-1-azabicyclo[4.2.0]oct-2-en-2-carbonsäure-(4-nitrophenyl)methylester in 5 ml Tetrahydroforamin wird mit 100 mg 5%igem Platin auf Kohle versetzt. Das Gemisch wird 48 Stunden bei 3,5 kg/cm$^2$ hydriert. Der Katalysator wird abfiltriert und das Lösungsmittel unter vermindertem Druck abgedampft. Der Rückstand wird in 3 ml Ethylacetat und 3 ml 2%iger wässriger Natriumbicarbonatlösung aufgenommen und 15 Minuten heftig gerührt. Die wässrige Phase wird abgetrennt und chromatographiert (Reverse Phase, 0 - 30% Acetonitril in Wasser). Man erhält nach Lyophilisierung der das Produkt enthaltenden Fraktionen 45 mg (53%) eines weissen Feststoffs.

NMR (DMSO-$d_6$): 2.32 (s) 6H (2OAc); 3.19, 3.59 (d von d, J=18Hz) 2H ($CH_2$); 3.49 (s) 3H ($OCH_3$); 3.79,

3.89 (d von d, J=16Hz) 2H ($CH_2CO$); 4.98, 5.17 (d von d, J=14Hz) 2H ($CH_2O$); 5.00 (s) 1H (C6); 6.96 (m) 2H (Thiophen); 7.38 (m) 1H (Thiophen); 7.45 (d, J=8Hz) 1H (Ar); 8.84 (s) 1H (Ar); 8.91 (d, J=8Hz) 1H (Ar); 9.41 (s) 1H NH. IR (KBr): 3400, 1770, 1618, 1612. MS: m/z 672 (M + H). UV (EtOH): λ max 236 nm (ε=16,620). HRMS berechnet (M + H) für $C_{26}H_{24}NaN_2O_{11}S_2$ : 627.0719. Gefunden : 627.0676.

## Beispiel 29

Herstellung von (6R-cis)-3-[[(3,4-Dihydroxybenzoyl)oxy]methyl]-7-methoxy-8-oxo-7-[(2-thienylacetyl)amino]-5-thia-1-azabicyclo[4.2.0]oct-2-en-2-carbonsäure-mononatriumsalz

Eine Lösung von 65 mg (0,104 mmol) (6R-cis)-3-[[[3,4-bis(Acetyloxy)benzoyl]oxy]methyl]-7-methoxy-8-oxo-7-[(2-thienylacetyl)amino]-5-thia-1-azabicyclo[4.2.0]oct-2-en-2-carbonsäure-natriumsalz (Beispiel 28) in 4 ml gesättigter wässriger Natriumbicarbonatlösung wird mit 1 ml Methanol versetzt. Die Lösung wird 1 Stunde bei Zimmertemperatur gerührt. Das Methanol wird unter vermindertem Druck abgedampft und die zurückbleibende wässrige Lösung chromatographiert (Reverse Phase, 0 - 20% Acetonitril in Wasser). Man erhält nach Lyophilisierung der das Produkt enthaltenen Fraktionen 52 mg (92%) eines weissen Feststoffs.

NMR (DMSO-$d_6$): 3.11 (d von d, J=18Hz) 1H (1/2 $CH_2S$, andere Hälfte durch HOD verdunkelt); 3.37 (s) 3H ($OCH_3$); 3.78 (d von d, J=16Hz) 2H ($CH_2CO$); 4.83, 5.01 (d von d, J=12Hz, 2H ($CH_2O$); 4.98 (s) 1H (C6); 5.40 (br) 3H (2OH, NH); 6.38, 6.95 (m) 1H (Ar); (m) 2H (Thiophen); 7.11 (s) 1H (Ar); (d, J=8Hz) 1H (Ar); 7.36 (m) 1H (Thiophen). IR (KBr): 1762, 1675, 1610. MS: m/z 543 (M + H). UV (EtOH). λ max 222 nm (ε=15,960), λ max 265 nm (ε=10,280). (M + H) für $C_{22}H_{20}N_2O_9S_2Na$: 543.0508. Gefunden: 543.0506.

## Beispiel 30

Herstellung von [6R-[6 alpha, 7 beta(Z)]]-3-[[[3,4-bis(Acetyloxy)benzoyl]amino]methyl]-7-[[(2-amino-4-thiazolyl)(methoxyimino)acetyl]amino]-5-thia-1-azabicyclo[4.2.0]oct-2-en-2-carbonsäure-mononatriumsalz

Eine Lösung von 61,3 mg (0,258 mmol) 3,4-Diacetoxybenzosäure in 10 ml trockenem Dimethylformamid wird mit 68 mg (0,258 mmol) Triphenylphosphin versetzt, gefolgt durch 86 mg 2,2′-Dibenzothiazolyldisulfid. Nach 20 Minuten ist das Ganze in Lösung gegangen, und die Lösung wird 10 Minuten gerührt. Diese Lösung wird tropfenweise mit einer Lösung von 100 mg (0,206 mmol) [6R-[6 alpha, 7 beta(Z)]]-3-(Aminomethyl)-7-[[(2-amino-4-thiazolyl)(methoxyimino)acetyl]amino]-5-thia-1-azabicyclo[4.2.0]oct-2-en-2-carbonsäure-monohydrochloridsalz und 150 µl Triethylamin in 5 ml trockenem Dimethylformamid versetzt. Die erhaltene Lösung wird 3 Stunden gerührt und das Lösungsmittel unter vermindertem Druck abgedampft. Der Rückstand wird mit 5 ml Ethylacetat und 5 ml 2%iger wässriger Natriumbicarbonatlösung versetzt. Das Zweiphasengemisch wird 15 Minuten gerührt und die wässrige Phase abgetrennt und chromatographiert (Reverse Phase, 0 - 30% Acetonitril in Wasser); man erhält 62 mg (45%) eines weissen Feststoffs.

NMR (DMSO-$d_6$): 2.30 (s) 6H (2OAc); 3.21, 3.54 (d von d, J=18Hz) 2H ($CH_2S$); 3.82 (s) 3H ($OCH_3$); 3.99 (d von d, J=12Hz, J=4Hz) 1H (1/2 $CH_2N$); 4.05 (d von d, J=12Hz, 1H (1/2 $CH_2N$); 4.95 (d, J=6Hz) 1H (C6); 5.52 (d von d, J=6Hz, J=10Hz) 1H (C7); 6.72 (s) 1H (Thiazol); 7.20 (s) 2H ($NH_2$); 7.36 (d, J=8Hz) 1H (Ar); 7.70 (s) 1H (Ar); 7.75 (d, J=8Hz) IH (Ar); 9.20 (br) 1H (NH), 9.49 (d, J=10Hz) 1H (NH). IR (KBr): 3360, 1763, 1645, 1612. MS: m/z 655 (M + H). UV (EtOH): λ max 228 nm (ε=21,250). HRMS berechnet (M + H) für $C_{25}H_{25}NaN_6O_{10}S_2$: 655.0893. Gefunden 655.0886

## Beispiel 31

Herstellung von [6R-[6 alpha, 7 beta(Z)]]-3-[[[(3,4-Dihydroxy)benzoyl]amino]methyl]-7-[[(2-amino-4-thiazolyl)-(methoxyimino)acetyl]amino]-5-thia-1-azabicyclo[4.2.0]oct-2-en-2-carbonsäure-mononatriumsalz

In der in Beispiel 35 angegebenen Weise erhält man aus 20 mg (0,031 mmol) [6R-[6 alpha, 7 beta(Z)]]-3-[[[3,4-bis(Acetyloxy)benzoyl]amino]methyl]-7-[[2-amino-4-thiazolyl)(methoxyimino)acetyl]amino]-5-thia-1-azabicyclo[4.2.0]oct-2-en-2-carbonsäure-mononatriumsalz (Beispiel 30) in 1 ml gesättigter wässriger Natriumbicarbonatlösung und 500 µl Methanol nach Chromatographie (Reverse Phase, 0 - 20% Acetonitril in Wasser und Lyophilisierung 10 mg (60%) eines weissen Feststoffs.

NMR ($D_2O$): 3.38, 3.67 (d von d, J=18Hz) 2H ($CH_2S$); 3.99 (s) 3H ($OCH_3$); 4.13, 4.39 (d von d, J=14Hz) 2H ($CH_2N$); 5.21 (d, J=6Hz) 1H (C6); 5.30 (d, J=6Hz) 1H (C7); 6.75 (d, J=8Hz) 1H (Ar); 7.02 (s) 1H (Thiazol); 7.22 (m) 2H (Ar). HRMS berechnet (M + H) für $C_{21}H_{19}NaN_6O_8S_2$: 571.0862. Gefunden: 571.0646.

## Beispiel 32

Herstellung von [6R-[6 alpha, 7 beta(Z)]]-7-[[(2-Amino-4-thiazolyl)(methoxyimino)acetyl]amino]-3-[[(3,4-dihydroxybenzoyl)thio]methyl]-8-oxo-5-thia-1-azabicyclo[4.2.0]oct-2-en-2-carbonsäure-mononatriumsalz

Eine Aufschlämmung von 2.03 g (8,0 mmol) 3,4-bis(Acetyloxy)benzolcarbothionsäure in 80 ml Wasser wird mit 2,02 g (24,0 mmol) Natriumbicarbonat versetzt. Die erhaltene Lösung wird 15 Minuten bei 50 °C gerührt und anschliessend mit einer Lösung von 3,82 g (8,0 mol) 3-[(Acetyloxy)methyl]-7-[[(2-amino-4-thiazolyl)(methoxyimino)acetyl]amino]-8-oxo-5-thia-1-azabicyclo[4.2.0]oct-2-en-carbonsäure-mononatriumsalz in 20 ml Wasser versetzt. Das Reaktionsgemisch wird 18 Stunden bei 50 °C gerührt, abgekühlt, filtriert und chromatographiert ($C_{18}$, 0 - 20% Acetonitril in Wasser); man erhält nach Lyophilisierung 1,55 g (29%) Produkt.

NMR ($D_2O$): 3.35, 3.75 (d von d, J=18Hz) 2H ($CH_2S$); 3.86, 4.23 (d von d, J=14Hz) 2H ($CH_2SCO$); 3.98 (s) 3H ($OCH_3$); 5.19 (d, J=6Hz) 1H (C6); 5,79 (d, J=6Hz) 1H (C7); 6.96 (d, J=8Hz) 1H (Ar), 7.01 (s) 1H (Thiazol); 7.45 (s) 1H (Ar); 7.52 (d, J=8Hz). IR (KBr); 3345, 1770, 1695, 1600. UV ($H_2O$): $\lambda$ max 203 nm ( =36,500), $\lambda$ max 230 nm ($\epsilon$=32,000), max 250 nm ($\epsilon$=24.000). HRMS berechnet (M + H) für $C_{21}H_{19}NaN_5O_8S_3$ : 588.0294. Gefunden: 588.0287.

## Beispiel 33

Herstellung von [6R-[6 alpha, 7 beta(Z)]]-3-[[(3,4-bis(Acetyloxy)benzoyl)thio]methyl]-8-oxo-7-[[[2-[-(triphenylmethyl)amino]-4-thiazoyl](methoxyimino)acetyl]amino]-5-thia-1-azabicyclo[4.2.0]oct-2-en-2-carbonsäure-1,1-dimethylethylester

In der in Beispiel 4 angegebenen Weise werden 171 mg (0,208 mmol) [6R-[6 alpha, 7 beta(Z)]]-3-(Jodmethyl)-7-[[2-(methoxyimino)-2-[(tritylamino)-4-thiazolyl]-acetyl]amino] 8-oxo-5-thia-1-azabicyclo[4.2.0]-oct-2-en-2-carbonsäure-1,1-dimethylethylester in 2 ml Dimethylformamid mit 61 mg (0,220 mmol) 3,4-bis-(Acetyloxy)benzolcarbothionsäure-mononatriumsalz in 2 ml trockenem Dimethylformamid versetzt. Nach 3-stündigem Rühren erhält man nach Kieselgelchromatographie (9:1 Methylenchlorid/methanol) 112 mg (57%) eines cremefarbenen Feststoffs.

NMR ($CDCl_3$): 1.54 (s) 9H (t-Bu); 2.31 (s) 6H (2 OAc); 2.32, 2.62 (d von d, J=18Hz) 2H ($CH_2S$); 4.05, 4.30 (d von d, J=10Hz) 2H ($CH_2SCO$); 5.02 (d, J=6Hz) 1H (C6); 5.90 (d von d, J=6Hz, J=10Hz) 1H (C7) 6.68 (d, J=10Hz) 1H (NH) 6.70 (s) 1H (Thiazol); 6.99 (br s) 1H (NH) 7.26 (s) 16H ($CPh_3$, Ar); 7.81 (s) 1H (Ar); 7.86 (d, J=8Hz) 1H (Ar). IR (KBr): 1780, 1715, 1665. UV (EtOH): $\lambda$ max 214 nm ($\epsilon$=36,000), $\lambda$ max 267 nm ($\epsilon$=29,750). M.S.: M/Z 948 (M + H).

## Beispiel 34

Herstellung von [6R-[6 alpha, 7 beta(Z)]]-7-[[(2-Amino-4-thiazolyl)(methoxyimino)acetyl]amino]-3-[[[(3,4-bis-(acetyloxy)benzoyl]thio]methyl]-8-oxo-5-thia-1-azabicyclo[4.2.0]-oct-2-en-2-carbonsäure-mononatriumsalz

In der in Beispiel 10 angegebenen Weise werden 208 mg (0,219 mmol) [6R-[6 alpha, 7 beta(Z)]]-3-[[-(3,4-bis(Acetyloxy)benzoyl)thio]methyl]-8-oxo-7-[[[2-[(triphenylmethyl)amino]-4-thiazolyl](methoxyimino)-acetyl]amino]-5-thia-1-azabicyclo[4.2.0]oct-2-en-2-carbonsäure-1,1-dimethyl-ethylester (Beispiel 33) in 4 ml Methylenchlorid und 500 µl Anisol mit 4 ml Trifluoressigsäure behandelt. Nach 5-stündigem Rühren und Aufarbeitung nach Beispiel 10 erhält man, nach Reverse Phase Chromatographie (0 - 20% Acetonitril in Wasser), 77 mg (52%) eines weissen Pulvers.

NMR ($D_2O$): 2.40 (s) 6H (2OAc); 3.39, 3.73 (d von d, J=18Hz) 2H ($CH_2S$); 4.01 (s) 3H ($OCH_3$); 3.96. 4.39 (d von d, J=14Hz) 2H ($CH_2SCO$); 5.21 (d, J=6Hz) 1H (C6); 5.81 (d, J=6Hz) 1H (C7); 7.03 (s) 1H (Thiazol) 7.45 (d, J=8Hz) 1H (Ar); 7.89 (s) 1H (Ar); 8.98 (s) 1H (Ar). UV ($H_2O$): max 237 nm ($\epsilon$=20,200), $\lambda$ max 275 nm ($\epsilon$=17,000). M.S.: M/Z 672 (M + H).

## Beispiel 35

Herstellung von [6R-[6 alpha, 7 beta(Z)]]-3-[[[(3,4-bis(Acetyloxy)-2,5-dichlorbenzoyloxy]-methyl]-7-[[-(methoxyimino)[2-[(triphenylmethyl)amino]-4-thiazolyl]acetyl]amino]-8-oxo-5-thia-1-azabicyclo[4.2.0]oct2-en-carbonsäure-1,1-dimethylethylester

In der in Beispiel 4 angegebenen Weise werden 208 mg (0,253 mmol) [6R-[6 alpha, 7 beta(Z)]]-3-(Jodmethyl)-7-[[2-(methoxyimino-2-[(tritylamino)-4-thiazolyl]-acetyl]amino]-8-oxo-5-thia-1-azabicyclo[4.2.0]-oct-2-en-2-carbonsäure-1,1-dimethylethylester mit 87 mg (0,264 mmol) 3,4-bis(Acetyloxy)-2,5-dichlorbenzolsäure-natriumsalz in 2 ml Dimethylformamid versetzt. Nach 4-stündigem Rühren erhält man, nach Kieselgelchromatographie (9:1 Methylenchlorid/Methanol), 176 mg (70%) Produkt.

NMR ($CDCl_3$): 1.50 (s) 9H (t-Bu); 2.36 (s) 6H (2 OAc); 2.36, 2.60 (d von d, J=18Hz) 2H ($CH_2S$); 4.03 (s) 3H ($OCH_3$) 5.01 (m) 2H (1/2 $CH_2O$, C6) 5.38 (d, J=14Hz) 1H (1/2 $CH_2O$) 5.92 (d von d, J=6Hz, J=10Hz) 1H (C7); 6.68 (m) 2H (NH, Thiazol) 7.12 (s) 15H ($CPh_3$); 7.81 (s) 1H (Ar)

## Beispiel 36

Herstellung von [6R-[6 alpha, 7 beta(Z)]]-7-[[(2-Amino-4-thiazolyl)(methoxyimino)acetyl]amino]-3-[[[(3,4-bis-(acetyloxy)-2,5-dichlorbenzoyl]oxy] methyl]-8-oxo-5-thia-1-azabicyclo[4.2.0]oct-2-en-2-carbonsäure-mononatriumsalz

Eine Lösung von 146 mg (0,146 mmol) [6R-[6 alpha, 7 beta(Z)]]-3-[[[3,4-bis(Acetyloxy]-2,5-dichlorbenzoyl]oxy] methyl]-7-[[(methoxyimino)[2-[(triphenylmethyl)amino]-4-thiazolyl]amino]-8-oxo-5-thia-1-azabicyclo[4.2.0]oct-2-en-2-carbonsäure-1,1-dimethylethylester (Beispiel 35) in 4,6 ml 70%iger wässriger Ameisensäure wird bei Zimmertemperatur 3 Stunden gerührt. Das Lösungsmittel wird unter vermindertem Druck abgedampft und der Rückstand an Kieselgel chromatographiert (0 - 100% Ethylacetat in Methylenchlorid), wobei 80,7 mg (72%) enttritylliertes Produkt erhalten wird. Eine Lösung von 39 mg (0.051 mmol) dieses Produkts in 1,4 ml Methylenchlorid und 100 µl Anisol wird auf 0° C gekühlt und mit 1,2 ml Trifluoressigsäure behandelt. Das Reaktionsgemisch wird 6,5 Stunden bei 0° C gerührt und das Lösungsmittel anschliessend unter vermindertem Druck abgedampft. Der ölige Rückstand wird in 1 ml Aceton gelöst und unter Rühren zu 50 ml n-Hexan gegeben. Der entstandene Niederschlag wird abfiltriert, in 0,5 ml

Ethylacetat gelöst und mit 9,0 mg (0,054 mmol) 2-Ethyl-natrium-n-hexanoat behandelt. Der entstandene Niederschlag wird abgetrennt und getrocknet. Man erhält 7 mg (19%) eines weissen Feststoffs.

NMR ($D_2O$). 2.30 (s) 6H (2 OAc); 3.30, 3.60 (d von d, J=18Hz) 2H ($CH_2S$); 3.82 (s) 3H ($OCH_3$) 4.80 (d, J=12Hz) 1H (1/2 $CH_2O$); 5.06 (m) 2H (1/2 $CH_2O$, C6); 5.66 (d, J=6Hz) 1H (C7); 6.84 (s) 1H (Thiazol); 7.91 (s) 1H (Ar).

Beispiel 37

Herstellung von [(6R-trans)-3-[[[[3,4-bis(Acetyloxy)phenyl]amino]carbonyl]oxy]methyl]-7-[[(1,1-dimethylethoxy)carbonyl]amino]-8-oxo-5-thia-1-azabicyclo[4.2.0]oct-2-en-2-carbonsäure-diphenylmethylester

Eine Lösung von 160 mg (0,8 (mmol) 3,4-bis-(Acetyloxy)phenyl isocyanat in 7 ml trockenem Metylenchlorid wird mit 0,2 ml Pyridin und anschliessend mit 198 mg (0,4mmol) (6R-trans)-3-[Hydroxymethyl]-7-[[(1,1-dimethylethoxy)carbonyl]amino]-8-oxo-5-thia-1-azabicyclo[4.2.0]oct-2-en-2carbonsäure-diphenylmethylester in einer Argonatmosphäre bei Zimmertemperatur versetzt. Das Reaktionsgemisch wird 5 Stunden gerührt und das Lösungsmittel anschliessend unter vermindertem Druck abgedampft. Der Rückstand wird in Ethylacetat aufgenommen und die organische Lösung mit verdünnter Salzsäure gewaschen und über wasserfreiem Natriumsulfat getrocknet. Das so erhaltene Rohprodukt wird an Kieselgel chromatographiert (Ethylacetat/n-Hexan, 1:1 wobei 250 mg (84%) eines weissen Feststoffs erhalten wird.

NMR ($CDCl_3$): 1.46 (s) 9H (t-bu); 2.28 (s) 6H (2-OAc); 3.43, 3.64 (d von d, J=18Hz) 2H ($CH_2S$); 4.84, 5.18 (d von d, J=12Hz) 2H ($CH_2O$); 4.95 (d, J=6Hz) 1H (C6); 5.68 (d von d, J=6Hz J=10Hz) 1H (C7); 5.23 (d, J=10Hz) 1H (NH); 6.62 (s), 1H (Thiazol, Ar); 7.0 (s) 1H (CH $Ar_2$); 7.14 (s) 2H (Ar); 7.36 (m) 1H (Ar). IR (Kbr): 3340, 1773, 1722, 1530, 700.

Beispiel 38

Herstellung von [6R-[6 alpha, 7 beta(Z)]]-3[[[[3,4-bis(Acetyloxy)phenyl]amino] carbonyl]oxy]methyl]-7-[[(2-amino-4-thiazolyl)(methoxyimino)acetyl]amino]-8-oxo-5-thia-1-azabicyclo[4.2.0]oct-2-en-2-carbonsäure-mononatriumsalz

Eine Lösung von 233 mg (0,31 mmol) [(6R-trans)-3-[[[[3,4-bis(Acetyloxy)phenyl]amino]carbonyl]oxy]-methyl]-7-[[(1,1-dimethylethoxy)carbonyl]amino]-8-oxo-5-thia-1-azabicyclo[4.2.0]oct-2-en-2-carbonsäure-diphenylmethylester (Beispiel 37) in 5 ml trockenem 1,2-Dichlorethan wird auf 0° C abgekühlt und mit 0,6 ml Anisol und anschliessend mit 4 ml Trifluoressigsäure behandelt. Das Reaktionsgemisch wird 5 Stunden bei 0° C gerührt und das Lösungsmittel anschliessend unter vermindertem Druck abgedampft. Der Rückstand wird mit Hilfe von wasserfreiem Diethylether ausgefällt und abfiltriert. Der erhaltene Feststoff (130 mg) in 10 ml trockenem Methylenchlorid und 0,1 ml Triethylamin werden bei 0° C zu 106 mg (0,33 mmol) s-(2-Benzothiazoyl)-(Z)-aminothio-4-thiazol-glyoxylate-0-(methyl)-oxim und 5 ml Aceton gegeben. Das Gemisch wird in einer Argonatmosphäre bei Zimmertemperatur 15 Stunden gerührt. Das Lösungsmittel wird unter vermindertem Druck abgedampft und der Rückstand zu 5 ml Ethylacetat und 10 ml 1%iger wässriger Natriumbicarbonatlösung gegeben. Die wässrige Lösung wird an einem $C_{18}$ Reverse Phase Chromatograph (0 - 20% Acetonitril in Wasser) gereinigt, wobei 39 mg (20%) des gewünschten Produktes erhalten wird.

NMR (DMSO-$d_6$): 2,23 (s) 3H (OAc); 2.26 (s) 3H (OAc); 3.24, 3.50 (d von d, J=18Hz) 2H ($CH_2S$); 3.84 (s) 3H ($OCH_3$); 4.90, 4.98 (d von d, J=12Hz) 2H ($OCH_2$); 5.0 (d, J=6Hz) 1H (C6); 5.60 (d von d, J=6Hz J=10Hz) 1H (C7); 6.78 (s) 1H (Thiazol); 7.18 (d, J=8Hz) 1H (Ar); 7.32 (d von d, J=3Hz J=8Hz) 1H (Ar) 7.44 (d, J=3Hz) 1H (Ar); 7.30 (br s) 2H ($NH_2$); 9.56 (br d, J=10Hz), 1H (NH). IR (KBr): 3330, 1762, 1672, 1732 inf. 1612.

## Beispiel 39

Herstellung von [6R-[6 alpha, 7 beta(Z)]]-3-[[[[[3,4-bis (Acetyloxy)phenyl]amino]carbonyl]oxy]methyl]-7-[[[2-amino[(2-amino-2-oxoethoxy)imino]-4-thiazolyl]acetyl] amino]-8-oxo-5-thia-1-azabicyclo[4.2.0]oct-2-en-2-carbonsäure-mononatriumsalz

In der in Beispiel 38 angegebenen Weise werden 233 mg (0,31 mmol) [(6R-trans)-3-[[[[[3,4-bis-(Acetyloxy)phenyl]amino]carbonyl]oxy]methyl]-7-[[(1,1-Dimethylethoxy)carbonyl]amino]-8-oxo-5-thia-l-azabicyclo[4.2.0]oct-2-en-2-carbonsäure-diphenylmethylester (Beispiel 37) in 5 ml trockenem 1,2-Dichlorethan und 0,6 ml Anisol mit 4 ml Trifluoressigsäure behandelt,gefolgt durch Umsetztung mit 87 mg (0,22 mmol) s-(2-Benzothiazolyl)-Z-aminothio-4-thiazol-glyoxylat-O-(carbamoylmethyl)oxim. Reinigung an einer $C_{18}$ Reverse Phase Chromatographie-Kolonne (0 - 20% Acetonitril in Wasser) ergibt 48 mg (23%) eines weissen Feststoffs.

NMR (DMSO-$d_6$) 2.23 (s) 3H (OAc); 2.26 (s) 3H (OAc); 3.24, 3.52 (d von d, J=16Hz) 2H ($CH_2S$); 4.42 (s) 2H ($CH_2CON$); 4.93 (s) 2H ($OCH_2$); 5.03 (d, J=6Hz) 1H (C6); 5.64 (d von d, J=6Hz J=10Hz) 1H (C7); 6.87 (s) 1H (Thiazol); 7.17 (d, J=9Hz) 1H (Ar); 7.32 (d von d, J=9Hz) 1H (Ar); 7.35 (br s) 2H ($NH_2$); 7.42 (d, J=3Hz) 1H (Ar); 9.75 (d, J=10Hz) 1H (NH); 10.15 (br s) 1H (NH).

## Beispiel 40

Herstellung von [(6R-trans)-3-[[[(3,4-bis(Acetyloxy)benzoyl]thio]methyl] -8-oxo-7-[(phenoxyacetyl)amino]-5-thia-1-azabicyclo [4.2.0]oct-2-en-2-carbonsäure-1,1-dimethylethylester

In der in Beispiel 4 angegebenen Weise werden 411 mg (0.775 mmol) [6R-[6 alpha, 7 beta(Z)]]-3-Jodmethyl)-7-[(phenoxyacetyl)amino]-8-oxo-5-thia-1-azabicyclo[4.2.0]oct-2-en-2-carbonsäure-1,1-dimethylethylester in 5 ml Dimethylformamid mit 230 mg (0.832 mol) 3,4-bis(Acetyloxy)-benzolcarbothionsäure-natriumsalz in 5 ml Dimethylformamid versetzt. Nach 2 1/2-stündigem Rühren erhält man, nach Kieselgelchromatographie (9:1 Methylenchlorid/Ethylacetat), 206 mg (40%) eines weissen Feststoffs.

NMR ($CDCl_3$): 1.44 (s) 9H (t-Bu); 2.42 (s) 8H (2 OAc); 3.35, 3.61 (d von d, J=18Hz) 2H ($CH_2S$); 4.00, 4.29 (d von d, J=12Hz) 2H ($CH_2SCO$); 4.55 (s) 3H ($OCH_3$); 5.01 (d, J=6Hz), 1H (C6) 5.91 (d von d, J=6Hz, J=10Hz) 1H (C7); 6.90 (d, J=8Hz) 1H (Ar); 7.00 (m) 1H (Ar); 7.2, 7.3 (m) 3H (Ar); 7.79 (s) 1H (Ar); 7.86 (d, J=8Hz) 1H (Ar). IR (KBr): 3320, 1780, 1712, 1692, 1662, UV (EtOH): $\lambda$ max 248 nm ($\epsilon$=17,000), $\lambda$ max 274 nm ($\epsilon$=1,850).

## Beispiel 41

Herstellung von (6R-trans)-7-Amino-3-[[[3,4-bis(acetyloxy)benzoyl]thio]methyl]-8-oxo-5-thia-1-azabicyclo-[4.2.0]oct-2-en-2-carbonsäure-1,1-dimethylethylester-monohydrochlorid

In der in Beispiel 16 angebenen Weise werden 27 µl (0.333 mmol) Pyridin und 61.7 mg (0.296 mmol) Phosphorpentachlorid mit 147 mg (0.233 mmol) (6R-trans)-3-[[[3,4-bis (Acetyloxy)benzoyl]thio]methyl]-8-oxo-7-[(phenoxyacetyl) amino]-5-thia-1-azabicyclo[4.2.0]oct-2-en-2-carbonsäure-1,1-Dimethylethylester (Beispiel 40) in 0.7 ml Methylenchlorid versetzt. Man erhält 70.5 mg (56%) eines weissen Pulvers.

NMR (DMSO-$d_6$): 1.53 (s) 9H (t-Bu); 2.30 (s) 3H (OAc); 2.31 (s) 3H (OAc); 3.54, 3.83 (d von J, J = 18Hz) 2H ($CH_2S$); 3.96, 4.33 (d von d, J = 12Hz) 2H ($CH_2SCO$); 5.17 (d, J = 6Hz) 1H (C6); 5.22 d, J = 6Hz) 1H (C7); 7.48 (d, J = 8Hz) 1H (Ar); 7.84 (s) 1H (Ar); 7.88 (d, J = 8Hz) 1H (Ar). IR (KBr): 1780, 1712, 1668. UV (EtOH) $\lambda$ max 277 nm ($\epsilon$ = 16,760).

## Beispiel 42

[6R-[6alpha,7beta(Z)]]-3-[[(3,4-Dihydroxybenzoyl)oxy]methyl]-7-[[[2-amino-4-thiazolyl][(2-amino-2-oxoethoxy)imino]acetyl]amino-8-oxo-5-thia-1-azabicyclo[4.2.0]oct-2-en-2-carbonsäure-mononatriumsalz

In der in Beispiel 20 angegebenen Weise werden 350 mg (0.477 mmol) [6R-[6alpha,7beta(Z)]]-3-[[[3,4-bis(Acetyloxy)benzoyl]oxy]methyl]-7-[[[2-amino-[(2-amino-2-oxoethoxy)imino]-4-thiazolyl]acetyl]amino]-8-oxo-5-thia-1-azabicyclo[4.2.0]oct-2-en-2--carbonsäure-1,1-dimethylethylester in 5 ml Methylenchlorid und 500 µg Anisol mit 5 ml Trifluoressigsäure behandelt, gefolgt durch Rühren mit Ethylacetat/Methanol und gesättigter, wässriger Natriumbicarbonatlösung (1:1:1). Man erhält 165 mg (54%) eines weissen Feststoffs nach Reverse Phase Chromatographie (0-20% Acetontril in Wasser.

NMR (DMSO$d_6$): 3.56 (d, J = 18Hz) 1H (1/2 $CH_2S$); 4.41 (s) 2H ($CH_2$); 4.89 5.17 (d von d, J = 12Hz) 2H ($CH_2OCO$); 5.05 (d,J = 6Hz) 1H (C6); 5.64 (d von d, J = 6Hz, J = 10Hz) 1H (C7); 6.77 (d, J = 8Hz) 1H (Ar); 6.84 (s) 1H (thiazole); 7.12 (s) 1H (OH); 7.29 (d, J = 8Hz) 1H (Ar); 7.34 (s) 1H (Ar); 7.50 (s) 1H (OH) 9.75 (d, J = 8Hz) 1H (NH), I.R. (KBr); 1762 1672 1608. UV ($H_2O$): $\lambda$ max 218 nm ($\epsilon$ = 17,900), $\lambda$ max 261 ($\epsilon$ = 14,350). Berechnet für $C_{22}H_{21}N_6O_{10}S_2Na$ (M + H): 616.0614. Gefunden: 616.0658

## Beispiel 43

[6R-[alpha,7beta(Z)]]-3-[[[3,4-bis(Acetyloxy)benzoyl]thio]methyl]-7-[[[(2-amino-2-oxoethoxy)imino](2-amino-4-thiazolyl)-acetyl]amino]-8-oxo-5-thia-1-azabicyclo[4.2.0]oct-2-en-2-carbonsäure-1,1-dimethylethylester

In der in Beispiel 18 angebenen Weise werden 243 mg (0.434 mmol) (6R-trans)-3-[[[3,4-bis(Acetyloxy)-benzoyl]thio]methyl]-7-amino-8-oxo-5-thia-1-azabicyclo[4.2.0]oct2-en-2-carbonsäure-1,1-dimethylethylester-monohydrochlorid mit 158 mg (0.400 mmol) S-(2-Benzothiazolyl)-Z-aminothio-4-thiazolyl-glyoxylat-O-(carbamoylmethyl)oxim in 8 ml Dimethylformamid und 16 ml Methylenchlorid umgesetzt. Man erhält nach dem Chromatographieren (0-50% Methanol in Methylenchlorid) 180 mg eines weissen Feststoffs.

NMR ($CDCl_3$): 1.57 (s) 9H (t-Bu); 2.31 (s) 6H (2 OAc); 3.39, 3.63 (d von d, J = 18Hz) 2H ($CH_2S$) 3.98, 4.36 (d von d J = 15Hz) 2H ($CH_2SCO$); 5.07 (d, J = 6Hz) 1H (C6); 5.60 (br) 2H ($NH_2$); 5.95 (d von d, J = 6Hz, J = 10Hz) 1H (C7); 6.85 (s) 1H Thiazol); 7.30 (d, J = 8Hz) 1H (Ar); 7.80 (d, J = 2Hz) 1H (Ar); 7.88 (d von d, J = 2Hz, J = 8Hz) 1H (Ar); 8.32 (br) 1H (NH). IR (KBr): 1778, 1710, 1672, UV (EtOH): max 240 nm ($\epsilon$ = 20,750), max 278 nm ($\epsilon$ = 17,300). M.S.: M/Z 749 (M + H).

## Beispiel 44

[6R-[6alpha,7beta(Z)]]-7-[[[2-Amino[(2-amino-2-oxoethoxy]imino]-4-thiazolyl]acetyl]amino]-3-[[(3,4 dihydroxy-benzoyl)thio]-methyl]-8-oxo-5-thia-1-azabicyclo[4.2.0]oct-2-en-2-carbonsäure-mononatriumsalz

In der in Beispiel 20 angegebenen Weise werden 305 mg (0.408 mmol) [6R-[6alpha,7beta(Z)]]-3-[[(3,4-bis(Acetyloxy)benzoyl]thio]-methyl]-7-[[(2-amino-2-oxoethoxy)imino](2-amino-4-thiazolyl)acetyl]amino]-8-oxo-5-thia-1-azabicyclo[4.2.0]oct-2-en-2-carbonsäure-1,1-dimethylethylester (Beispiel 43) in 6 ml Methylenchlorid und 600 µl Anisol mit 13 ml Trifluoressigsäure behandelt, gefolgt durch Rühren mit Ethylacetat, Methanol und gesättigter wässriger Natriumbicarbonatlösung (1:1:1). Man erhält, nach Reverse Phase Chromatographie (0-20% Acetonitril in Wasser), 200 mg eines weissen Feststoffes.

NMR (DMSO-$d_6$): 3.09, 3.51 (d von d, J=18Hz) 2H ($CH_2S$); 4.03, 4.18 (d von d, J=12Hz) 2H ($CH_2SCO$) 4.41 (s) 2H ($OCH_2$); 5.00 (d, J=6Hz) 1H (C6); 5.59 (d von d J=6Hz, J=10Hz) 1H (C7); 6.79 (d, J=8Hz) 1H (Ar); 6.83 (s) 1H (Thiazol); 7.09 (s) 1H (OH); 7.30 (m) 6H (2Ar, $2NH_2$) 7.48(s)1H (OH); 9.73 (d, J=10Hz) 1H (NH). IR(KBr): 1760, 1672, 1592, 1162, 1088, UV ($H_2O$): λ max 230 (ε=16850); λ max 282 (ε=12,900). HRMS berechnet für $C_{22}H_{19}N_6O_9S_3Na$: 631.0375: Gefunden: 631.0352.

## Beispiel 45

[6R-[6alpha,7beta(Z)]]-3-[[[3,4-bis(Acetyloxy)benzoyl]oxy]methyl]-7-[[(2-amino-4-thiazolyl)[[2-(1,1-dimethylethoxy)-2-oxoethoxy]imino]acetyl]amino]-8-oxo-5-thia-1-azabicyclo[4.2.0]oct-2-en-2-carbonsäure-1,1-dimethylethylester

In der in Beispiel 18 angegebenen Weise werden 1,20 g (2.37 mmol) (6R-trans)-7-amino-3-[[[3,4-bis-(acetyloxy)benzoyl]oxy]methyl-8-oxo-5-thia-1-azabicyclo[4.2.0]oct-2-en-2-carbonsäure-1,1-dimethylethylester-monohydrochlorid in 46 ml Methylenchlorid und 1,0 g (222 mmol) 2-[[[1-(2-Amino-4-thiazolyl)-2-(benzothiazol-2-yl-thio)-2-oxoethyl]imino]oxy]essigsäure-1,1-dimethylethylester in 46 ml Dimethylformamid und 23 ml Methylenchlorid umgesetzt. Nach 4-stündigem Rühren erhält man nach Kieselgel-Chromatographie (8:2 Methylenchlorid/Ethylacetat) 920 mg eines weisses Feststoffs.

NMR ($CDCl_3$) 1.42 (s) 9H (t-bu); 1.55 (s) 9H (t-but); 2.31 (s) 6H (2OAC); 3.41, 3.62 (d von d, J=18Hz) 2H ($CH_2S$); 4.73, 4.77 (d von d, J=17Hz) 2H ($CH_2OCOAr$); 5.06 5.39 d von d, J=16Hz) 2H) ($OCH_2CO$); 5.09 (d, J=7Hz) 1H (C6); 5.97 (d von d, J=7Hz J=10Hz) 1H (C7); 7.03 (s) 1H (Thiazol); 7.29 (d, J=8Hz e.c.) 1H (Ar); 7.85 (d, J=2Hz) 1H (Ar); 7.95 (d von d, J=2Hz, J=8Hz) 1H (Ar); 8.63 (d, J=10Hz) 1H (NH). IR (KBr): 3400, 1780, 1722, 1682. UV (EtOH): λ max 236 nm (ε=27,590). M.S.: M/T 790 (M+H).

## Beispiel 46

[6R-[6alpha,7beta(Z)]-7-[[(2-Amino-4-thiazolyl)[(carboxymethoxy)imino]acetyl]amino]-3-[[3,4-dihydroxybenzoyl)oxy]methyl]-8-oxo-5-thia-1-azabicyclo[4.2.0]oct-2-en-2-carbonsäure-dinatriumsalz

In der in Beispiel 20 angegebenen Weise werden 920 mg (1.16 mmol) [6R-[6alpha,7beta(Z)]]-3-[[[3,4-bis(Acetyloxy)benzoyl]oxy]methyl]-7-[[(2-amino-thiazolyl)[2-(1,1-dimethyl ethoxy-2-oxoethoxy]imino]acetyl]-amino]-8-oxo-5-thia-1-azabicyclo[4.2.0]oct-2-en-2-carbonsäure-1,1-dimethylethylester in 24 ml Methylenchlorid und 2.3 ml Anisol mit 24 ml Trifluoressigsäure behandelt, gefolgt durch Rühren des Rückstandes mit Ethylacetat, Methanol und gestättigter wässriger Natriumbicarbonatlösung. Man erhält nach Reverse Phase Chromatographie (0-30% Acetonitril in Wasser), 385 mg (62%) eines weissen Feststoffs.

NMR ($D_2O$) 3.28, 3.56 (d von d, J=18Hz) 2H ($CH_2S$), 4.42 (S) 2H ($CH_2CO_2Na$), 4.67 4.96 (d von d, J=15Hz) 2H ($CH_2O$), 5.07 (d, J=6Hz) 1H (C6), 5.68 (d, J=6Hz) 1H (C7), 6.71 (d, J=8Hz) 1H (Ar) 6.87 (S9 1H (Thiazol) 7.30 (m) 2H (Ar); IR (KBr): 1755, 1650, 1602; UV ($H_2O$): λ max 203 nm (ε=17,600), λ max 231 nm (ε=15,400), λ max 260 nm (ε=9,800), max 302 (ε=8,080).

## Beispeil 47

(6R-trans)-7-[[(2-Amino-4-thiazolyl)[[1-(aminocarbonyl)-1-methylethoxy]imino]acetyl]amino]-3-[[[3,4-bis-(acetyloxy)benzoyl]oxy]methyl]-8-oxo-5-thia-1-azabicyclo[4.2.0]oct-2-en-carbonsäure-1,1-dimethylethylester

Eine Lösung von 542 mg (1.01 mmol) (6R-trans)-7-Amino -3-[[[3,4-bis(Acetyloxy)benzoyl]oxy]methyl]-8-oxo-5-thia-1-azabicyclo[4.2.0]oct-2-en-2-carbonsäure-1,1-dimethylethylester-monohydrochlorid in 25 ml Methylenchlorid wird mit 2x25 ml einer gesättigten wässrigen Natriumbicarbonat/Kochsalz Lösung (1:4) gewaschen. Die organische Lösung wird über Magnesiumsulfat getrocknet und filtriert. Diese Lösung wird mit einer Lösung von 411 mg (1.00 mmol) 2-2-Amino-α-[(2-amino-1,1-dimethyl-2-oxoethoxy)imino]-4-thiazolessigsäure, 136 mg (1.00 mmol) N-Hydroxybenzotriazol (NHBT) und 211 mg (1.00 mmol) Dicyclohexylcarbodiimid (DCC) in 25 ml trockenem Dimethylformamid versetzt. Die Lösung wird 24 Stunden gerührt; man erhält nach Kieselgel-Chromatograpie 320 mg (42%) Endprodukt.

NMR ($CDCl_3$: 1.50 (s) 6H (2 $CH_3$); 1.54 (s) 9H (t-bu); 3.42, 3.61 (d von d, J=18Hz); 5.06 (m) 2H (1/2 $CH_2O$, C6); 5.32 (d, J=17Hz) 1H (1/2 $CH_2O$); 5.90 (d von d, J=5Hz, J=9Hz) 1H (C7); 6.40 (br s) 2H ($NH_2$); 6.75 (s) 1H (Thiazol); 7.28 (d, J=8Hz) 1H (Ar); 7.83 (d, J=2Hz) 1H (Ar); 7,93 (d von d, J=2Hz, J=8Hz). I.R. (KBr) 1780, 1722, 1680, 1532. UV (EtOH): λ max 236 nm (ε=29,050). M.S.: M/Z 761 (M+H).

Beispiel 48

(6R-trans)-7-[[(2-Amino-4-thiazolyl)[[1-(aminocarbonyl)-1-methylethoxy]imino]acetyl]amino]-3-[[(3,4-dihydroxybenzoyl)-oxy]methyl]-8-oxo-5-thia-1-azabicyclo[4.2.0]oct-2-en-2-carbonsäure-mononatriumsalz

In der in Beispiel 20 angegebenen Weise werden 320 mg (6R-trans)-7-[[(2-Amino-4-thiazolyl)[[l-(aminocarbonyl-1-methylethoxy]imino]acetyl]amino]-3-[[[3,4-bis(acetyloxy)benzoyl]oxy]methyl-8-oxo-5-thia-1-azabicyclo[4.2.0]oct-2-en-2-carbonsäure-1,1-dimethylethylester in 5 ml Methylen chlorid und 500 µl Anisol mit 5 ml Trifluoressigsäure behandelt und anschliessend 3 Stunden gerührt. Der Rückstand wird mit Ethylacetat, Methanol und gesättigter, wässriger Natriumbicarbonatlösung behandelt; man erhält, nach Reverse Phase Chromatographie (0-30% Actontril in Wasser), 123 mg (45%) eines weissen Feststoffs.

NMR ($D_2O$): 1.60 (s) 6H (2 $CH_3$) 3.53, 3.78 (d von d, J=18Hz) 2H ($CH_2S$ 4.93, 5.17 (d von d, J=17Hz) 2H ($CH_2O$); 5.28 (d, J=4Hz) 1H (C6); 5.90 (d, J=4Hz) 1H (C7); 688 (d, J=8Hz) 1H (Ar); 7.12 (s) 1H (Thiazol); 7.50 (s) 1H (Ar); 7.55 (d, J=8Hz) 1H (Ar). IR (KBr): 1762, 1672, 1605. UV ($H_2O$): λ. max 218 nm (ε=22,300). Berechnet für $C_{24}H_{23}N_6O_{12}S_2N_2$: 643.0947. Gefunden 643.0893.

Beispiel A

Herstellung von Trockenampullen für die intramuskuläre Verabreichung:

Es wird in üblicher Weise ein Lyophilisat von [6R-[6alpha,7 beta(Z)]]3-[[3,4]-Dihydroxybenzoyloxy]-methyl]-7-[[[2-amino-4-thiazolyl][(2-amino-2-oxoethoxy)imino] acetyl]amino]-8-oxo-5-thia-1-azabicyclo[4,2,g]-oct-2-en-2-carbonsäure-mononatriumsalz hergestellt und in eine Ampulle abgefüllt. Die sterile Wasserampulle enhält 10% Propylenglykol. Vor der Verabreichung wird das Lyophilisat mit 2.5 ml einer 2%-igen wässrigen Lidocainhydrochloridlösung versetzt.

**Ansprüche**

1. Acylderivate der allgemeinen Formel

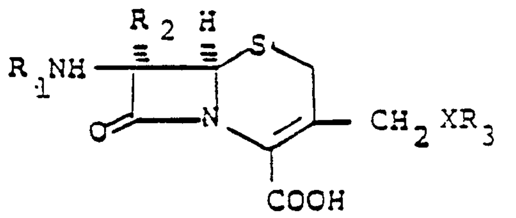

I

in der X -OCO-, -SCO-, -NHCO- oder -OCONH-; $R_1$ eine Acylgruppe; $R_2$ Wasserstoff oder niederes Alkoxy; und $R_3$ die Gruppe

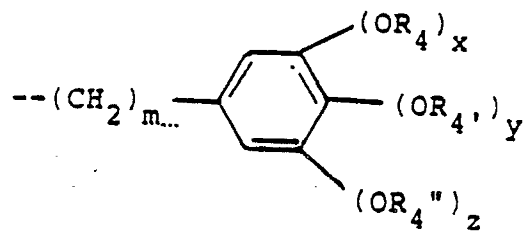

oder

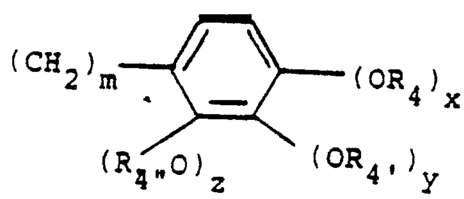

oder

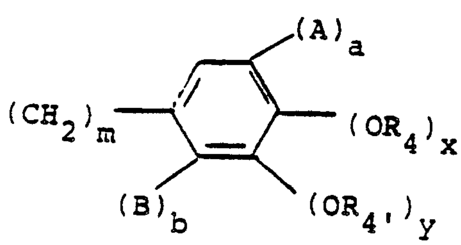

darstellen,

worin $R_4$, $R_4'$ und $R_4''$ unabhängig voneinander Wasserstoff oder $-COR_{200}$ mit $R_{200}$ geradkettigem oder verzweigtem niederem Alkyl, A und B Halogen und a, b, x, y und z unabhängig voneinander Null (wobei in der entsprechenden Ringposition ein Wasserstoffatom vorliegt) oder 1 bedeuten, wobei mindestens zwei der Symbole x, y und z 1 darstellen, und m Null oder eine Zahl von 1 bis 8 bedeutet,
sowie leicht hydrolysierbare Ester und pharmazeutisch verträgliche Salze dieser Verbindungen und Hydrate der Verbindungen der Formel I bzw. von deren Estern und Salzen.

2. Verbindungen nach einem Anspruch 1, dadurch gekennzeichnet, dass m Null oder 1 darstellt.

3. Verbindungen nach Anspruch 1 oder 2, dadurch gekennzeichnet, dass $R_4$, $R_4'$ und $R_4''$ (sofern vorhanden) unabhängig voneinander Wasserstoff oder $-COCH_3$ darstellen.

4. Verbindungen nach einem der Ansprüche 1 bis 3, dadurch gekennzeichnet, dass A und B Chlor darstellen.

5. Verbindungen nach einem der Ansprüche 1 bis 4, dadurch gekennzeichnet, dass R Wasserstoff darstellt.

6. Verbindungen nach einem der Ansprüche 1 bis 5, dadurch gekennzeichnet, dass X -OCO- darstellt.

7. Verbindungen nach einem der Ansprüche 1 bis 5, dadurch gekennzeichnet, dass X-SCO- darstellt.

8. Verbindungen nach einem der Ansprüche 1-5, dadurch gekennzeichnet, das X -NHCO- darstellt.

9. Verbindungen nach einem der Ansprüche 1-5, dadurch gekennzeichnet, dass X -OCONH- darstellt.

10. Verbindungen nach einem der Ansprüche 1-9, dadurch gekennzeichnet, dass die Acylgruppe $R_1$ ein aliphatische Gruppe der Formel

$R_5CO$-

in der $R_5$ niederes Alkyl, niederes Cycloalkyl, niederes Alkoxy, niederes Alkenyl, niederes Cycloalkenyl oder Cyclohexadienyl; oder durch einen oder mehrere der Reste Halogen, Cyan, Nitro, Amino, Mercapto, niederes Alkylthio oderr Cyanmethylthio substituiertes niederes Alkyl oder niederes Alkenyl darstellt, bedeutet.

11. Verbindungen nach einem der Ansprüche 1 bis 9, dadurch gekennzeichnet, dass die Acylgruppe $R_1$ eine carbocyclische aromatische Gruppe der allgemeinen Formel

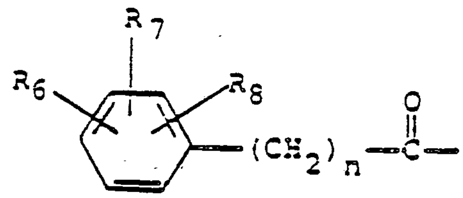

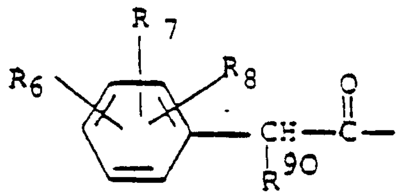

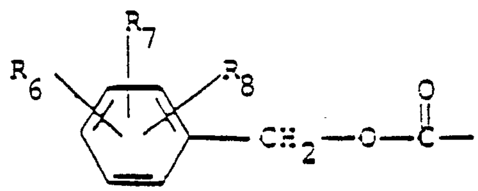

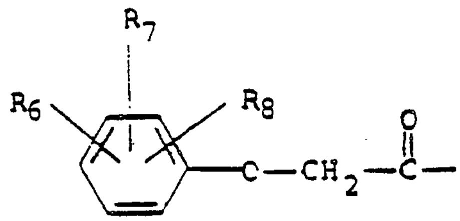

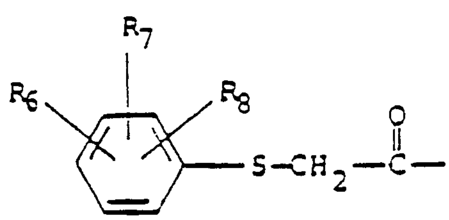

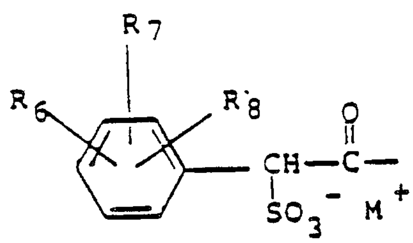

und

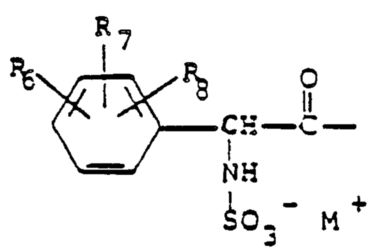

in der n 0, 1, 2 oder 3; $R_6$, $R_7$ und $R_8$ unabhängig voneinander Wasserstoff, Halogen, Hydroxy, Nitro, Amino, Ctan, Trifluormethyl, $C_1$-$C_4$ Alkyl, $C_1$-$C_4$ Alkoxy oder Aminomethyl; $R_{90}$ Amino, Acylamino, Hydroxy, ein Carboxysalz, geschütztes Carboxy, Formyloxy, Azido oder ein Sulfosalz und M ein Kation darstellen, bedeutet.

12. Eine Verbindung nach einem der Ansprüche 1 bis 9, dadurch gekennzeichnet, dass die Acylgruppe $R_1$ eine heteroaromatische Gruppe der allgemeinen Formel

$$R_{101}-(CH_2)_n-CO-$$

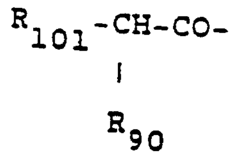

$$R_{101}-O-CH_2-CO-$$

$$R_{101}-S-CH_2-CO-$$ und

$$R_{101}-CO-CO-$$

in denen n 0, 1, 2 oder 3; $R_{90}$ Amino, Acylamino, Hydroxy, ein Carboxysalz, geschütztes Carboxy, Azido oder ein Sulfosalz; und $R_{101}$ einen gegebenenfalls substituierten 5-, 6- oder 7-gliedrigen heterocyclischen Ring mit 1, 2, 3 oder 4 Stickstoff-, Sauerstoff- und/oder Schwefelatomen darstellen,
bedeutet.

13. Verbindung nach einem der Ansprüche 1 bis 9, dadurch gekennzeichnet, dass die Acylgruppe $R_1$ eine [[(4-Substituierte-2,3-dioxo-1-piperazinyl)carbonyl]-amino]arylacetylgruppe der Formel

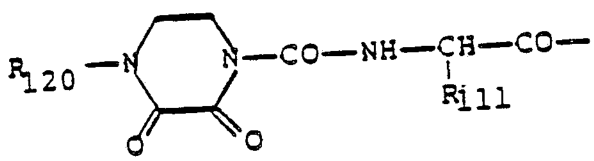

in der $R_{111}$ niederes Alkyl, Hydroxy-niederes Alkyl, einen gegebenenfalls substituierten 5-, 6- oder 7-gliedrigen heterocyclischen Ring mit 1, 2, 3 oder 4 Stickstoff-, Sauerstoff- und/oder Schwefelatomen, oder eine aromatische Gruppe der allgemeinen Formel

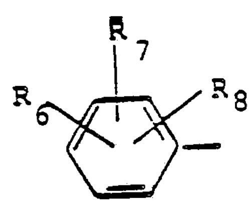

darstellt,( worin $R_6$, $R_7$ und $R_8$ unabhängig voneinander Wasserstoff, Halogen, Hydroxy, Nitro, Amino, Cyan, Trifluormethyl, $C_1$-$C_4$ Alkyl, $C_1$-$C_4$ Alkoxy oder Aminomethyl) und $R_{120}$ gegebenenfalls durch eine oder mehrere der Gruppen Halogen, Cyan, Nitro, Amino oder Mercapto substituiertes niederes Alkyl darstellt,
bedeutet.

14. Verbindung nach einem der Ansprüche 1 bis 9, dadurch gekennzeichnet, dass Acylgruppe $R_1$ eine (Acylamino)arylacetylgruppe der allgemeinen Formel

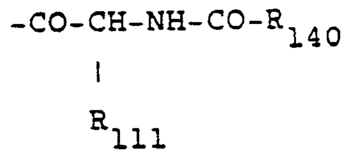

in der $R_{111}$ niederes Alkyl, Hydroxy-niederes Alkyl oder eine aromatischen Gruppe der Formel

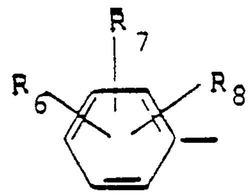

in der $R_6$, $R_7$ und $R_8$ unabhängig voneinander Wasserstoff, Halogen, Hydroxy, Nitro, Amino, Cyan, Trifluormethyl, $C_1$-$C_4$ Alkyl, $C_1$-$C_4$ Alkoxy, Aminomethyl oder einen gegebenenfalls substituierten 5-, 6- oder 7-gliedrigen heterocyclischen Ring mit 1, 2, 3 oder 4 Stickstoff-, Sauerstoff- und/oder Schwefelatomen und $R_{140}$ die Gruppe

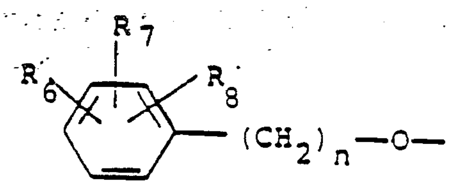

(worin $R_6$, $R_7$ und $R_8$ die obige Bedeutung hat und n 0, 1, 2 oder 3 bedeutet) Wasserstoff und gegebenenfalls substituiertes niederes Alkyl, Amino, niederes Alkylamino, di-niederes Alkylamino, Cyan-niederes Alkyl)amino, Hydrazino, niederes Alkylhydrazino, Arylhydrazino oder Acylhydrazino darstellt, bedeutet.

15. Verbindung nach einem der Ansprüche 1-9, dadurch gekennzeichnet, dass die Acylgruppe $R_1$ eine substituierte Acyliminoacylacetylgruppe der Formel

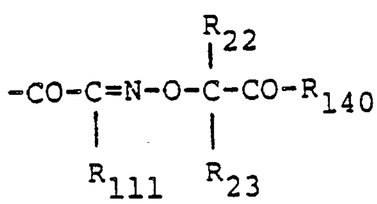

in der $R_{111}$ niederes Alkyl, Hydroxy-niederes Alkyl oder eine aromatische Gruppe der Formel

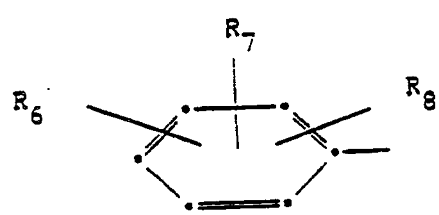

worin $R_6$, $R_7$ und $R_8$ unabhängig voneinander Wasserstoff, Halogen, Hydroxy, Nitro, Amino, Cyan, Trifluormethyl, $C_1$-$C_4$ Alkyl, $C_1$-$C_4$ Alkoxy, Aminomethyl oder einen gegebenenfalls substituierten 5-, 6- oder

7-gliedrigen heterocyclischen Ring mit 1, 2, 3 oder 4 Stickstoff-, Sauerstoff- und/oder Schwefelatomen, und $R_{140}$ die Gruppe

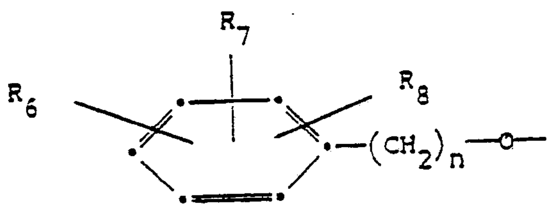

(worin $R_6$, $R_7$ und $R_8$ die obige Bedeutung hat und n 0, 1, 2 oder 3 darstellt), Wasserstoff oder gegebenenfalls substituiertes niederes Alkyl, Amino, niederes Alkylamino, di-niederes Alkylamino, (Cyan-niederes Alkyl)amino, Hydrazino, niederes Alkylhydrazino, Arylhydrazino oder Acylhydrazino und $R_{22}$ und $R_{23}$ unabhängig voneinander Wasserstoff oder niederes Alkyl oder $R_{22}$ und $R_{23}$ zusammen mit dem benachbarten Kohlenstoffatom einer $C_3$-$C_7$ carbocyclischen Ring darstellen,
bedeutet.

16. Verbindung nach einem der Ansprüche 1 bis 9, dadurch gekennzeichnet, dass die Acylgruppe $R_1$ eine [[[3-Substituierte-2-oxo-1-imidazolidinyl]carbonyl]amino]arylacetylgruppe der Formel

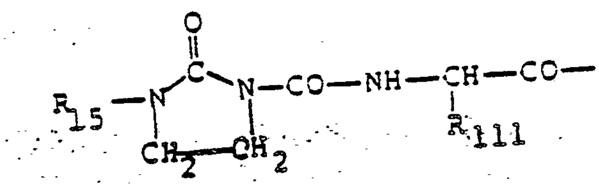

in der $R_{111}$ niederes Alkyl, Hydroxy-niederes Alkyl oder eine aromatische Gruppe der Formel

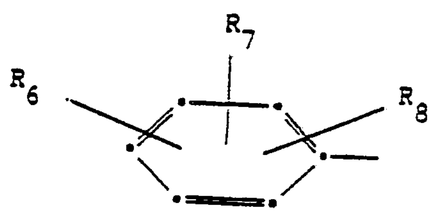

worin $R_6$, $R_7$ und $R_8$ unabhängig voneinander Wasserstoff, Halogen, Hydroxy, Nitro, Amino, Cyan, Trifluormethyl, $C_1$-$C_4$ Alkyl, $C_1$-$C_4$ Alkoxy, Aminomethyl oder einen gegebenenfalls substituierten 5-, 6- oder 7-niedrigen hetrocyclischen Ring mit 1, 2, 3 oder 4 Stickstoff, Sauerstoff- und/oder Schwefelstoffatomen und $R_{15}$ Wasserstoff, niederes Alkylsulfonyl, Arylmethylenamino (d.h. -$NCHR_{111}$, worin $R_{111}$ die oben gegebene Bedeutung hat), -$COR_{16}$ (worin $R_{16}$ Wasserstoff, niederes Alkyl oder Halogen-niederes Alkyl darstellt), eine aromatische Gruppe (wie oben für $R_{111}$ definiert) oder gegebenenfalls durch eine oder mehrere der Gruppen Halogen, Cyan, Nitro, Amino und/oder Mercapto substituiertes niederes Alkyl darstellen,
bedeutet.

17. Verbindung nach einem der Ansprüche 1-9, dadurch gekennzeichnet, dass $R_1$ eine Gruppe der Formel

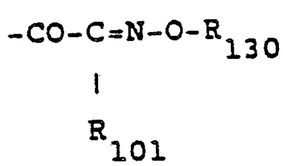

in der $R_{101}$ einen gegebenenfalls durch Halogen, Hydroxy, Nitro, Amino, Cyan, Trifluormethyl, $C_1$-$C_4$ Alkyl oder $C_1$-$C_4$ Alkoxy substituierten 5-, 6- oder 7-gliedrigen hetrocyclischen Ring mit 1, 2, 3 oder 4 Stickstoff-, Sauerstoff- und/oder Schwefelatomen und $R_{130}$ Wasserstoff, niederes Alkyl, $C_3$-$C_7$ Cycloalkyl,

Carboxy-$C_3$-$C_7$-cycloalkyl oder substituiertes niederes Alkyl darstellt, wobei die niedere Alkylgruppe substituiert ist durch einen oder mehrere der Reste Halogen, Cyan, Nitro, Amino, Mercapto, niederes Alkylthio, eine durch $R_{111}$ oben definierte aromatische Gruppe, Carboxy (einschliesslich dessen Salze) Carbamoyl, niederes Alkoxycarbonyl, Phenylmethoxycarbonyl, Diphenylmethoxycarbonyl, Hydroxy-niederes-Alkoxyphosphinyl, di-Hydroxyphosphinyl, Hydroxy(phenylmethoxy)phosphinyl, und/oder di-niederes-Alkoxyphosphinyl,
bedeutet.

18. Verbindungen nach Anspruch 17, dadurch gekennzeichnet, dass $R_{101}$ eine Gruppe der Formel

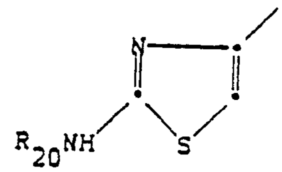

in der $R_{20}$ Wasserstoff oder eine Aminoschutzgruppe und $R_{130}$ Wasserstoff, niederes Alkyl oder eine Gruppe

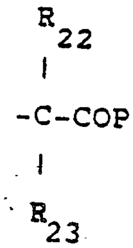

worin $R_{22}$ und $R_{23}$ Wasserstoff oder niederes Alkyl oder zusammen mit dem benachbarten Kohlenstoffatom einen $C_3$-$C_7$ carbocyclischen Ring bilden und P Hydroxy oder $NHR_{19}$ darstellt, worin $R_{19}$ Wasserstoff oder niederes Alkyl, Amino, niederes Alkylamino, Arylamino oder Acylamino bedeutet.
bedeutet.

19. Verbindungen nach Anspruch 18, dadurch gekennzeichnet, dass $R_{20}$ Wasserstoff oder Triphenylmethyl darstellt.

20. Verbindungen nach Anspruch 1,[6R-[6 alpha,7beta-(Z)]]-3-[[[3,4-bis(Acetyloxy)benzoyl]oxy]methyl]-7-[[[2-amino-4-thiazolyl][2-amino-2-oxoethoxy)imino]acetyl]amino]-8-oxo-5-thia-1-azabicyclo[4.2.0]oct-2-en-2-carbonsäure sowie Salze dieser Verbindung und Hydrate dieser Verbindung bzw. Salze.

21. Verbindungen nach Anspruch 1, [6R-[6 alpha, 7 beta(Z)]]-3-[[(3,4-Dihydroxybenzoyl]oxy]methyl]-7-[[[2-amino-4-thiazolyl][2-amino-2-oxoethoxy)imino]acetyl]amino]-8-oxo-5-thia-1-azabicyclo[4.2.0]oct-2-en-2-carbonsäure sowie Salze dieser Verbindung und Hydrate dieser Verbindung bzw. Salze.

22. Verbindungen nach Anspruch 1, [6R-[6 alpha,7beta (Z)-]]-7-[[(2-Amino-4-thiazolyl)(methoxyimino)-acetyl] amino]-3-[[(3,4-dihydroxybenzoyl]oxy]methyl]-8-oxo-5-thia-1-azabicyclo[4.2.0]-oct-2-en-2-carbonsäure,

[6R-[6 alpha,7beta(Z)]]-7-[[[(2-Amino-4-thiazolyl)[1-carboxy-1-methylethoxy]imino]acetyl]amino]-3-[[(3,4-dihyroxybenzoyl)oxy]methyl]-8-oxo-5-thia-1-azabicyclo[4.2.0]oct-2-en-2-carbonsäure,

[6R-[6 alpha,7beta(S*)]]-7-[[α-[[(4-Ethyl-2,3-dioxo-1-piperazinyl)carbonyl]amino]phenylacetyl]amino]-3-[[[(3,4-dihydroxy)benzoyl]oxy]methyl]-8-oxo-5-thia-1-azabicyclo[4.2.0]oct-2-en-carbonsäure,

[6R-[6 alpha,7beta(Z)]]-7-[[(2-Amino-4-thiazolyl)(methoxyimino)acetyl]amino]-3-[[[(3,4-bis(acetyloxy)-benzoyl)]oxy]methyl]-8-oxo-5-thia-1-azabicyclo[4.2.0]oct-2-en-2-carbonsäure,

[6R-[6 alpha,7beta(Z)]]-7-[[(2-Amino-4-thiazolyl)(methoxyimino)acetyl]amino]-3-[[[(2,3-dihydroxyphenyl) carbonyl]oxy]methyl]-8-oxo-5-thia-1-azabicyclo[4.2.0]oct-2-en-2-carbonsäure,

[6R-trans)-3-[[[3,4-bis(Acetyloxy)benzoyl]oxy] methyl]-8-oxo-7-[(phenoxyacetyl)amino]-5-thia-1-azabicyclo[4.2.0]oct-2-en-carbonsäure,

[6R-[6 alpha,7beta(Z)]]-3-[[[3,4,5-tris(Acetyloxy)benzoyl]oxy]methyl]-7-[[(2-amino-4-thiazolyl)(methoxy)-imino)-acetyl]amino]-8-oxo-5-thia-1-azabicyclo[4.2.0]oct-2-en-2-carbonsäure,

[6R-[6 alpha,7beta(Z)]]-7-[[[2-Amino-4-thiazolyl)(methoxyimino)acetyl]amino]-3-[[2-(3,4-dihydroxyphenylacetoxy]methyl]-8-oxo-5-thia-1-azabicyclo[4.2.0]oct-2-en-2-carbonsäure,

[6R-[6 alpha,7beta(Z)]]-3-[[[3,4-bis(Acetyloxy) benzoyl]oxy]methyl]-7-[[(2-amino-4-thiazolyl)[(1-carboxy-1-methylethoxy)imino]acetyl]amino]-8-oxo-5-thia-1-azabicyclo[4.2.0]oct-2-en-2-carbonsäure,
[6R-[6 alpha,7beta(S*)]]-3-[[[3,4-bis(Acetyloxy)benzoyl]oxy]methyl]-7-[[α-[[(4-ethyl-2,3-dioxo-1-piperazinyl)carbonyl]amino]phenylacetyl]amino]-8-oxo-5-thia-1-azabicyclo[4.2.0]oct-2-en-2-carbonsäure,
[6R.cis)-3-[[[3,4-bis(Acetyloxy)benzoyl]oxy]methyl]-7-methoxy-8-oxo-7-[(2-thienylacetyl)amino]-5-thia-1-azabicyclo[4.2.0]oct-2-en-2-carbonsäure,
[6R-cis)-3-[[(3,4-Dihydroxybenzoyl)oxy]methyl]-7-methoxy-8-oxo-7-[(2-thienylacetyl)amino]-5-thia-1-azabicyclo[4.2.0]oct-2-en-2-carbonsäure,
[6R-[6 alpha,7beta(Z)]]-7-[[(2-Amino-4-thiazolyl)(methoxyimino)acetyl]amino]-3-[[[(3,4-bis(acetyloxy)-2,5-dichlorbenzoyl]oxy]methyl]-8-oxo-5-thia-1-azabicyclo[4.2.0]oct-2-en-2-carbonsäure,
[6R-[6 alpha,7beta(Z)]]-3-[[[[[3,4-bis(Acetyloxyphenyl]amino]carbonyl]oxy]methyl]-7-[[(2-amino-4-thiazolyl)(methoxyimino)acetyl]amino]-8-oxo-5-thia-1-azabicyclo[4.2.0]oct-2-en-2-carbonsäure,
[6R-[6 alpha,7beta(Z)]]-3-[[[[[3,4-bis(Acetyloxyphenyl]amino]carbonyl]oxy]methyl]-7-[[[[2-amino-[2-amino-2-oxoethoxy]imino]-4-thiazolyl]acetyl]amino]-8-oxo-5-thia-1-azabicyclo[4.2.0]oct-2-en-2-carbonsäure,
[6R-[6 alpha,7beta(Z)]]-7-[[(2-Amino-4-thia-zolyl)(methoxyimino)acetyl]amino]-3-[[(3,4-dihydroxybenzoyl)thio]methyl]-8-oxo-5-thia-1-azabicyclo[4.2.0]oct-2-en-2-carbonsäure,
[6R-[6 alpha,7beta(Z)]]-7-[[(2-Amino-4-thia-zolyl)(methoxyimino)acetyl]amino]-3-[[[(3,4-bis(acetyloxy)-benzoyl)]thio]methyl]-8-oxo-5-thia-1-azabicyclo[4.2.0]oct-2-en-2-carbonsäure,
[6R-[6 alpha,7beta(Z)]]-3-[[(3,4-bis(Acetyloxy)benzoyl]amino]methyl]-7-[[(2-amino-4-thiazolyl)-(methoxyimino)acetyl]amino]-5-thia-1-azabicyclo[4.2.0]oct-2-en-2-carbonsäure,
[6R-[6 alpha,7beta(Z)]]-3-[[[(3,4-Dihydroxy)benzoyl-]amino]methyl]-7-[[(2-amino-4-thiazolyl)-(methoxyimino)acetyl]amino]-5-thia-1-azabicyclo[4.2.0]oct-2-en-2-carbonsäure
und Salze dieser Verbindung und Hydrate dieser Verbindung bzw. Salze.

23. Verbindungen nach Anspruch 1, [6R-[6alpha,7beta(Z)]]-7-[[[2-Amino[(2-amino-2-oxoethoxy]imino]-4-thiazolyl]acetyl]amino]-3-[[(3,4-dihydroxybenzoyl)thio]methyl]-8-oxo-5-thia-1-azabicyclo[4.2.0]oct-2-en-2-carbonsäure,
[6R-[6alpha,7beta(Z)]-7-[[(2-Amino-4-thiazolyl)[carboxy-methoxy)imino]acetyl]amino]-3-[[3,4-dihydroxy-benzoyl)oxy]methyl]-8-oxo-5-thia-1-azabicyclo[4.2.0]oct-2-en-2-carbonsäure,
[6R-trans)-7-[[(2-Amino-4-thiazolyl)[[1-(aminocarbonyl)-1-methylethoxy]imino]acetyl]amino]-3-[[(3,4-dihydroxybenzoyl)oxy]methyl]-8-oxo-5-thia-1-azabicyclo[4.2.0]oct-2-en-2-carbonsäure
sowie Salze dieser Verbindungen und Hydrate dieser Verbindungen bzw. Salze.

24. Verbindungen der Formel

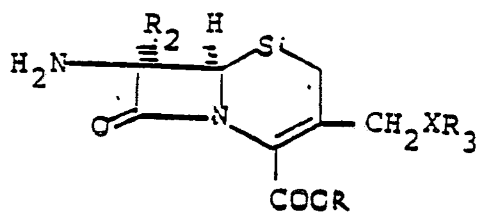

XVIII

in der X -OCO-, -SCO, -NHCO- oder -OCONH-; R Wasserstoff oder eine Carbonsäureschutzgruppe; $R_2$ Wasserstoff oder niederes Alkoxy; und $R_3$ eine der Gruppen

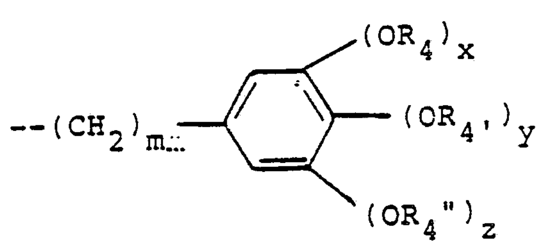

or

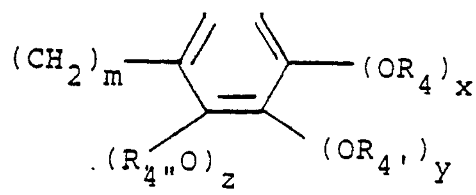

or

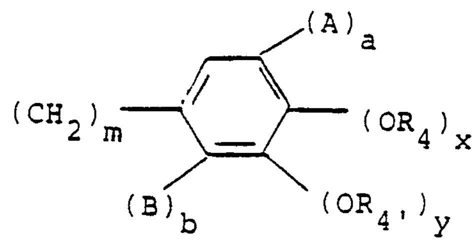

worin $R_4$, $R_4'$ und $R_4''$ unabhängig voneinander Wasserstoff oder $-COR_{200}$ mit $R_{200}$ geradkettigem oder verzweigtem niederem Alkyl, A und B Halogen und a, b, x, y und z unabhängig voneinander Null (wobei in der entsprechenden Ringposition ein Wasserstoffatom vorliegt) oder 1 bedeuten, wobei mindestens zwei der Symbole x, y und z 1 darstellen, und m Null oder eine Zahl von 1 bis 8 bedeutet,
und Salze dieser Verbindungen.

25. Verbindungen nach Anspruch 24, (6R-trans)-7-Amino-3-[[[3,4-bis(acetyloxy)benzoyl]oxy]methyl]-8-oxo-5-thia-1-azabicyclo[4.2.0]oct-2-en-2-carbonsäure-1,1-dimethylethylester und Säueadditionssalze davon.

26. Verbindungen nach Anspruch 24, (6R-trans)-7-Amino-3-[[[3,4-bis(acetyloxy)benzoyl]thio]methyl]-8-oxo-5-thia-1-azabicyclo[4.2.0]oct-2-en-2-carbonsäure-1,1-dimethylethylester und Säueadditionssalze davon.

27. Verbindungen nach einem der Ansprüche 1-23 als pharmazeutische Wirkstoffe.

28. Verbindungen nach einem der Ansprüche 1-23 als pharmazeutische Wirkstoffe zur Behandlung und Prophylaxe von Infektionskrankheiten.

29. Verfahren zur Herstellung von Verbindungen gemäss einem der Ansprüche 1-23, dadurch gekennzeichnet, dass man

a) eine Verbindung der allgemeinen Formel

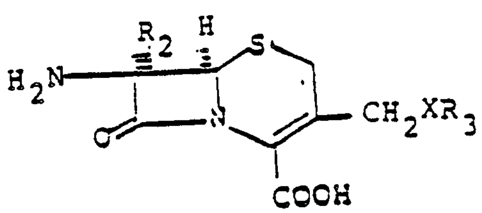

XVIII

in der X und $R_3$ die obige Bedeutung haben und die Carboxygruppe und/oder die Aminogruppe in geschützter Form vorliegen können,
mit einer Säure der Formel $R_1COOH$, worin $R_1$ die obige Bedeutung hat, oder mit einem reaktionsfähigen Derivat davon umsetzt, oder dass man

b) zur Herstellung einer Carbonsäure der Formel I einen Ester der Formel

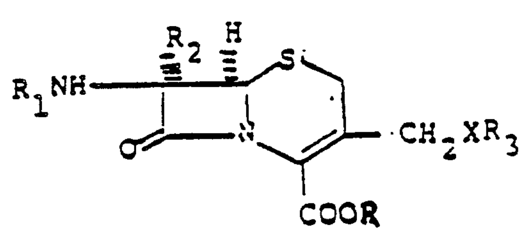

(XIX)

in der $R_1$, $R_2$ und $R_3$ die obige Bedeutung haben und R eine Carbonsäureschutzgruppe darstellt,

in die Carbonsäure der Formel I überführt, oder dass man

c) zur Herstellung einer Verbindung der Formel I, worin $R_1$ einen Aminosubstituenten enthält oder eines Esters oder Salzes davon, die Aminoschutzgruppe im Substituenten $R_{10}$ einer Verbindung der Formel

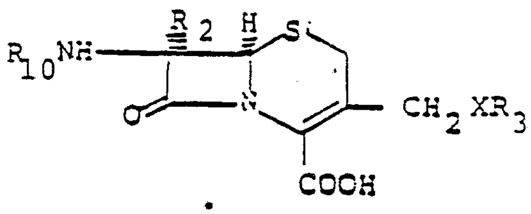

(XX)

in deren $R_2$ und $R_3$ die oben gegebene Bedeutung haben und $R_{10}$ einen eine geschützte Aminogruppe enthaltenden Acylrest darstellt,
oder eines ihrer Ester oder Salze abspaltet, oder dass man

(d) zur Herstellung einer Verbindung der Formel I, worin X -OCO- oder -SCO- darstellt, eine Verbindung der Formel

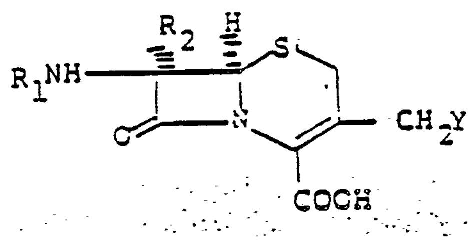

(XXI)

in der $R_1$ die obige obige Bedeutung hat und Y eine austretende Gruppe darstellt,
oder einen Ester oder Salz davon mit einem Salz der Carbonsäure $R_3COQH$, worin $R_3$ die obige Bedeutung hat und Q O oder S darstellt, umsetzt, oder dass man

(e) zur Herstellung einer Verbindung der Formel I worin X -OCO- oder -OCONH- darstellt, eine Verbindungen der Formel

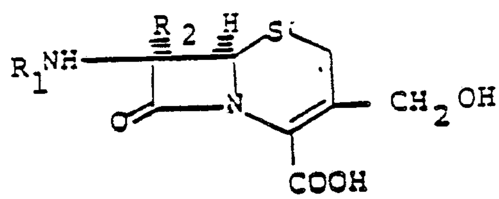

(XXII)

in der $R_1$ und $R_2$ die obige Bedeutung haben,
oder einen Ester oder Salz davon mit einer Verbindung der Formel $R_3COZ$, $R_3NHCOZ$ oder $R_3NCO$, worin $R_3$ die obige Bedeutung hat und Z eine acylaktivierende Gruppe darstellt, umsetzt, oder dass man

(f) zur Herstellung einer Verbindung der Formel I, worin X -NHCO- darstellt, eine Verbindung der Formel

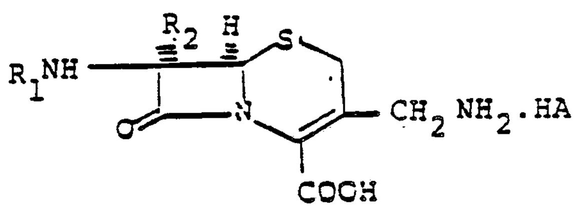

XXIII

in der $R_1$ und $R_2$ die obige Bedeutung haben und A das Anion einer Säure darstellt,
oder einen Ester oder Salz davon mit einer Säure der Formel $R_3$-COOH, worin $R_3$ die obige Bedeutung hat, umsetzt, oder dass man

(g) zur Herstellung eines leicht hydrolysierbaren Esters einer Verbindung der Formel I eine Carbonsäure der Formel I einer entsprechenden Veresterung unterwirft, oder dass man

(h) zur Herstellung von Salzen oder Hydraten einer Verbindung der Formel I bzw. von Hydraten dieser Salze eine Verbindung der Formel I in ein Salz oder Hydrat bzw. in ein Hydrat dieses Salzes überführt.

30. Pharmazeutische Präparate gekennzeichnet durch einen Gehalt an einer Verbindung gemäss einem der Ansprüche 1-23.

31. Pharmazeutische Präparate zur Behandlung und Prophylaxe von Infektionskrankheiten gekennzeichnet durch einen Gehalt an einer Verbindung gemäss einem der Ansprüche 1-23.

32. Verwendung von Verbindungen gemäss einem der Ansprüche 1-23 bei der Behandlung bzw. Prophylaxe von Krankheiten.

33. Verwendung von Verbindungen gemäss einem der Ansprüche 1-23 bei der Behandlung bzw. Prophylaxe von Infektionskrankeiten.

34. Verwendung von Verbindungen gemäss einem der Ansprüche 1-23 bei der Herstellung von Arzneimitteln für die Behandlung bzw. Prophylaxe von Infektionskrankheiten.

Patentansprüche für folgende Vertragsstaaten : GR, ES

1. Verfahren zur Herstellung von Acylderivaten der allgemeinen Formel

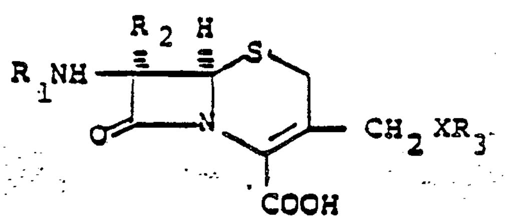

I

in der X -OC0-, -SCO-, -NHCO- oder -OCONH-; $R_1$ eine Acylgruppe; $R_2$ Wasserstoff oder niederes Alkoxy; und $R_3$ die Gruppe

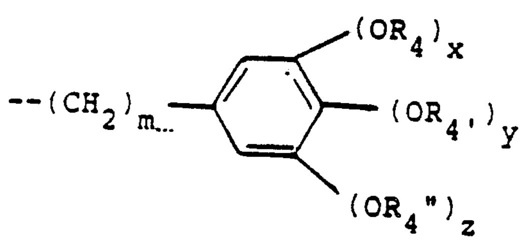

oder

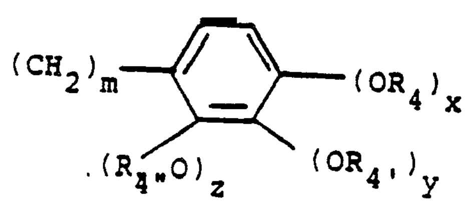

oder

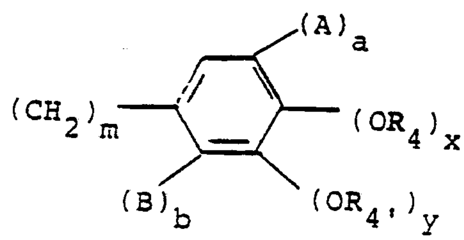

darstellen,

worin $R_4$, $R_4'$ und $R_4''$ unabhängig voneinander Wasserstoff oder $-COR_{200}$ mit $R_{200}$ geradkettigem oder verzweigtem niederem Alkyl, A und B Halogen und a, b, x, y und z unabhängig voneinander Null (wobei in der entsprechenden Ringposition ein Wasserstoffatom vorliegt) oder 1 bedeuten, wobei mindestens zwei der Symbole x, y und z 1 darstellen, und m Null oder eine Zahl von 1 bis 8 bedeutet,
sowie von leicht hydrolysierbaren Estern und pharmazeutisch verträglichen Salzen dieser Verbindungen und von Hydraten der Verbindungen der Formel I bzw. von deren Estern und Salzen, dadurch gekennzeichnet, dass man

a) eine Verbindung der allgemeinen Formel

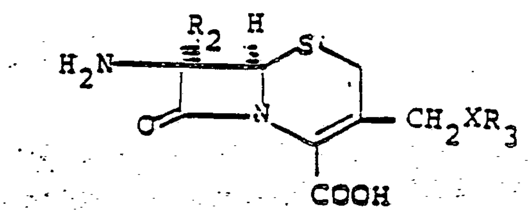

XVIII

in der X und $R_3$ die obige Bedeutung haben und die Carboxygruppe und/oder die Aminogruppe in geschützter Form vorliegen können,
mit einer Säure der Formel $R_1COOH$, worin $R_1$ die obige Bedeutung hat, oder mit einem reaktionsfähigen Derivat davon umsetzt, oder dass man

b) zur Herstellung einer Carbonsäure der Formel I einen Ester der Formel

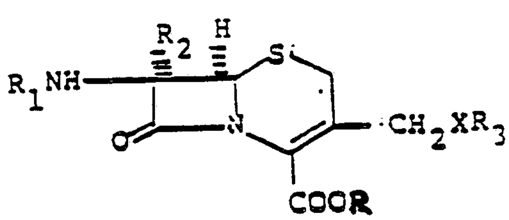

(XIX)

in der $R_1$, $R_2$ und $R_3$ die obige Bedeutung haben und R eine Carbonsäureschutzgruppe darstellt, in die Carbonsäure der Formel I überführt, oder dass man

c) zur Herstellung einer Verbindung der Formel I, worin $R_1$ einen Aminosubstituenten enthält oder eines Esters oder Salzes davon, die Aminoschutzgruppe im Substituenten $R_{10}$ einer Verbindung der Formel

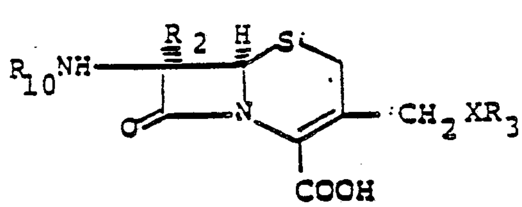

(XX)

in der $R_2$ und $R_3$ die oben gegebene Bedeutung haben und $R_{10}$ einen eine geschützte Aminogruppe enthaltende Acylrest darstellt,
oder eines ihrer Ester oder Salze abspaltet, oder dass man

(d) zur Herstellung einer Verbindung der Formel I, worin X -OCO- oder -SCO- darstellt, eine Verbindung der Formel

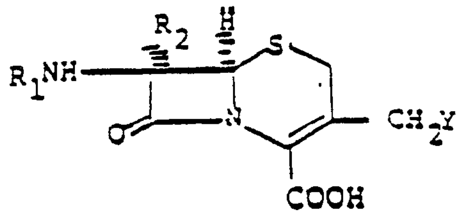

(XXI)

in der $R_1$ die obige Bedeutung hat und Y eine austretende Gruppe darstellt,
oder einen Ester oder Salz davon mit einem Salz der Carbonsäure $R_3COQH$, worin $R_3$ die obige Bedeutung hat und Q O oder S darstellt, umsetzt, oder dass man

(e) zur Herstellung einer Verbindung der Formel I worin X -OCO- oder -OCONH- darstellt, eine Verbindung der Formel

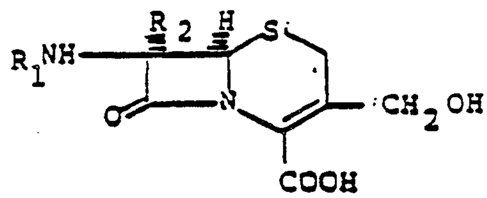

(XXII)

in der $R_1$ und $R_2$ die obige Bedeutung haben,
oder einen Ester oder Salz davon mit einer Verbindung der Formel $R_3COZ$, $R_3NHCOZ$ oder $R_3NCO$, worin $R_3$ die obige Bedeutung hat und Z eine acylaktivierende Gruppe darstellt, umsetzt, oder dass man

(f) zur Herstellung einer Verbindung der Formel I, worin X -NHCO- darstellt, eine Verbindung der Formel

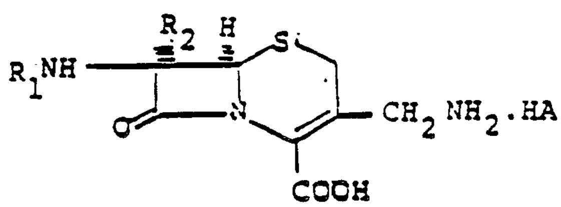

XXIII

in der $R_1$ und $R_2$ die obige Bedeutung haben und A das Anion einer Säure darstellt,
oder einen Ester oder Salz davon mit einer Säure der Formel $R_3$-COOH, worin $R_3$ die obige Bedeutung hat, umsetzt, oder dass man

(g) zur Herstellung eines leicht hydrolysierbaren Esters einer Verbindung der Formel I eine Carbonsäure der Formel I einer ontsprechenden Veresterung unterwirft, oder dass man

(h) zur Herstellung von Salzen oder Hydraten einer Verbindung der Formel I bzw. von Hydraten dieser Salze eine Verbindung der Formel I in ein Salz oder Hydrat bzw. in ein Hydrat dieses Salzes überführt.

2. Verfahren zur Herstellung von Verbindungen nach Anspruch 1, worin m Null oder 1 darstellt, dadurch gekennzeichnet, dass man entsprechend substituierte Ausgangsverbindungen einsetzt.

3. Verfahren zur Herstellung von Verbindungen nach Anspruch 1 oder 2, worin $R_4$, $R_4'$ und $R_4''$ (sofern vorhanden) unabhängig voneinander Wasserstoff oder $-COCH_3$ darstellen, dadurch gekennzeichnet, dass man entsprechend substituierte Ausgangsverbindungen einsetzt.

4. Verfahren zur Herstellung von Verbindungen nach einem der Ansprüche 1 bis 3, worin A und B Chlor darstellen, dadurch gekennzeichnet, dass man entsprechend substituierte Ausgangsverbindungen einsetzt.

5. Verfahren zur Herstellung von Verbindungen nach einem der Ansprüche 1 bis 4, worin R Wasserstoff darstellt, dadurch gekennzeichnet, dass man entsprechend substituierte Ausgangsverbindungen einsetzt.

6. Verfahren zur Herstellung von Verbindungen nach einem der Ansprüche 1 bis 5, worin X -OCO- darstellt, dadurch gekennzeichnet, dass man entsprechend substituierte Ausgangsverbindungen einsetzt.

7. Verfahren zur Herstellung von Verbindungen nach einem der Ansprüche 1 bis 5, worin X -SCO- darstellt, dadurch gekennzeichnet, dass man entsprechend substituierte Ausgangsverbindungen einsetzt.

8. Verfahren zur Herstellung von Verbindungen nach einem der Ansprüche 1-5, worin X -NHCO- darstellt, dadurch gekennzeichnet, dass man entsprechend substituierte Ausgangsverbindungen einsetzt.

9. Verfahren zur Herstellung von Verbindungen nach einem der Ansprüche 1-5, worin X -OCONH- darstellt, dadurch gekennzeichnet, dass man entsprechend substituierte Ausgangsverbindungen einsetzt.

10. Verfahren zur Herstellung von Verbindungen nach einem der Ansprüche 1-9, worin die Acylgruppe $R_1$ eine aliphatische Gruppe der Formel

$R_5CO-$

in der $R_5$ niederes Alkyl, niederes Cycloalkyl, niederes Alkoxy, niederes Alkenyl, niederes Cycloalkenyl oder Cyclohexadienyl; oder durch einen oder mehrere der Reste Halogen, Cyan, Nitro, Amino, Mercapto, niederes Alkylthio oder Cyanmethylthio substituiertes niederes Alkyl oder niederes Alkenyl darstellt, bedeutet, dadurch gekennzeichnet, dass man entsprechend substituierte Ausgangsverbindungen einsetzt.

11. Verfahren zur Herstellung von Verbindungen nach einem der Ansprüche 1 bis 9, worin die Acylgruppe $R_1$ eine carbocyclische aromatische Gruppe der allgemeinen Formel

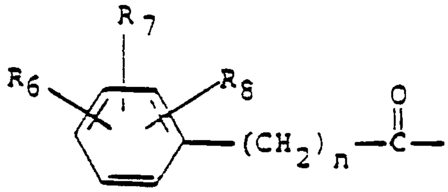

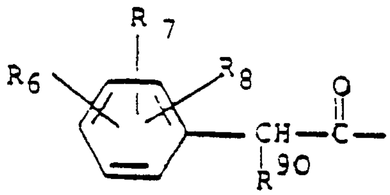

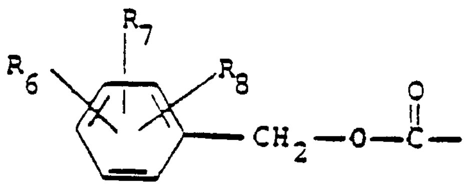

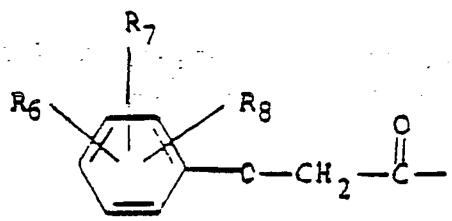

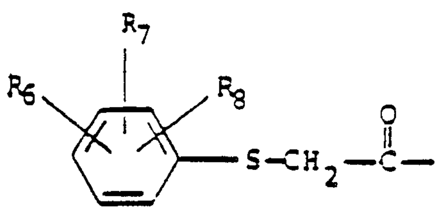

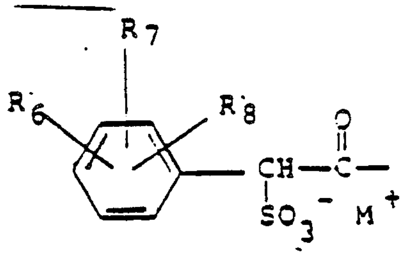

und

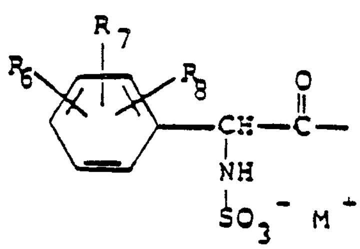

in der n 0, 1, 2 oder 3; $R_6$, $R_7$ und $R_8$ unabhängig voneinander Wasserstoff, Halogen, Hydroxy, Nitro, Amino, Cyan, Trifluormethyl, $C_1$-$C_4$ Alkyl, $C_1$-$C_4$ Alkoxy oder Aminomethyl; $R_{90}$ Amino, Acylamino, Hydroxy, ein Carboxysalz, geschütztes Carboxy, Formyloxy, Azido oder ein Sulfosalz und M ein Kation darstellen,
bedeutet, dadurch gekennzeichnet, dass man entsprechend substituierte Ausgangsverbindungen einsetzt.

12. Verfahren zur Herstellung einer Verbindung nach einem der Ansprüche 1 bis 9, worin die Acylgruppe $R_1$ eine heteroaromatische Gruppe der allgemeinen Formel

$$R_{101}-(CH_2)_n-CO-$$

$$R_{101}-\underset{\displaystyle R_{90}}{\underset{|}{CH}}-CO-$$

$$R_{101}-O-CH_2-CO-$$

$$R_{101}-S-CH_2-CO- \quad \text{und}$$

$$R_{101}-CO-CO-$$

in denen n 0, 1, 2 oder 3; $R_{90}$ Amino, Acylamino, Hydroxy, ein Carboxysalz, geschütztes Carboxy, Azido oder ein Sulfosalz; und $R_{101}$ einen gegebenenfalls substituierten 5-, 6- oder 7-gliedrigen heterocyclischen Ring mit 1, 2, 3 oder 4 Stickstoff-, Sauerstoff- und/oder Schwefelatomen darstellen,
bedeutet, dadurch gekennzeichnet, dass man entsprechend substituierte Ausgangsverbindungen einsetzt.

13. Verfahren zur Herstellung einer Verbindung nach einem der Ansprüche 1 bis 9, worin die Acylgruppe $R_1$ eine [[(4substituierte-2,3-dioxo-1-piperazinyl)carbonyl]-amino]arylacetylgruppe der Formel

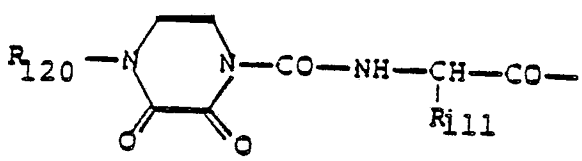

in der $R_{111}$ niederes Alkyl, Hydroxy-niederes Alkyl, einen gegebenenfalls substituierten 5-, 6- oder 7-gliedrigen heterocyclischen Ring mit 1, 2, 3 oder 4 Stickstoff-, Sauerstoff- und/oder Schwefelatomen, oder eine aromatische Gruppe der allgemeinen Formel

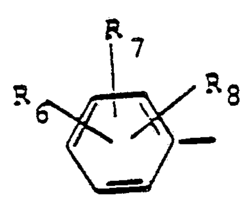

darstellt, (worin $R_6$, $R_7$ und $R_8$ unabhängig voneinander Wasserstoff, Halogen, Hydroxy, Nitro, Amino, Cyan, Trifluormethyl, $C_1$-$C_4$ Alkyl, $C_1$-$C_4$ Alkoxy oder Aminomethyl) und $R_{120}$ gegebenenfalls durch eine oder mehrere der Gruppen Halogen, Cyan, Nitro, Amino oder Mercapto substituiertes niederes Alkyl darstellt,
bedeutet, dadurch gekennzeichnet, dass man entsprechend substituierte Ausgangsverbindungen einsetzt.

14. Verfahren zur Herstellung einer Verbindung nach einem der Ansprüche 1 bis 9, worin die Acylgruppe $R_1$ eine (Acylamino)arylacetylgruppe der allgemeinen Formel

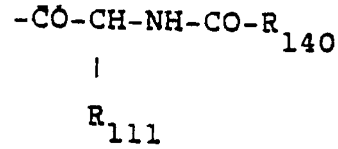

in der $R_{111}$ niederes Alkyl, Hydroxy-niederes Alkyl oder eine aromatischen Gruppe der Formel

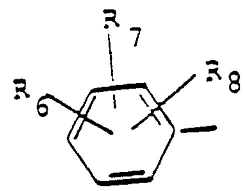

in der $R_6$, $R_7$ und $R_8$ unabhängig voneinander Wasserstoff, Halogen, Hydroxy, Nitro, Amino, Cyan, Trifluormethyl, $C_1$-$C_4$ Alkyl, $C_1$-$C_4$ Alkoxy, Aminomethyl oder einen gegebenenfalls substituierten 5-, 6- oder 7-gliedrigen heterocyclischen Ring mit 1, 2, 3 oder 4 Stickstoff-, Sauerstoff- und/oder Schwefelatomen und $R_{140}$ die Gruppe

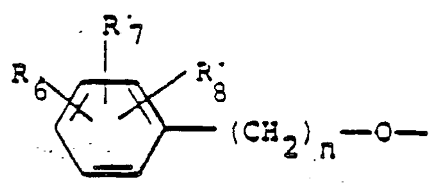

(worin $R_6$, $R_7$ und $R_8$ die obige Bedeutung hat und n 0, 1, 2 oder 3 bedeutet) Wasserstoff und gegebenenfalls substituiertes niederes Alkyl, Amino, niederes Alkylamino, di-niederes Alkylamino, Cyan-niederes Alkyl)amino, Hydrazino, niederes Alkylhydrazino, Arylhydrazino oder Acylhydrazino darstellt, bedeutet, dadurch gekennzeichnet, dass man entsprechend substituierte Ausgangsverbindungen einsetzt.

15. Verfahren zur Herstellung einer Verbindung nach einem der Ansprüche 1-9, worin die Acylgruppe $R_1$ eine substituierte Acyliminoarylacetylgruppe der Formel

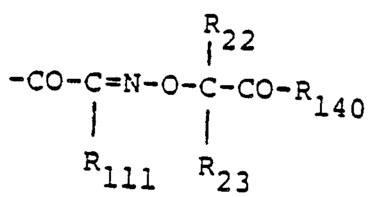

in der $R_{111}$ niederes Alkyl, Hydroxy-niederes Alkyl oder eine aromatische Gruppe der Formel

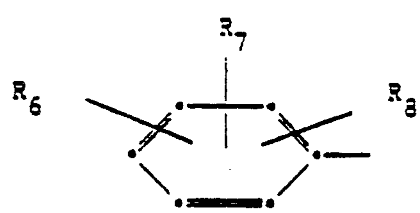

worin $R_6$, $R_7$ und $R_8$ unabhängig voneinander Wasserstoff, Halogen, Hydroxy, Nitro, Amino, Cyan, Trifluormethyl, $C_1$-$C_4$ Alkyl, $C_1$-$C_4$ Alkoxy, Aminomethyl oder einen gegebenenfalls substituierten 5-, 6- oder 7-gliedrigen heterocyclischen Ring mit 1, 2, 3 oder 4 Stickstoff-, Sauerstoff- und/oder Schwefelatomen, und $R_{140}$ die Gruppe

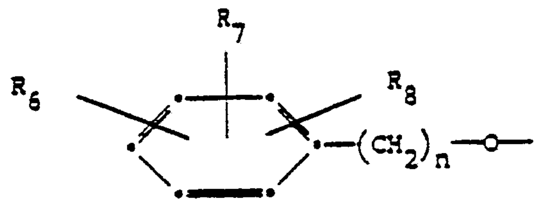

(worin $R_6$, $R_7$ und $R_8$ die obige Bedeutung hat und n 0, 1, 2 oder 3 darstellt), Wasserstoff oder gegebenenfalls substituiertes niederes Alkyl, Amino, niederes Alkylamino, di-niederes Alkylamino, (Cyan-niederes Alkyl)amino, Hydrazino, niederes Alkylhydrazino, Arylhydrazino oder Acylhydrazino und $R_{22}$ und $R_{23}$ unabhängig voneinander Wasserstoff oder niederes Alkyl oder $R_{22}$ und $R_{23}$ zusammen mit dem benachbarten Kohlenstoffatom einen $C_3$-$C_7$ carbocyclischen Ring darstellen,
bedeutet, dadurch gekennzeichnet, dass man entsprechend substituierte Ausgangsverbindungen einsetzt.

16. Verfahren zur Herstellung einer Verbindung nach einem der Ansprüche 1 bis 9, worin die Acylgruppe $R_1$ eine [[[3-Substituierte-2-oxo-1-imidazolidinyl]carbonyl]amino]arylacetylgruppe der Formel

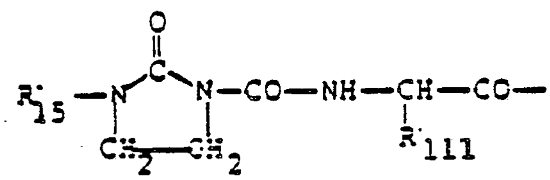

in der $R_{111}$ niederes Alkyl, Hydroxy-niederes Alkyl oder eine aromatische Gruppe der Formel

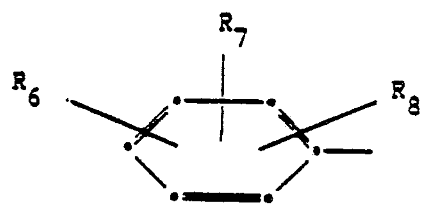

worin $R_6$, $R_7$ und $R_8$ unabhängig voneinander Wasserstoff, Halogen, Hydroxy, Nitro, Amino, Cyan, Trifluormethyl, $C_1$-$C_4$ Alkyl, $C_1$-$C_4$ Alkoxy, Aminomethyl oder einen gegebenenfalls substituierten 5-, 6- oder 7-gliedrigen hetrocyclischen Ring mit 1, 2, 3 oder 4 Stickstoff-, Sauerstoff- und/oder Schwefelstoffatomen und $R_{15}$ Wasserstoff, niederes Alkylsulfonyl, Arylmethylenamino (d.h. -$NCHR_{111}$, worin $R_{111}$die oben gegebene Bedeutung hat), -$COR_{16}$ (worin $R_{16}$ Wasserstoff, niederes Alkyl oder Halogen-niederes Alkyl darstellt), eine aromatische Gruppe (wie oben für $R_{111}$ definiert) oder gegebenenfalls durch eine oder mehrere der Gruppen Halogen, Cyan, Nitro, Amino und/oder Mercapto substituiertes niederes Alkyl darstellen,
bedeutet, dadurch gekennzeichnet, dass man entsprechend substituierte Ausgangsverbindungen einsetzt.

17. Verfahren zur Herstellung einer Verbindung nach einem der Ansprüche 1-9, worin $R_1$ eine Gruppe der Formel

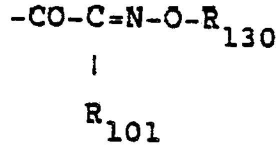

in der $R_{101}$ einen gegebenenfalls durch Halogen, Hydroxy, Nitro, Amino, Cyan, Trifluormethyl, $C_1$-$C_4$ Alkyl oder $C_1$-$C_4$ Alkoxy substituierten 5-, 6- oder 7-gliedrigen hetrocyclischen Ring mit 1, 2, 3 oder 4 Stickstoff-, Sauerstoff- und/oder Schwefelatomen und $R_{130}$ Wasserstoff, niederes Alkyl, $C_3$-$C_7$ Cycloalkyl, Carboxy-$C_3$-$C_7$-cycloalkyl oder substituiertes niederes Alkyl darstellt, wobei die niedere Alkylgruppe substituiert ist durch einen oder mehrere der Reste Halogen, Cyan, Nitro, Amino, Mercapto, niederes Alkylthio, eine durch $R_{111}$ oben definierte aromatische Gruppe, Carboxy (einschliesslich dessen Salze) Carbamoyl,

niederes Alkoxycarbonyl, Phenylmethoxycarbonyl, Diphenylmethoxycarbonyl, Hydroxy-niederes-Alkoxyphosphinyl, di-Hydroxyphosphinyl, Hydroxy(phenylmethoxy)phosphinyl, und/oder di-niederes-Alkoxyphosphinyl,
bedeutet, dadurch gekennzeichnet, dass man entsprechend substituierte Ausgangsverbindungen einsetzt.

18. Verfahren zur Herstellung von Verbindungen nach Anspruch 17, worin $R_{101}$ eine Gruppe der Formel

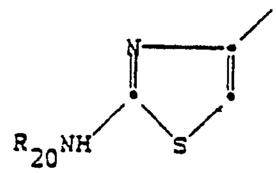

in der $R_{20}$ Wasserstoff oder eine Aminoschutzgruppe und $R_{130}$ Wasserstoff, niederes Alkyl oder eine Gruppe

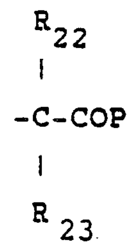

worin $R_{22}$ und $R_{23}$ Wasserstoff oder niederes Alkyl oder zusammen mit dem benachbarten Kohlenstoffatom einen $C_3$-$C_7$ carbocyclischen Ring bilden und P Hydroxy oder $NHR_{19}$ darstellt, worin $R_{19}$ Wasserstoff oder niederes Alkyl, Amino, niederes Alkylamino, Arylamino oder Acylamino bedeutet,
bedeutet, dadurch gekennzeichnet, dass man entsprechend substituierte Ausgangsverbindungen einsetzt.

19. Verfahren zur Herstellung von Verbindungen nach Anspruch 18, worin $R_{20}$ Wasserstoff oder Triphenylmethyl darstellt, dadurch gekennzeichnet, dass man entsprechend substituierte Ausgangsverbindungen einsetzt.

20. Verfahren zur Herstellung von Verbindungen nach Anspruch 1,[6R-[6 alpha,7beta(Z)]]-3-[[[3,4-bis-(Acetyloxy)benzoyl]oxy]methyl]-7-[[[2-amino-4-thiazolyl][2-amino-2-oxoethoxy)imino]acetyl]amino]-8-oxo-5-thia-1-azabicyclo[4.2.0]oct-2-en-2-carbonsäure sowie von Salzen dieser Verbindung und von Hydraten dieser Verbindung bzw. Salze, dadurch gekennzeichnet, dass man entsprechend sutstituierte Ausgangsverbindungen einsetzt.

21. Verfahren zur Herstellung von Verbindungen nach Anspruch 1, [6R-[6 alpha, 7 beta(Z)]]-3-[[(3,4-Dihydroxybenzoyl)oxy]methyl]-7-[[[2-amino-4-thiazolyl][2-amino-2-oxoethoxy)imino]acetyl]-amino]-8-oxo-5-thia-1-azabicyclo[4.2.0]oct-2-en-2-carbonsäure sowie von Salzen dieser Verbindung und von Hydraten dieser Verbindung bzw. Salze, dadurch gekennzeichnet, dass man entsprechend substituierte Ausgangsverbindungen einsetzt.

22. Verfahren zur Herstellung von Verbindungen nach Anspruch 1, [6R-[6 alpha,7beta (Z)-]]-7-[[(2-Amino-4-thiazolyl)(methoxyimino)acetyl]amino]-3-[[(3,4-dihydroxybenzoyl]oxy]methyl]-8-oxo-5-thia-1-azabicyclo[4.2.0]-oct-2-en-2-carbonsäure,

[6R-[6 alpha,7beta(Z)]]-7-[[[(2-Amino-4-thiazolyl)[1-carboxy-1-methylethoxy]imino]acetyl]amino]-3-[[(3,4-dihyroxybenzoyl)oxy]methyl]-8-oxo-5-thia-1-azabicyclo[4.2.0]oct-2-en-2-carbonsäure,

[6R-[6 alpha,7beta(S*)]]-7-[[α-[[(4-Ethyl-2,3-dioxo-1-piperazinyl)carbonyl]amino]phenylacetyl]amino]-3-[[-[(3,4-dihydroxy)benzoyl]oxy]methyl]-8-oxo-5-thia-1-azabicyclo[4.2.0]oct-2-en-carbonsäure,

[6R-[6 alpha,7beta(Z)]]-7-[[(2-Amino-4-thiazolyl)(methoxyimino)acetyl]amino]-3-[[[(3,4-bis(acetyloxy)-benzoyl)]oxy]methyl]-8-oxo-5-thia-1-azabicyclo[4.2.0]oct-2-en-2-carbonsäure,

[6R-[6 alpha,7beta(Z)]]-7-[[(2-Amino-4-thiazolyl)(methoxyimino)acetyl]amino]-3-[[[(2,3-dihydroxyphenyl) carbonyl]oxy]methyl]-8-oxo-5-thia-1-azabicyclo[4.2.0]oct-2-en-2-carbonsäure,

[6R-trans)-3-[[[3,4-bis(Acetyloxy)benzoyl]oxy] methyl]-8-oxo-7-[(phenoxyacetyl)amino]-5-thia-1-azabicyclo[4.2.0]oct-2-en-carbonsäure,

[6R-[6 alpha,7beta(Z)]]-3-[[[3,4,5-tris(Acetyloxy)benzoyl]oxy]methyl]-7-[[(2-amino-4-thiazolyl)-(methoxyimino)-acetyl]amino]-8-oxo-5-thia-1-azabicyclo[4.2.0]oct-2-en-2-carbonsäure,

[6R-[6 alpha,7beta(Z)]]-7-[[[2-Amino-4-thiazolyl)(methoxyimino)acetyl]amino]-3-[[2-(3,4-dihydroxyphenyl)acetoxy]methyl]-8-oxo-5-thia-1-azabicyclo[4.2.0]oct-2-en-2-carbonsäure,

[6R-[6 alpha,7beta(Z)]]-3-[[[3,4-bis(Acetyloxy) benzoyl]oxy]methyl]-7-[[(2-amino-4-thiazolyl[[(1-carboxy-1-methylethoxy)imino]acetyl]amino]-8-oxo-5-thia-1-azabicyclo[4.2.0]oct-2-en-2-carbonsäure,

[6R-6 alpha,7beta(S*)]]-3-[[[3,4-bis(Acetyloxy)benzoyl]oxy]methyl]-7-[[α-[[(4-ethyl-2,3-dioxo-1-piperazinyl)carbonyl]amino]phenylacetyl]amino]-8-oxo-5-thia-1-azabicyclo[4.2.0]oct-2-en-2-carbonsäure,

[6R.cis)-3-[[[3,4-bis(Acetyloxy)benzoyl]oxy]methyl]-7-methoxy-8-oxo-7-[(2-thienylacetyl)amino]-5-thia-1-azabicyclo[4.2.0]oct-2-en-2-carbonsäure,

[6R-cis)-3-[[(3,4-Dihydroxybenzoyl)oxy]methyl]-7-methoxy-8-oxo-7-[(2-thienylacetyl)amino]-5-thia-1-azabicyclo[4.2.0]oct-2-en-2-carbonsäure,

[6R-[6 alpha,7beta(Z)]]-7-[[(2-Amino-4-thiazolyl)(methoxyimino)acetyl]amino]-3-[[[(3,4-bis(acetyloxy-2,5-dichlorbenzoyl)]oxy]methyl]-8-oxo-5-thia-1-azabicyclo[4.2.0]oct-2-en-2-carbonsäure,

[6R-[6 alpha,7beta(Z)]]-3-[[[[[3,4-bis(Acetyloxyphenyl]amino]carbonyl]oxy]methyl]-7-[[(2-amino-4-thiazolyl)(methoxyimino)acetyl]amino]-8-oxo-5-thia-1-azabicyclo[4.2.0]oct-2-en-2-carbonsäure,

[6R-[6 alpha,7beta(Z)]]-3-[[[[[3,4-bis(Acetyloxyphenyl]amino]carbonyl]oxy]methyl]-7-[[[[2-amino-[2-amino-2-oxoethoxy]imino]-4-thiazolyl]acetyl]amino]-8-oxo-5-thia-1-azabicyclo[4.2.0]oct-2-en-2-carbonsäure,

[6R-[6 alpha,7beta(Z)]]-7-[[(2-Amino-4-thia-zolyl)(methoxyimino)acetyl]amino]-3-[[(3,4-dihydroxy-benzoyl)thio]methyl]-8-oxo-5-thia-1-azabicyclo[4.2.0]oct-2-en-2-carbonsäure,

[6R-[6 alpha,7beta(Z)]]-7-[[(2-Amino-4-thia-zolyl)(methoxyimino)acetyl]amino]-3-[[[(3,4-bis(acetyloxy)-benzoyl)]thio]methyl]-8-oxo-5-thia-1-azabicyclo[4.2.0]oct-2-en-2-carbonsäure,

[6R-[6 alpha,7beta(Z)]]-3-[[(3,4-bis(Acetyloxy)benzoyl)]amino]methyl]-7-[[(2-amino-4-thiazolyl)-(methoxyimino)acetyl]amino]-5-thia-1-azabicyclo[4.2.0]oct-2-en-2-carbonsäure,

[6R-[6 alpha,7beta(Z)]]-3-[[[(3,4-Dihydroxy)benzoyl-]amino]methyl]-7-[[(2-amino-4-thiazolyl)-(methoxyimino)acetyl]amino]-5-thia-1-azabicyclo[4.2.0]oct-2-en-2-carbonsäure

sowie von Salzen dieser Verbindungen und von Hydraten dieser Verbindungen bzw. Salze.

23. Verfahren zur Herstellung von Verbindungen nach Anspruch 1, [6R-[6alpha,7beta-(Z)]]-7-[[[2-Amino[-(2-amino-2-oxoethoxy]imino]-4-thiazolyl]acetyl]amino]-3-[[(3,4-dihydroxybenzoyl)thio]-methyl]-8-oxo-5-thia-1-azabicyclo[4.2.0]oct-2-en-2-carbonsäure,

[6R-[6alpha,7beta(Z)]-7-[[(2-Amino-4-thiazolyl)[carboxy-methoxy)imino]acetyl]amino]-3-[[3,4-dihydroxy-benzoyl)oxy]methyl]-8-oxo-5-thia-1-azabicyclo[4.2.0]oct-2-en-2-carbonsäure,

[6R-trans)-7-[[(2-Amino-4-thiazolyl)[[1-(aminocarbonyl)-1-methylethoxy]imino]acetyl]amino]-3-[[(3,4-dihydroxybenzoyl)oxy]methyl]-8-oxo-5-thia-1-azabicyclo[4.2.0]oct-2-en-2-carbonsäure

sowie von Salzen dieser Verbindungen und von Hydraten dieser Verbindungen bzw. Salze, dadurch gekennzeichnet, dass man entsprechend substituierte Ausgangsverbindungen einsetzt.

24. Verfahren zur Herstellung von pharmazeutischen Präparaten, dadurch gekennzeichnet, dass man ein in Anspruch 1 definiertes Acylderivat der Formel I oder einen leicht hydrolysierbaren Ester oder ein Salz dieser Verbindung oder ein Hydrat einer Verbindung der Formel I bzw. eines Esters oder Salzes davon als wirksamen Bestandteil mit zur therapeutischen Verabreichung geeigneten, nichttoxischen, inerten, an sich in solchen Präparaten üblichen festen und flüssigen Trägern und/oder Excipientien vermischt.

25. Verwendung von Verbindungen gemäss einem der Ansprüche 1-23 bei der Behandlung bzw. Prophylaxe von Krankheiten.

26. Verwendung von Verbindungen gemäss einem der Ansprüche 1-23 bei der Behandlung bzw. Prophylaxe von Infektionskrankheiten.

27. Verwendung von Verbindungen gemäss einem der Ansprüche 1-23 bei der Herstellung von Arzneimitteln für die Behandlung bzw. Prophylaxe von Infektionskrankheiten.

28. Verbindungen der Formel

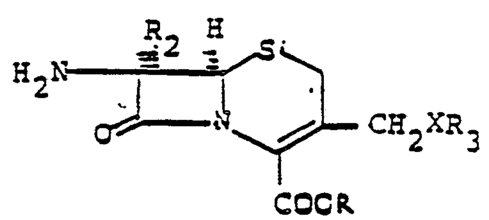

XVIII

in der X -OCO-, -SCO, -NHCO- oder -OCONH-; R Wasserstoff oder eine Carbonsäureschutzgruppe; $R_2$ Wasserstoff oder niederes Alkoxy; und $R_3$ eine der Gruppen

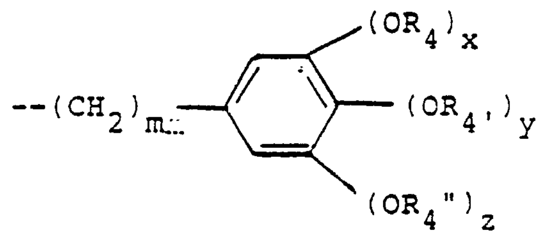

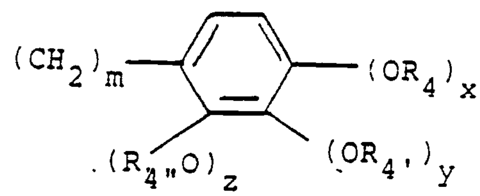

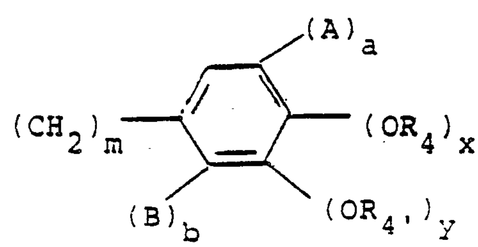

worin $R_4$, $R_4'$ und $R_4''$ unabhängig voneinander Wasserstoff oder $-COR_{200}$ mit $R_{200}$ geradkettigem oder verzweigtem niederem Alkyl, A und B Halogen und a, b, x, y und z unabhängig voneinander Null (wobei in der entsprechenden Ringposition ein Wasserstoffatom vorliegt) oder 1 bedeuten, wobei mindestens zwei der Symbole x, y und z 1 darstellen, und m Null oder eine Zahl von 1 bis 8 bedeutet,
und Salze dieser Verbindungen.

29. Verbindungen nach Anspruch 28, (6R-trans)-7-Amino-3-[[[3,4-bis(acetyloxy)benzoyl]oxy]methyl]-8-oxo-5-thia-1-azabicyclo[4.2.0]oct-2-en-2-carbonsäure-1,1-dimethylethylester und Säueadditionssalze davon.

30. Verbindungen nach Anspruch 28, (6R-trans)-7-Amino-3-[[[3,4-bis(acetyloxy)benzoyl]thio]methyl]-8-oxo-5-thia-1-azabicyclo[4.2.0]oct-2-en-2-carbonsäure-1,1-dimethylethylester und Säueadditionssalze davon.